# FASCICULE DE BREVET EUROPEEN

(11) **EP 2 041 119 B1**
(45) Date de publication et mention de la délivrance du brevet: **10.05.2017**
(21) Numéro de dépôt: 07803834.6
(22) Date de dépôt: 03.07.2007
(51) Int. Cl.: C07D 403/04, C07D 413/14, C07D 403/14, C07D 405/14, A61K 31/551, A61K 31/4184, A61K 31/5377, A61K 31/496, A61P 35/00

(54) **DERIVES DE PYRAZOLYLBENZIMIDAZOLE, COMPOSITIONS LES CONTENANT ET LEUR UTILISATION**
PYRAZOLYLBENZIMIDAZOLDERIVATE, DIESE ENTHALTENDE ZUSAMMENSETZUNGEN UND IHRE VERWENDUNG
PYRAZOLYLBENZIMIDAZOLE DERIVATIVES, COMPOSITIONS CONTAINING THEM AND USE THEREOF

(30) Priorité: 04.07.2006 FR 0606064
(43) Date de publication de la demande: 01.04.2009
(73) Titulaire: Aventis Pharma S.A., 92160 Antony (FR)
(72) Inventeur: CHERRIER, Marie-Pierre, 92160 Antony (FR); PARMANTIER, Eric, 92160 Antony (FR); MINOUX, Hervé, 92160 Antony (FR); CLERC, François, 92160 Antony (FR); ANGOUILLANT-BONIFACE, Odile, 92160 Antony (FR); BROLLO, Maurice, 92160 Antony (FR); SCHIO, Laurent, 92160 Antony (FR)
(74) Mandataire: Lavoix
(86) Numéro de dépôt international: PCT/FR2007/001126
(87) Numéro de publication internationale: WO 2008/003857

(56) Documents cités:
- WO-A-03/035065
- WO-A-2005/002552
- G.A. PATANI ET AL.: "Bioisosterism: A Rational Approach in Drug Design", CHEM. REV., vol. 96, 1996, pages 3147-3176,

## Description

La présente invention est relative à des dérivés de pyrazolylbenzimidazole, les compositions les contenant, et les dérivés pour leur utilisation comme médicaments, notamment comme agent anticancéreux via l'inhibition de prolifération de cellules tumorales. L'invention est aussi relative au procédé de préparation de ces dérivés ainsi qu'à certains des intermédiaires réactionnels.

### [Domaine technique]

A ce jour, la plupart des produits commerciaux utilisés en chimiothérapie sont des composés cytotoxiques qui posent des problèmes importants d'effets secondaires et de tolérance par les patients. Ces effets pourraient être limités dans la mesure où les médicaments utilisés agissent sélectivement sur les cellules cancéreuses, à l'exclusion des cellules saines. Une des solutions pour limiter les effets indésirables d'une chimiothérapie peut donc consister en l'utilisation de médicaments agissant sur des voies métaboliques ou des éléments constitutifs de ces voies, exprimés majoritairement dans les cellules cancéreuses et qui ne sont pas ou peu exprimés dans les cellules saines. On cherche aussi à ce que, lorsque les composés sont administrés sous forme liquide, ils présentent une solubilité en milieu aqueux suffisante, en particulier à un pH standard de 7,4.

Un problème technique qu'entend résoudre l'invention est donc d'améliorer la sélectivité enzymatique et cellulaire de composés chimiques tout en maintenant une activité anticancéreuse acceptable sur des lignées de cellules tumorales.

### [L'art antérieur]

Les demandes internationales WO 03/035065 et WO 03/035644 décrivent de façon très large des benzimidazoles et leurs analogues comme inhibiteurs de kinases. La demande internationale WO 2005/002552 décrit des composés de type pyrazoles pouvant être selon une forme particulière des benzimidazoles qui peuvent être utilisés comme inhibiteurs de kinases. Toutefois, les composés divulgués dans ces documents ne semblent pas présenter un profil de candidat médicament optimisé : en effet, lorsqu'ils sont testés, certains des composés présentent des activités sur certaines kinases et/ou certaines lignées cellulaires défavorables en termes d'effets secondaires pouvant résulter de ce profil. Contre toute attente, et c'est ce qui fait l'objet de la présente invention, il a été trouvé qu'il est possible d'améliorer la sélectivité enzymatique et cellulaire de pyrazolylbenzimidazoles tout en maintenant une activité anticancéreuse acceptable sur des lignées de cellules tumorales.

La demande internationale WO 2006/070202 décrit des composés du type benzimidazole-pyrazoles agissant comme inhibiteurs de kinases pour lesquels l'un des substituants sur le noyau benzimidazoles est -OMe ou -CH₂-morpholine, -CH₂-pipérazine ou -CH₂-pipérazine-Me. Aucune de ces demandes ne décrit les composés de la présente invention.

### [Description de l'invention]

### Définitions utilisées

Sauf mention contraire, les définitions suivantes ont été utilisées dans la présente demande.
- halogène : un atome de de fluor, de chlore, de brome et d'iode ;
- alkyle : groupe hydrocarboné aliphatique saturé, linéaire ou ramifié, renfermant au plus 12 atomes de carbone. Il est choisi par exemple parmi les radicaux méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle et également heptyle, octyle, nonyle, décyle, undécyle et dodécyle, ainsi que leurs isomères de position linéaires ou ramifiés. De préférence, ils renferment au plus 6 atomes de carbone ;
- alkyloxy: radical -O-alkyle, dans lequel le radical alkyle a la signification indiquée ci-dessus. Il est choisi par exemple parmi les radicaux méthoxy, éthoxy, propoxy, isopropoxy, butoxy linéaire, secondaire ou tertiaire, pentoxy, hexoxy et heptoxy ainsi que leurs isomères de position linéaires ou ramifiés ;
- cycloalkyle : radical carbocyclique, monocyclique ou bicyclique, renfermant de 3 à 10 chaînons. Par exemple, il s'agit des radicaux cyclopropyle (Cy), cyclobutyle, cyclopentyle et cyclohexyle ;
- aryle : un radical insaturé carbocyclique, monocyclique ou constitué de cycles condensés. On peut citer par exemple les radicaux phényle ou naphtyle. On cite plus particulièrement le radical phényle ;
- aryloxy : un radical O-aryle dans lequel le radical aryle a la signification indiquée ci-dessus ;
- hétérocycloalkyle : un radical carbocyclique saturé constitué au plus de 7 chaînons interrompus par un ou plusieurs hétéroatomes, identiques ou différents, choisis parmi les atomes d'oxygène, d'azote ou de soufre. Comme exemples de radicaux hétérocycloalkyles, on peut citer notamment les radicaux dioxolane, dioxane, dithiolane, thiooxolane, thiooxane, oxirannyle, oxolannyle, dioxolannyle, pipérazinyle, pipéridinyle, pyrrolidinyle, imidazolidinyle, pyrazolidinyle, morpholinyle ou encore tétrahydrofuryle, tétrahydrothiényle, chromanyle, dihydrobenzofuranyle, indolinyle, pipéridinyle, perhydropyranyle, pyrindolinyle, tétrahydroquinoléinyle, tétrahydroisoquinoléinyle ou thioazolidinyle, tous ces radicaux étant éventuellement substitués. Parmi les radicaux hétérocycloalkyles, on peut citer plus particulièrement les radicaux pipérazinyle éventuellement substitué, pipéridinyle éventuellement substitué, pyrrolidinyle éventuellement substitué, imidazolidinyle, pyrazolidinyle, morpholinyle ou thioazolidinyle ;
- hétéroaryle : un radical carbocylique partiellement ou totalement insaturé constitué au plus de 7 chaînons interrompus par un ou plusieurs hétéroatomes, identiques ou différents, choisis parmi les atomes d'oxygène, d'azote ou de soufre. Parmi les radicaux hétéroaryles à 5 chaînons (« five-membered »), on peut citer par exemple les radicaux furyle tel que 2-furyle, thiényle tel que 2-thiényle et 3-thiényle, pyrrolyle, diazolyle, thiazolyle, thiadiazolyle, thiatriazolyle, isothiazolyle, oxazolyle oxadiazolyle, 3- ou 4-isoxazolyle, imidazolyle, pyrazolyle, isoxazolyle. Parmi les radicaux hétéroaryles à 6 chaînons, on peut citer notamment les radicaux pyridyle tel que 2-, 3- et 4-pyridyle, pyrimidyle, pyrimidinyle, pyridazinyle, pyrazinyle et tétrazolyle. Comme radicaux hétéroaryles condensés contenant au moins un hétéroatome choisi parmi le soufre, l'azote et l'oxygène, on peut citer par exemple benzothiényle tel que 3-benzothiényle, benzofuryle, benzofurannyle, benzopyrrolyle, benzimidazolyle, benzoxazolyle, thionaphtyle, indolyle, purinyle, quinoléinyle, isoquinoléinyle et naphtyridinyle. Parmi les radicaux hétéroaryles condensés, on peut citer plus particulièrement les radicaux benzothiényle, benzofuranyle, indolyle ou quinoléinyle, benzimidazolyle, benzothiazolyle, furyle, imidazolyle, indolizinyle, isoxazolyle, isoquinolinyle, isothiazolyle, oxadiazolyle, pyrazinyle, pyridazinyle, pyrazolyle, pyridyle, pyrimidinyle, pyrrolyle, quinazolinyle, 1,3,4-thiadiazolyle, thiazolyle, thiényle et groupes triazolyle, ces radicaux étant éventuellement substitués comme indiqué pour les radicaux hétéroaryles ;
- acyle : un radical R-C(=0)- renfermant au plus 12 atomes de carbone dans lequel le radical R représente un radical alkyle, cycloalkyle, hétérocycloalkyle, aryle ou hétéroaryle, ces radicaux ayant les significations indiquées ci-dessus et étant éventuellement substitués comme indiqué ci-dessus. On cite par exemple les radicaux formyle, acétyle, propionyle, butyryle ou benzoyle, ou encore valéryle, hexanoyle, acryloyle, crotonoyle ou carbamoyle ;
- substitué : fait référence à la présence d'un ou plusieurs susbtituants(s) classiques de la chimie organique et connus de l'homme du métier. Il s'agit notamment des substituants suivants : halogène, alkyle, cycloalkyle, hydroxy, alkyloxy, aryloxy, amino, alkylamino, dialkylamino, acyle ou phényle.

### Composés de l'invention

Les composés de la présente invention répondent à la formule générale (I) suivante : 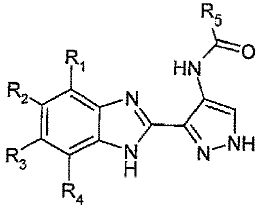 dans laquelle :
- R₁ et R₄ sont indépendamment sélectionnés dans le groupe constitué par : H, Me, Et, CO₂R_{c}, CH₂OR_{c}, OR_{c}, F, Cl, C(=O)NHR_{d} ; dans lequel R_{c} est choisi parmi H, (C₁-C₆)alkyle, (C₁-C₆)alkyle substitué, (C₃-C₇)cycloalkyle, (C₃-C₇)cycloalkyle substitué, aryle, aryle substitué, hétéroaryle et hétéroaryle substitué ; et dans lequel R_{d} est choisi parmi H, (C₁-C₆)alkyle, (C₁-C₆)alkyle substitué, (C₃-C₇)cycloalkyle, (C₃-C₇)cycloalkyle substitué et (C₃-C₇)hétérocycloalkyle comprenant de 1 à 3 hétéroatomes choisis parmi N, O et S, éventuellement substitué,
- R₂ est F,
- R₃ représente un groupe choisi parmi les groupes morpholinyle, homomorpholinyle, -O(CH₂)ₙpipéridinyle avec n = 2, 3 ou 4, -O(CH₂)ₙN(Me)₂ avec n=2, 3 ou 4, N-méthylpipérazinyle et N- méthylhomopipérazinyle, -NH(CH₂)₂NMe₂, et
- R₅ est sélectionné dans le groupe constitué par NMeEt, NH(ⁱPr), NEt₂, N(ⁱPr)₂, NEt(ⁱPr), pyrrolidinyle, pipéridinyle, morpholinyle, N-méthylpipérazinyle, NHCy, NCy₂, NMe(ⁱPr), NH(^{t}Bu), NH(ⁱBu), N(ⁿBu)₂, pipérazinyle, NH(Et), N(ⁿPr)₂, NEt(ⁱPr), où Cy désigne un radical cyclopropyle,
où substitué fait référence à la présence d'un ou plusieurs susbtituants(s) choisis parmi halogène, C₁-C₆ alkyle, C₃-C₁₀ cycloalkyle, hydroxy, C₁-C₆ alkyloxy, aryloxy, amino, C₁-C₆ alkylamino, C₁-C₆ dialkylamino, acyle ou phényle.

Dans la formule I, R₅ est donc relié au groupe C=O par un atome d'azote. Il est sélectionné plus particulièrement dans le groupe constitué par : NMeEt, NH(ⁱPr), NEt₂, N(ⁱPr)₂, NEt(ⁱPr), pyrrolidinyle, pipéridinyle, morpholinyle, N-méthylpipérazinyle, NHCy, NCy₂.

R₃ sous la forme OR₉ ou NRₑN_{f} peut être l'un de ceux décrits dans le Tableau I. On citera par exemple : morpholinyle, homomorpholinyle, -O(CH₂)ₙpipéridinyle avec n = 2, 3 ou 4,-O(CH₂)ₙN(Me)₂ avec n= 2, 3 ou 4, -N-méthylpipérazinyle et -N- méthylhomopipérazinyle,-NH(CH₂)₂NMe₂. De par la définition de R3, les composés suivants qui sont décrits dans WO 03/035065 sont donc exclus de la présente demande :

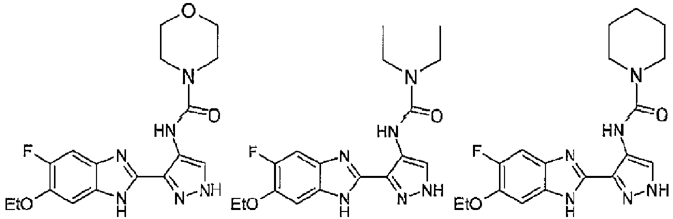

De préférence, l'invention a pour objet les composés de formule (I) dans laquelle :
- R₂ est F, R₃ est choisi parmi morpholinyle, homomorpholinyle, O(CH₂)ₙpipéridinyle avec n = 2, 3 ou 4, O(CH₂)ₙNMe₂ avec n=2, 3 ou 4, N-méthylpipérazinyle et N-méthylhomopipérazinyle et R₅ représente NH(ⁱPr), NHEt, NEt₂, N(ⁱPr)2, NEt(ⁱPr), pyrrolidinyle, pipéridinyle, morpholinyle ;

Plus particulièrement, R1 et R4 désignent tous les deux H.

L'invention a aussi pour objet les composés de formule (la) : 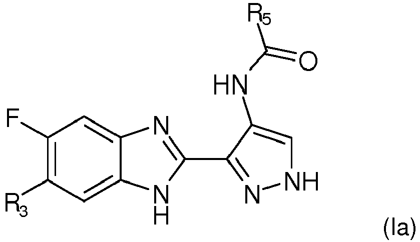 dans laquelle R₃ est tel que défini précédemment et R₅ représente NH(ⁱPr), NHEt, NEt₂, N(ⁱPr)₂, NEt(ⁱPr), pyrrolidinyle, pipéridinyle ou morpholinyle.

Tout particulièrement, R₃ représente morpholinyle, homomorpholinyle, - O(CH₂)ₙpipéridinyle avec n = 2, 3 ou 4, -O(CH₂)ₙNMe₂ avec n=2, 3, 4, N-méthylpipérazinyle et N-méthylhomopipérazinyle et R₅ représente NEt2, ou pipéridinyle

Il est entendu que l'invention a également pour objet les composés de formule (Ib) : 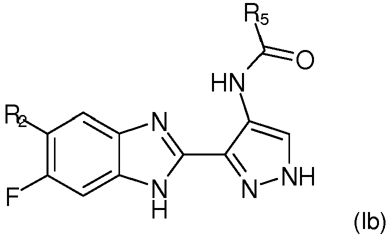 qui correspond aux composés de formule (la) avec une rotation à 180 degrés du benzymidazole (et délocalisation de la double liaison). L'invention a également pour objet les formes tautomères du cycle pyrazole des composés de formules (Ia) ou (Ib).

L'invention a tout particulièrement pour objet un composé de formules (Ia) ou (Ib) dans lesquelles R₃ est choisi parmi -O(CH₂)ₙNMe₂ avec n= 2, 3 ou 4 (de préférence 3), N-méthylpipérazinyle, N-méthylhomopipérazinyle, morpholin-4-yle et homomorpholinyle,

L'invention a tout particulièrement pour objet un composé de formules (Ia) ou (Ib) dans lesquelles R₅ est pipéridin-1-yle.

Les composés selon l'invention peuvent être sous une forme : non chirale, ou racémique, ou enrichie en un stéréo-isomère, ou enrichie en un énantiomère ; sous toutes leur formes tautomères et éventuellement salifiés.

Les composés de l'invention peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention. Comme exemples de sels pharmaceutiquement acceptables, peuvent être cités les sels suivants :
benzènesulfonate, bromhydrate, chlorhydrate, citrate, éthanesulfonate, fumarate, gluconate, iodate, maléate, iséthionate, méthanesulfonate, méthylène-bis-b-oxynaphtoate, nitrate, oxalate, pamoate, phosphate, salicylate, succinate, sulfate, tartrate, théophyllinacétate et p-toluènesulfonate.

Selon un second aspect, l'invention a trait à un médicament, comprenant un composé selon l'invention, ou un sel d'addition de ce composé à un acide pharmaceutiquement acceptable, ou encore un hydrate ou un solvate du dudit composé.

### Composition pharmaceutique

Selon un troisième aspect, l'invention concerne aussi une composition pharmaceutique comprenant un composé selon l'invention, en combinaison avec un excipient pharmaceutiquement acceptable selon le mode d'administration choisi. La composition pharmaceutique peut se présenter sous forme solide, liquide ou de liposomes.

Parmi les compositions solides, on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions, de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants. Les formes liquides seront de préférence injectables et, de ce fait, auront une formulation acceptable pour une telle utilisation. Des voies d'administration par injection acceptables incluent les voies intraveineuse, intra-péritonéale, intramusculaire, et sous cutanée, la voie intraveineuse étant préférée.

La dose administrée des composés de l'invention sera adaptée par le praticien en fonction de la voie d'administration du patient et de l'état de ce dernier.

### Utilisation des composés de l'invention

Selon un quatrième aspect, l'invention concerne l'utilisation d'un composé selon l'invention, pour la fabrication d'un médicament utile pour traiter un état pathologique, de préférence le cancer, ou un état pathologique choisi parmi le psoriasis, le glaucome, les leucémies et les tumeurs solides, les inflammations, et maladies liées à une dérégulation de protéines kinases. La présente invention concerne ainsi des dérivés des pyrazolylbenzimidazoles pour leur utilisation comme agents anticancéreux. Elle concerne également l'utilisation desdits dérivés pour la préparation d'un médicament destiné au traitement de l'homme.

Les composés selon l'invention peuvent donc être utiles dans la prévention et le traitement des leucémies, des tumeurs solides à la fois primaires et métastatiques, des carcinomes et cancers, en particulier : cancer du sein ; cancer du poumon ; cancer de l'intestin grêle, cancer du colon et du rectum ; cancer des voies respiratoires, de l'oropharynx et de l'hypopharynx ; cancer de l'oesophage ; cancer du foie, cancer de l'estomac, cancer des canaux biliaires, cancer de la vésicule biliaire, cancer du pancréas ; cancers des voies urinaires y compris rein, urothélium et vessie; cancers du tractus génital féminin y compris cancer de l'utérus, du col de l'utérus, des ovaires, chloriocarcinome et trophoblastome; cancers du tractus génital masculin y compris cancer de la prostate, des vésicules séminales, des testicules, tumeurs des cellules germinales; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs malignes hématopoïétiques ; leucémies, (Acute Lymphocytic Leukemia (ALL), Acute Myeloid Leukemia (AML), Chronic Myeloid Leukemia (CML), Chronic lymphocytic leukemia (CLL)) chloromes, plasmocytomes, leucémies des cellules T ou B, lymphomes non hodgkiniens ou hodgkiniens, myélomes, hémopathies malignes diverses.

Les composés de la présente invention peuvent être administrés seuls ou en mélange avec d'autres anticancéreux. Parmi les associations possibles on peut citer :
- les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine ;
- les dérivés du platine comme notamment le cisplatine, le carboplatine ou l'oxaliplatine ;
- les agents antibiotiques comme notamment la bléomycine, la mitomycine, la dactinomycine ;
- les agents antimicrotubules comme notamment la vinblastine, la vincristine, la vindésine, la vinorelbine, les taxoides (paclitaxel et docétaxel) ;
- les anthracyclines comme notamment la doxorubicine, la daunorubicine, l'idarubicine, l'épirubicine, la mitoxantrone, la losoxantrone ;
- les agents inhibiteurs de topoisomérases des groupes I et II telles que l'étoposide, le teniposide, l'amsacrine, l'irinotecan, le topotecan et le tomudex ;
- les fluoropyrimidines telles que le 5-fluorouracile, l'UFT, la floxuridine ;
- les analogues de cytidine telles que la 5-azacytidine, la cytarabine, la gemcitabine, la 6-mercaptomurine, la 6-thioguanine ;
- les analogues d'adénosine telles que la pentostatine, la cytarabine ou le phosphate de fludarabine ;
- le méthotrexate et l'acide folinique ;
- les enzymes et composés divers tels que la L-asparaginase, l'hydroxyurée, l'acide trans-rétinoique, la suramine, la dexrazoxane, l'amifostine, l'herceptin ainsi que les hormones oestrogéniques, androgéniques ;
- les agents antivasculaires tels que les dérivés de la combretastatine ou de la colchicine et leurs prodrogues.

Il est également possible d'associer aux composés de la présente invention un traitement par des radiations. Ces traitements peuvent être administrés simultanément, séparément, séquentiellement. Le traitement sera adapté par le praticien en fonction du malade à traiter.

Les produits selon l'invention présentent une forte activité cellulaire dans un test d'inhibition de viabilité/prolifération des cellules tumorales (lignée HeLa) et une grande sélectivité vis-à-vis des lymphocytes du sang périphérique (PBL) quiescents, utilisés comme modèle de cellules quiescentes.

### Procédé d'obtention des composés selon l'invention

Un procédé de préparation des composés de formule générale (la) ou (Ib) peut-être schématisé de la façon générale suivante :

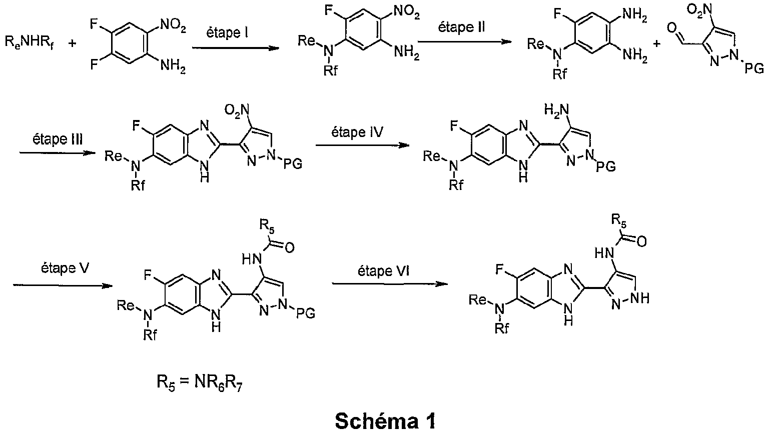

L'étape l'en remplacement de l'étape I permet de préparer de façon similaire des dérivés alkoxy par réaction du dérivé fluoré avec un alcoolate RₑO⁻. Celui-ci peut être préparé *in situ* par réaction avec une base telle que l'hydrure de sodium dans un solvant comme le DMF selon le schéma 2 :

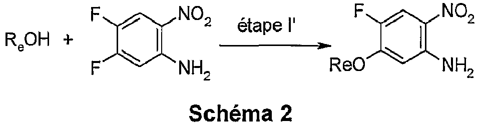

L'invention a donc également pour objet un procédé de préparation des composés de formule (I) selon le schéma 3 ci-dessous :

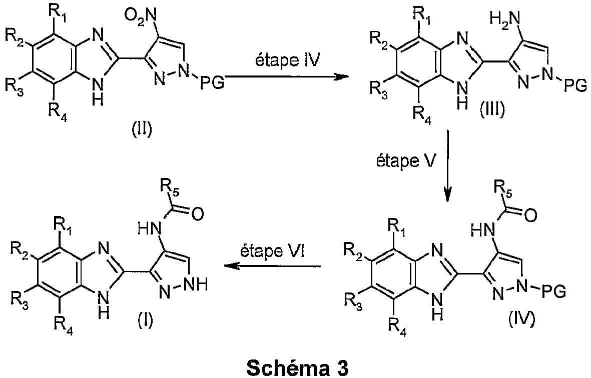

Dans les schémas 1 et 3, PG désigne un groupement protecteur qui a pour fonction d'éviter les réactions non désirées sur le cycle pyrazole lors d'une ou de plusieurs des étape(s) réactionnelles. On trouvera des exemples de groupements protecteurs dans T.W. Greene et coll. dans « Protective Groups in Organic Synthesis », third edition, 1999, Wiley-Interscience ou encore dans J.F.W. McOmie dans « Protective Groups in Organic Chemistry », Plenum Press, 1973. De manière préférée, le groupement protecteur est le suivant :

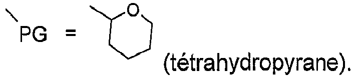

De manière générale, l'étape IV correspond à la réduction du groupement nitro porté par le cycle pyrazole. Elle peut se faire par hydrogénation catalytique dans un solvant tel que par exemple l'acétate d'éthyle en présence d'un catalyseur (par exemple au palladium). La fonctionnalisation de cette amine lors de l'étape V peut se faire dans un solvant (comme le THF), en présence d'un agent acylant permettant d'introduire le motif R₅CO-, par exemple d'un chlorure de carbamoyle R₅COCl, et éventuellement en présence d'une base (par ex. la N,N-diisopropyléthylamine). Une autre voie consiste à former un intermédiaire carbamate puis déplacement par une amine R₆NHR₇. L'étape VI correspond à une étape de déprotection dont les conditions dépendent de la nature du groupement protecteur. En présence d'un tétrahydropyrane, cette étape peut être réalisée en milieu acide concentré.

L'invention a donc pour objet un procédé de préparation dans lequel on soumet le composé de formule (II) à une réaction de réduction afin d'obtenir un dérivé aminé de formule (III). Ce dérivé est ensuite acylé en présence de chlorure de carbamoyle et éventuellement d'une base (telle que la N,N-diisopropyléthylamine), ou bien par formation d'un intermédiaire carbamate puis déplacement par une amine afin d'obtenir le pyrazole de formule (IV). L'étape suivante de déprotection permet de récupérer le composé de formule (I) et est connue de l'homme du métier. Ainsi, la déprotection peut être réalisée en milieu acide concentré dans le cas d'un tétrahydropyrane. Le composé de formule (I) est ensuite le cas échéant salifié.

Le composé de formule (II) est obtenu selon la méthode suivante. De manière générale, lors de l'étape I, le précurseur de la diamine aromatique nécessaire à la formation du cycle benzimidazole est formé par substitution nucléophile sélective d'un atome de fluor du 4,5-difluoro-2-nitroaniline par une amine RₑNHR_{f} dans un solvant tel que le DMF en présence d'une base telle que le bicarbonate de sodium. Lors de l'étape II, le groupement nitro aromatique est réduit. Cette réaction peut être conduite en présence d'hydrogène et d'un catalyseur au palladium. L'étape III de formation du cycle benzimidazole se fait par condensation de la diamine obtenue avec un aldéhyde convenablement fonctionnalisé dans un solvant tel que le DMF ou le méthanol éventuellement en présence d'un catalyseur, tel que par exemple le trichlorure de fer.

L'invention a également pour objet les composés intermédiaires de formule (II), (III) et (IV) tels que définis plus haut, de préférence avec le groupement protecteur tétrahydropyrane défini précédemment et selon toutes les limitations décrites pour R₁ à R₅ pour les composés de formule (I).

### [Exemples]

L'invention a aussi pour objet les composés tels que préparés dans les exemples ci-dessous.

### Analyses LC/MS

Les analyses LC/MS ont été réalisées sur un appareil Micromass modèle LCT relié à un appareil HP 1100. L'abondance des produits a été mesurée à l'aide d'un détecteur à barrette de diodes HP G1315A sur une gamme d'onde de 200-600 nm et un détecteur à dispersion de lumière Sedex 65. L'acquisition des spectres de masses Mass spectra a été réalisée sur une gamme de 180 à 800 nm. Les données ont été analysées en utilisant le logiciel Micromass MassLynx. La séparation a été effectuée sur une colonne Hypersil BDS C18, 3 µm (50x4,6 mm), en éluant par un gradient linéaire de 5 à 90% d'acétonitrile contenant 0,05 % (v/v) d'acide formique dans l'eau contenant 0,05 % (v/v) d'acide formique en 3,5 mn à un débit de 1 mL/mn. Le temps total d'analyse, incluant la période de rééquilibrage de la colonne, est de 7 minutes.

Purification par chromatographie flash : les produits bruts sont purifiés par chromatographie flash sur cartouches de silice de granulométrie 50 µm. Les fractions correspondant au produit attendu sont rassemblées et concentrées sous pression réduite à l'évaporateur rotatif.

### Analyses RMN¹H

Spectre RMN ¹H à 400 MHz sur spectromètre BRUKER AVANCE DRX-400 avec les déplacements chimiques (δ en ppm) - dans le DMSO-d6 référencé à 2,50 ppm à la température de 303K le cas échéant après addition d'une goutte de TFA-d1 (CF₃COOD-d1) et d'une goutte d'acide acétique-d4-(CD₃OD-d4)

Spectre RMN ¹H à 300 MHz sur spectromètre BRUKER AVANCE DPX-300 avec les déplacements chimiques (δ en ppm) - dans le DMSO-d6 référencé à 2,50 ppm à la température de 303K le cas échéant après addition d'une goutte de TFA-d1 (acide trifluoroacétique CF₃COOD-d1) et d'une goutte d'acide acétique-d4-(CD₃OD-d4)

Les spectres de masse ont été réalisés en impact électronique sur un spectromètre Finnigan SSQ 7000 et en électrospray sur un Platform II de Micromass (meilleurs résultats obtenus en ionisation positive ou négative selon les structures).

Les composés des exemples ci-dessous sont préparés selon le schéma 1. **Exemple 1** : chlorhydrate de N-[3-(5-fluoro-6-morpholin-4-yl-1H-benzimidazol-2-yl)-1H-pyrazol-4-yl] pipéridine-1-carboxamide,

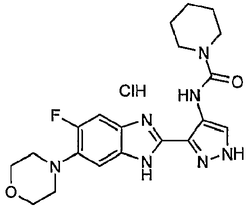

### Etape I : 4-Fluoro-5-morpholin-4-yl-2-nitro-phenylamine

Une solution de 26 g de 4,5-difluoroaniline dans 500 mL de DMF est agitée à 22°C. 39 mL de morpholine et 61,3 g de bicarbonate de sodium sont additionnés. Le milieu réactionnel est chauffé à 80°C à l'aide d'un bain d'huile pendant une heure. Un solide jaune précipite. Le milieu est refroidi à température ambiante puis 800 mL d'eau distillée sont ajoutés. Le milieu est refroidi à l'aide d'un bain de glace. Le solide est filtré, lavé à l'eau puis séché sous vide en présence de potasse jusqu'à poids constant. 35,1 g de 4-Fluoro-5-morpholin-4-yl-2-nitro-phenylamine sont obtenus sous forme de solide jaune. Point de fusion : 190-192°C (banc Kofler). RMN ¹H (400 MHz, (CD₃)₂SO, δ en ppm) : 3,15 (m : 4H) ; 3,73 (m : 4H) ; 6,43 (d, J=8 Hz : 1H) ; 7,37 (s large : 2H) ; 7,66 (d , J=15 Hz : 1H).

### Etape II : 4-Fluoro-5-morpholin-4-yl-benzène-1,2-diamine

35 g de 4-Fluoro-5-morpholin-4-yl-2-nitro-phenylamine en solution dans 618 mL méthanol sont hydrogénés sous 1 bar de pression d'hydrogène en présence de 3,4 g de palladium sur charbon à 22°C pendant 16 heures. Le brut réactionnel est filtré sur célite et le filtrat est concentré sous vide à l'évaporateur rotatif. 30 g de 4-Fluoro-5-morpholin-4-yl-benzene-1,2-diamine sous forme de solide brun sont isolés. RMN ¹H (400 MHz, (CD₃)₂SO, δ en ppm) : 2,78 (m : 4H) ; 3,68 (m : 4H) ; 4,25 (s large : 2H) ; 4,35 (s large : 2H) ; 6,27 (d, J=8 Hz : 1H) ; 6,31 (d , J=14 Hz :2H).

### Etape III: 6-fluoro-5-morpholin-4-yl-2-(4-nitro-1H-pyrazol-3-yl)-1H-benzimidazole

A une solution de 500 mg de 4-Fluoro-5-morpholin-4-yl-benzene-1,2-diamine dans 50 mL de méthanol, 530 mg de 4-Nitro-1-(tetrahydro-pyran-2-yl)-1H-pyrazole-3-carbaldehyde (dont la préparation est décrite dans WO 03/035065**,** référence exemple 6m page 479) sont ajoutés lentement puis 19,5 mg de chlorure ferrique sont ajoutés. Le milieu réactionnel est agité à 22°C pendant 18 heures. Après évaporation, le brut réactionnel est purifié par chromatographie flash sur un appareil Intelliflash sur cartouche RS-90 Analogix. L'élution se fait comme suit : pendant 20 minutes, cyclohexane 100% puis 20 minutes avec un mélange cyclohexane / acétate d'éthyle 80 : 20 et 120 minutes avec un mélange cyclohexane / acétate d'éthyle 20 : 80. 457 mg de 6-Fluoro-5-morpholin-4-yl-2-(4-nitro-1H-pyrazol-3-yl)-1H-benzimidazole sont isolés. RMN ¹H (400 MHz, (CD₃)₂SO, δ en ppm) : 1,42 - 1,80 (m : 3H) ; 1,91-2,06 (m : 2H) ; 2,18 (m : 1 H) ; 3,02 (m : 4H) ; 3,70 (m : 1H) ; 3,78 (m : 4H) ; 4,00 (d large J=10 Hz : 1 H) ; 5,60 (dd, J=10 et 2 Hz : 1 H) ; 7,18 (s très étalé : 1H) ; 7,45 (s très étalé : 1 H) ; 9,18 (s : 1 H) ; 12,85 (s très étalé : 1 H).

### Etape IV : 3-(5-Fluoro-6-morpholin-4-yl-1 H-benzimidazol-2-yl)-1-(tetrahydro-pyran-2-yl)-1H-pyrazol-4-ylamine (Intermédiaire Gamma)

Une suspension de 12 g de 6-Fluoro-5-morpholin-4-yl-2-(4-nitro-1H-pyrazol-3-yl)-1H-benzimidazole dans 200 mL de méthanol et 1 g de palladium sur charbon est hydrogénée sous 1 bar de pression d'hydrogène à température ambiante pendant 18 heures. Le milieu réactionnel est filtré sur célite et le filtrat concentré sous pression réduite à l'évaporateur rotatif. 10,6 g de 3-(5-Fluoro-6-morpholin-4-yl-1H-benzimidazol-2-yl)-1-(tetrahydro-pyran-2-yl)-1 H-pyrazol-4-ylamine sont obtenus sous forme de solide noir. LC/MS analytique : Tr=4,79 min, [M+H]⁺ = 387,12 DAD = 38%

### Etape V : Piperidine-1-carboxylic acid [3-(5-fluoro-6-morpholin-4-yl-1H-benzimidazol-2-yl)-1-(tetrahydro-pyran-2-yl)-1H-pyrazol-4-yl]-amide

A une solution de 10 g 3-(5-Fluoro-6-morpholin-4-yl-1H-benzimidazol-2-yl)-1-(tetrahydro-pyran-2-yl)-1 H-pyrazol-4-ylamine dans 200 mL de THF, 10 mL de chlorure de piperidinecarbonyle et 13,55 mL de N,N-diisopropyléthylamine sont additionnés. Le mélange réactionnel est chauffé à reflux pendant 3 heures puis laissé sous agitation à température ambiante pendant 48 heures. Après concentration du milieu sous vide à l'évaporateur rotatif, le brut réactionnel est purifié par chromatographie flash sur 1200 g de silice 40-63 µM avec pour éluant un mélange dichlorométhane / acétone 90 : 10,4 g de Pipéridine-1-carboxylic acid [3-(5-fluoro-6-morpholin-4-yl-1H-benzimidazol-2-yl)-1-(tetrahydro-pyran-2-yl)-1H-pyrazol-4-yl]-amide sont isolés. RMN ¹H (300 MHz, (CD₃)₂S, δ en ppm) :1,50-1,79 (m : 10H) ; 1,91-2,04 (m : 2H) ; 3,00 (m : 4H) ; 3,50 (m : 4H) ; 3,66 (m : 1H) ; 3,77 (m : 4H) ; 3,96 (d large J=11 Hz : 1H) ; 5,46 (dd, J=10 et 2 Hz : 1H) ; 7,10 (s très étalé : 1H) ; 7,40 (s très étalé : 1 H) ; 8,13 (s : 1 H) ; 9,96 (s large : 1H) ; 13,00 (s très étalé : 1H).

### Etape VI : Piperidine-1-carboxylic acid [3-(5-fluoro-6-morpholin-4-yl-1H-benzimidazol-2-yl)-1 H-pyrazol-4-yl]-amide, sous forme de chlorhydrate

Une solution de 4 g de Piperidine-1-carboxylic acid [3-(5-fluoro-6-morpholin-4-yl-1H-benzimidazol-2-yl)-1-(tetrahydro-pyran-2-yl)-1H-pyrazol-4-yl]-amide en solution dans 50 mL d'une solution d'acide chlorhydrique 4N dans le dioxanne est agitée à 22 °C pendant 4 heures. Afin que la réaction soit totale, le milieu réactionnel est concentré et à nouveau dissout dans 50 mL d'une solution d'acide chlorhydrique 4N dans le dioxanne et laissé sous agitation à température ambiante pendant 18 heures. La suspension est fitrée sur verre fritté et le solide est lavé avec du dioxanne. Après essorage, le solide est trituré dans l'éther isopropylique. Le solide obtenu étant hygroscopique, il est repris dans le méthanol et la solution est concentrée sous vide. L'huile obtenue est purifiée par chromatographie flash sur appareil Intelliflash sur une cartouche RS-120 analogix. L'élution se fait avec du dichlorométhane puis de l'acétate d'éthyle 100%, puis avec un mélange dichlorométhane / méthanol 95 / 5. Après concentration des fractions contenant le produit, celui-ci est trituré dans l'éther isopropylique et le solide obtenu est filtré et séché sous vide jusqu'à poids constant. 3.,2 g de piperidine-1-carboxylic acid [3-(5-fluoro-6-morpholin-4-yl-1 H-benzimidazol-2-yl)-1H-pyrazol-4-yl]-amides sont obtenus sous forme de chlorhydrate. RMN ¹H (300 MHz, (CD₃)₂SO, δ en ppm) : 1,48-1,67 (m : 6H) ; 3,03 (m : 4H) ; 3,48 (m : 4H) ; 3,78 (m : 4H) ; 7,18 (d, J=8 Hz : 1H) ; 7,46 (d, J=12 Hz : 1H) ; 8,01 (s : 1H) ; 9,53 (s large : 1 H) ; 12,50 (s très étalé : 1 H).

### Exemple 2: Trifluoroacétate de 1,1-Diethyl-3-[3-(5-fluoro-6-morpholin-4-yl-1H-benzimidazol-2-yl)-1 H-pyrazol-4-yl]-urée

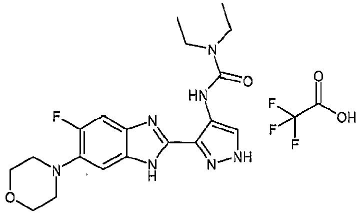

### Etape V : 1,1-diéthyl-3-[3-(5-fluoro-6-morpholin-4-yl-1H-benzimidazol-2-yl)-1-(tetrahydro-pyran-2-yl)-1H-pyrazol-4-yl]-urée

100 mg **d'Intermédiaire Gamma** sont mis en solution dans 10 mL de tétrahydrofuranne puis on ajoute 214 µL de N, N-diisopropyléthylamine et 1.2 mL de chlorure de diéthylcarbamoyle. Le milieu réactionnel est agité à 50°C pendant une nuit. La phase organique est lavée par 2 x 10 mL d'une solution saturée de bicarbonate de sodium. Les phases aqueuses sont extraites par 3x15 mL d'acétate d'éthyle. Les phases organiques rassemblées sont séchées sur sulfate de magnésium, filtrées et concentrées sous vide à l'évaporateur rotatif. Le brut réactionnel est purifié sur cartouche de 10 g de silice, éluant : AcOEt/cyclohexane 1/1. 95 mg de 1,1-diéthyl-3-[3-(5-fluoro-6-morpholin-4-yl-1H-benzimidazol-2-yl)-1-(tetrahydro-pyran-2-yl)-1H-pyrazol-4-yl]-urea sont isolés sous forme de mousse jaune. RMN ¹H (400 MHz, DMSO-d6, δ en ppm) : 1,23 (t, J=7,0 Hz, 6H) ; de 1,51 à 1,81 (m, 3H) ; 1,98 (m, 2H) ; 2,13 (m, 1H) ; 3,00 (m, 4H) ; 3,41 (q, J=7,0 Hz, 4H) ; 3,66 (m, 1H) ; 3,77 (m, 4H) ; 3,96 (m, 1 H) ; 5,46 (dd, J=2,0 et 10,0 Hz, 1 H) ; de 6,96 à 7,47 (m étalé, 2H) ; 8,12 (s, 1 H) ; 9,88 (m étalé, 1H) ; 13,0 (m étalé, 1H) .

### Etape VI : 1,1-Diéthyl-3-[3-(5-fluoro-6-morpholin-4-yl-1H-benzimidazol-2-yl)-1H-pyrazol-4-yl]-urée

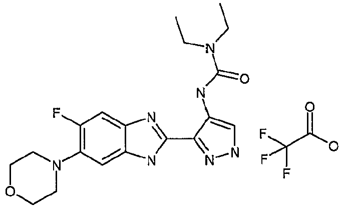

Une solution de 95 mg de 1,1-diéthyl-3-[3-(5-fluoro-6-morpholin-4-yl-1H-benzimidazol-2-yl)-1-(tetrahydro-pyran-2-yl)-1H-pyrazol-4-yl]-urea dans 3 mL de dichlorométhane, à laquelle on ajoute 750 µL d'acide trifluoroacétique, est agitée à température ambiante pendant 48 heures. Le milieu réactionnel est concentré à sec puis purifié par LC/MS préparative : injection du produit dans 2,5 mL de DMSO, élution par un gradient de 5% à 95% d'acétonitrile dans l'eau contenant respectivement 0,07% de TFA en 12 minutes. 49 mg de 1,1-Diethyl-3-[3-(5-fluoro-6-morpholin-4-yl-1H-benzimidazol-2-yl)-1H-pyrazol-4-yl]-urea sont isolés sous forme d'un sel de trifluoroacétate. RMN ¹H (300MHz, DMSO-d6, δ en ppm) : 1,23 (t, J = 7,0 Hz, 6H) ; 3,00 (m, 4H) ; 3,41 (m partiellement masqué, 4H) ; 3,78 (m, 4H) ; 7,11 (d large, J = 8,0 Hz, 1H) ; 7,33 (d large, J=12,0 Hz, 1 H) ; 7,99 (s, 1H) ; 9,78 (s, 1 H) ; 13,0 (m étalé, 2H).

### Exemple 3 : Chlorhydrate de N-{3-[5-fluoro-6-(2-piperidin-1-yl-ethoxy)-1H-benzimidazol-2-yl]-1H-pyrazol-4-yl} pipéridine-1-carboxamide

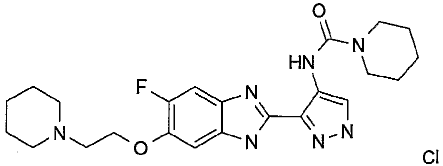

### Etape I : 4-Fluoro-2-nitro-5-(2-piperidin-1-yl-ethoxy)-phenylamine

Une solution de 1.11 g de 2-Pipéridin-1-yl-ethanol dans 20 ml de N, N-diméthylformamide (DMF) est refroidie à 0°C par un bain de glace. 689 mg d'hydrure de sodium (60% en suspension dans l'huile) sont additionnés par petites portions. La suspension obtenue est additionnée goutte à goutte sur une suspension contenant 500 mg de 4,5-difluoro-2-nitro-phénylamine et 724 mg de bicarbonate de sodium dans 15 ml de DMF. Le milieu réactionnel est agité à température ambiante (22°C) pendant 2 heures puis chauffé pendant 1 heure à 90°C. Le solvant est concentré à sec puis le brut réactionnel est repris dans 30 ml d'acétate d'éthyle et la phase organique est lavée par 2x30 ml d'eau distillée et 1x30 ml d'une solution aqueuse saturée de chlorure de sodium. La phase organique est séchée sur sulfate de magnésium et le solvant est évaporé sous vide à l'évaporateur rotatif. Le brut est trituré dans l'éther éthylique et le solide est filtré sur verre fritté et rincé à l'éther éthylique. 443 mg de 4-fluoro-2-nitro-5-(2-pipéridin-1-yl-éthoxy)-phénylamine sous forme d'un solide rouge sont isolés. RMN¹H (300 MHz, DMSO-d6, δ en ppm) : 1,37 (m, 2H) ; 1,48 (m, 4H) ; 2,43 (m, 4H) ; 2,69 (t, J = 6,5 Hz, 2H) ; 4,13 (t, J=6,5 Hz, 2H) ; 6,65 (d, J = 8,0 Hz, 1 H) ; 7,48 (s large, 2H) ; 7,75 (d, J = 12,0 Hz, 1H).

### Etape II : 4-Fluoro-5-(2-piperidin-1-yl-ethoxy)-benzene-1,2-diamine

443 mg de 4-Fluoro-2-nitro-5-(2-piperidin-1-yl-ethoxy)-phénylamine en solution dans 20 mL méthanol sont hydrogénés sous 1 bar de pression d'hydrogène en présence de 44 mg de palladium sur charbon à 25°C pendant 10 heures. Le brut réactionnel est filtré sur célite et le filtrat est concentré sous vide à l'évaporateur rotatif. 380 mg de 4-Fluoro-5-(2-pipéridin-1-yl-ethoxy)benzène-1,2-diamine sous forme d'une laque noire. Le composé est utilisé sans autre purification.

### Etape III : 5-Fluoro-2-[4-nitro-1-(tetrahydro-pyran-2-yl)-1H-pyrazol-3-yl]-6-(2-piperidin-1-yl-ethoxy)-1H-benzimidazole

Une solution de 248 mg 4-Nitro-1-(tétrahydro-pyran-2-yl)-1 H-pyrazole-3-carbaldehyde, 278,9 mg de 4-fluoro-5-(2-piperidin-1-yl-ethoxy)benzène-1,2-diamine dans 12 mL de méthanol est agitée à température ambiante pendant une nuit. Afin que la réaction soit complète on chauffe une demi-heure supplémentaire à reflux puis une nuit à température ambiante. Après concentration sous vide à l'évaporateur rotatif, 515 mg de 5-Fluoro-2-[4-nitro-1-(tétrahydro-pyran-2-yl)-1 H-pyrazol-3-yl]-6-(2-pipéridin-1-yl-ethoxy)-1H-benzimidazole sont isolés sous forme d'un solide foncé. RMN¹H (300MHz, DMSO-d6, δ en ppm) : pour ce lot, nous observons un dédoublement 60%- 40% de tautomères avec : de 1,31 à 1,78 (m, 9H) ; de 1,87 à 2,30 (m, 3H) ; 2,46 (m partiellement masqué, 4H) ; 2,72 (t, J=6,0 Hz, 2H) ; 3,69 (m, 1 H) ; 4,00 (m, 1 H) ; 4,19 (t, J=6,0 Hz, 2H) ; 5,60 (dd, J=2,0 et 9,0 Hz, 1H) ; 7,26 (d, J=7,5 Hz, 0,6H) ; 7,41 (d, J=11,5 Hz, 0,4H) ; 7,45 (d, J=7,5 Hz, 0,4H) ; 7,53 (d, J=11,5 Hz, 0,6H) ; 9,18 (s, 1 H) ; 12,85 (m large, 1H).

### Etape IV : 3-[5-Fluoro-6-(2-piperidin-1-yl-ethoxy)-1H-benzimidazol-2-yl]-1-(tetrahydro-pyran-2-yl)-1H-pyrazol-4-ylamine (Intermédiaire Alpha)

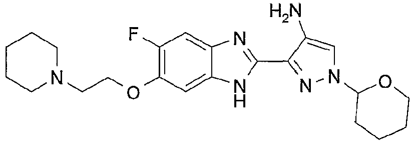

Une suspension de 160 mg de 5-Fluoro-2-[4-nitro-1-(tetrahydro-pyran-2-yl)-1H-pyrazol-3-yl]-6-(2-piperidin-1-yl-éthoxy)-1H-benzimidazole dans 10 mL d'acétate d'éthyle et 16 mg de palladium sur charbon est hydrogénée sous 1 bar de pression d'hydrogène à température ambiante pendant 16 heures. Le milieu réactionnel est filtré sur célite et le filtrat concentré sous pression réduite à l'évaporateur rotatif. 150 mg de 3-[5-Fluoro-6-(2-piperidin-1-yl-éthoxy)-1H-benzimidazol-2-yl]-1-(tetrahydro-pyran-2-yl)-1H-pyrazol-4-ylamine sont isolés sous forme d'huile rouge. LC/MS analytique : Tr=2,04 min, [M+H]⁺ = 429,05 DAD = 70%.

### Etape V : Piperidine-1-carboxylic acid [3-[5-fluoro-6-(2-piperidin-1-yl-ethoxy)-1H-benzimidazol-2-yl]-1-(tetrahydro-pyran-2-yl)-1H-pyrazol-4-yl]-amide

Une solution de 155 µl de chlorure de piperidine-1-carbonyle, 60 mg d'intermédiaire Alpha, 200 µl de N,N-diisopropylamine dans 6 ml de tétrahydrofuranne est chauffée à 50°C pendant 18 heures. Le milieu réactionnel est concentré à sec et le brut est purifié par chromatographie flash sur cartouche de silice, éluant : dichlorométhane/méthanol 100/0 à 90/10. 30 mg de Piperidine-1-carboxylic acid [3-[5-fluoro-6-(2-piperidin-1-yl-ethoxy)-1H-benzimidazol-2-yl]-1-(tetrahydro-pyran-2-yl)-1H-pyrazol-4-yl]-amide sont obtenus sous forme d'huile jaunâtre. LC/MS analytique : Tr=2,94 min, [M+H]⁺ = 540,38 DAD = 95%

### Etape VI : Chlorhydrate de N-{3-[5-fluoro-6-(2-piperidin-1-yl-ethoxy)-1H-benzimidazol-2-yl]-1H-pyrazol-4-yl} piperidine-1-carboxamide

Une solution de 30 mg piperidine-1-carboxylic acid [3-[5-fluoro-6-(2-piperidin-1-yl-ethoxy)-1H-benzimidazol-2-yl]-1-(tetrahydro-pyran-2-yl)-1H-pyrazol-4-yl]-amide dans 3 mL d'une solution d'acide chlorhydrique 3N dans le dioxanne est agitée à 22°C pendant 18 heures. Le milieu réactionnel est concentré à sec et le brut est purifié par chromatographie flash sur une cartouche de 2 g de silice, éluant : dichlorométhane/méthanol 90/10 à 70/30. 20 mg de chlorhydrate de N-{3-[5-fluoro-6-(2-piperidin-1-yl-ethoxy)-1H-benzimidazol-2-yl]-1H-pyrazol-4-yl}Piperidine-1-carboxamide sont obtenus sous forme d'un solide ocre. RMN¹H (400 MHz, DMSO-d6, δ en ppm) après addition d'une goutte d'acide acétique-d4-(CD₃OD-d4) et d'une goutte de TFA-d1 (acide trifluoroacétique CF₃COOD-d1) : de 1,32 à 1,88 (m, 12H) ; 3,05 (m, 2H) ; 3,43 (m, 4H) ; 3,57 (m, 4H) ; 4,50 (m, 2H) ; 7,44 (d, J=8,0 Hz, 1H) ; 7,61 (d, J=11,0 Hz, 1H) ; 8,00 (s, 1H). LC/MS analytique : Tr=2,66 min, [M+H]⁺ = 456,32 DAD = 100%

### Exemple 4: trifluoroacétate de 1,1-diethyl-3-{3-[5-fluoro-6-(2-piperidin-1-yl-ethoxy)-1H-benzimidazol-2-yl]-1H-pyrazol-4-yl}-urée

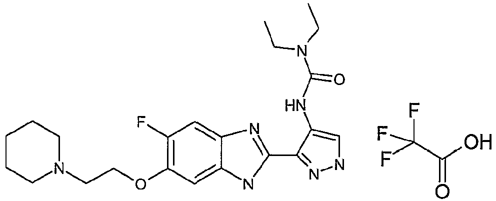

### Etape V :1,1-Diethyl-3-[3-[5-fluoro-6-(2-piperidin-1-yl-ethoxy)-1H-benzimidazol-2-yl]-1-(tetrahydro-pyran-2-yl)-1 H-pyrazol-4-yl]-urea

140 mg d'**intermédiaire Alpha** sont mis en solution dans 10 mL de tétrahydrofuranne, puis 414 µL de chlorure de diéthylcarbamoyle et 542 µL de N, N-diisopropyléthylamine sont additionnés. Le milieu réactionnel est agité 48 heures à 56°C. La phase organique est lavée par 2x15 mL d'une solution aqueuse saturée de bicarbonate de sodium. La phase aqueuse est extraite par 3x15 mL d'acétate d'éthyle. Les phases organiques rassemblées sont séchées sur sulfate de magnésium, filtrées et le solvant est évaporé sous vide à l'évaporateur rotatif. Le brut réactionnel est purifié par chromatographie flash sur une cartouche de 40 g de silice, éluant : dichlorométhane/méthanol 100/0 à 85/15. Les fractions contenant le produits sont rassemblées et concentrées sous vide à l'évaporateur rotatif. 38 mg de 1,1-Diethyl-3-[3-[5-fluoro-6-(2-piperidin-1-yl-ethoxy)-1H-benzimidazol-2-yl]-1-(tetrahydro-pyran-2-yl)-1H-pyrazol-4-yl]-urea sont isolés. RMN¹H (400 MHz, DMSO-d6, δ en ppm), pour ce lot, nous observons le sel de la structure attendue avec un dédoublement 60%-40% de tautomères : 1,23 (m, 6H) ; de 1,31 à 2,18 (m, 12H) ; de 2,99 à 3,75 (m étalé, 6H) ; 3,42 (m, 4H) ; 3,66 (m, 1H) ; 3,96 (m, 1 H) ; de 4,12 à 4,58 (m étalé, 2H) ; 5,46 (d large, J=10,0 Hz, 1H) ; de 7,10 à 7,55 (m, 2H) ; 8,14 (s, 1H) ; 9,69 (m étalé, 1H) ; 9,79 (s, 0,4H) ; 9,82 (s, 0,6H) ; 13,1 (m étalé, 1H).

### Etape VI: Trifluoroacétate de 1,1-diéthyl-3-{3-[5-fluoro-6-(2-pipéridin-1-yl-ethoxy)-1H-benzimidazol-2-yl]-1H-pyrazol-4-yl}-urée

Une solution de 38 mg de 1,1-Diethyl-3-[3-[5-fluoro-6-(2-piperidin-1-yl-ethoxy)-1H-benzimidazol-2-yl]-1-(tetrahydro-pyran-2-yl)-1H-pyrazol-4-yl]-urea est mis en solution dans 2 mL de dichlorométhane puis on ajoute 500 µL d'acide trifluoroacétique. Le milieu réactionnel est agité à température ambiante pendant une nuit. Le solvant est évaporé sous vide à l'évaporateur rotatif et le brut est remis en réaction dans 500 µL de dichlorométhane et 500 µL d'acide trifluoroacétique à température ambiante pendant une heure. Après concentration du solvant à l'évaporateur rotatif, le brut réactionnel est purifié par chromatographie flash sur cartouche de 4 g de silice, éluant : dichlorométhane/méthanol 100/0 à 90/10. 20 mg de trifluoroacétate de 1,1-Diethy)-3-{3-[5-fluoro-6-(2-piperidin-1-y)-ethoxy)-1H-benzimidazol-2-yl]-1H-pyrazol-4-yl}-urée sont isolés sous forme de solide jaune. RMN¹H (400MHz, DMSO-d6, δ en ppm), pour ce lot, nous dédoublement 60% - 40% de tautomères avec : 1,24 (m, 6H) ; de 1,32 à 1,93 (m, 6H) ; 3,06 (m étalé, 2H) ; de 3,23 à 3,68 (m étalé, 4H) ; 3,41 (m, 4H) ; 4,44 (m large, 2H) ; 7,21 (d, J=8,0 Hz, 0,6H) ; 7,32 (d, J=11,0 Hz, 0,4H) ; 7,38 (d, J=8,0 Hz, 0,4H) ; 7,49 (d, J=11,0 Hz, 0,6H) ; 8,01 (s, 1H) ; 9,32 (m étalé, 1H) ; 9,74 (s, 0,4H) ; 9,77 (s, 0,6H) ; 13,0 (s, 1,6H) ; 13,05 (s, 0,4H).

### Exemple 5: Trifluoroacétate de 3-{3-[6-(3-diméthylamino-propoxy)-5-fluoro-1H-benzimidazol-2-yl]-1H-pyrazol-4-yl}-1,1-diéthyl-urée

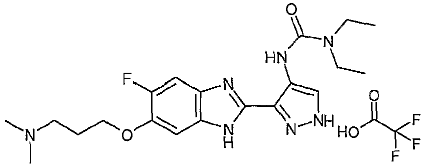

### Etape I : Préparation du 5-(3-dimethylamino-propoxy)-4-fluoro-2-nitro-phenylamine :

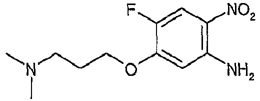

Une solution de 6,1 mL de 3-diméthylamino-1-propanol dans 100 mL de THF anhydre est refroidi à l'aide d'un bain de glace. Sous argon 1,1 g de NaH sont additionnés par petites portions (solution A). Le milieu réactionnel est laissé sous agitation 1 heure à 0°C. Une solution de 3 g de 4,5-difluoroaniline dans 100 mL de THF anhydre est agitée à 22°C. 4,3 g de bicarbonate de sodium sont additionnés. La solution contenant l'alcoolate (solution A) est ajoutée goutte à goutte. Le milieu réactionnel est chauffé à 80°C à l'aide d'un bain d'huile pendant 30 minutes. Le milieu réactionnel est refroidi à température ambiante et on ajoute 100 mL d'eau. La phase aqueuse est extraite avec 3 fois 100 mL d'AcOEt. Les phases organiques sont séchées sur MgSO₄, filtrées et concentrées sous vide à l'évaporateur rotatif. Le solide jaune orangé est repris dans le pentane, trituré, filtré, rincé au pentane et séché. 3,5 g de 5-(3-diméthylamino-propoxy)-4-fluoro-2-nitro-phénylamine sont obtenus sous forme de solide brun. LC/MS analytique :tr=2,16 min; [M+H]⁺ = 258,27; ELSD = 100%. RMN ¹H (400 MHz, DMSO-d6, δ en ppm) : 1,9 (m, J=6,0 Hz, 2H) ; 2,13 (s, 6H) ; 2,35 (t, J=6,0 Hz, 2H) ; 4,08 (t, J=6,0 Hz, 2H) ; 6,64 (d, J=8,0 Hz, 1H) ; 7,47 (s, 2H) ; 7,73 (d, J=11,0 Hz, 1H).

### Etape II : 4-(3-diméthylamino-propoxy)-5-fluoro-benzène-1,2-diamine

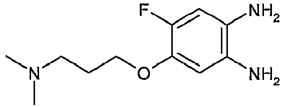

3,8 g de 5-(3-diméthylamino-propoxy)-4-fluoro-2-nitro-phénylamine en solution dans 100 mL de méthanol sont hydrogénés sous 1 bar de pression d'hydrogène en présence de 400 mg de palladium sur charbon à 25°C pendant 16 heures. Le brut réactionnel est filtré sur célite et le filtrat est concentré sous vide à l'évaporateur rotatif. 3,5 g de 4-(3-Dimethylamino-propoxy)-5-fluoro-benzene-1,2-diamine sous forme d'une huile noire sont isolés. RMN ¹H (400 MHz, DMSO-d6, δ en ppm) : 1,75 (m, 2H) ; 2,13 (m, 6H) ; 2,32 (m, 2H) ; 3,84 (m, 2H) ; 4,3 (m, 4H) ; 6,32 (m, 2H).

### Etape III : Préparation du (3-{6-Fluoro-2-[4-nitro-1-(tetrahydro-pyran-2-yl)-1H-pyrazol-3-yl]-3H-benzimidazol-5-yloxy}-propyl)-diméthyl-amine

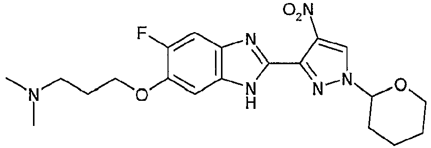

A une solution de 3,5 g de 4-(3-Dimethylamino-propoxy)-5-fluoro-benzene-1,2-diamine dans 150 mL de méthanol, 3,4 g de 4-Nitro-1-(tetrahydro-pyran-2-yl)-1H-pyrazole-3-carbaldéhyde sont ajoutés. Le milieu réactionnel est agité à 22°C pendant 18 heures. Après évaporation, le brut réactionnel est purifié par chromatographie flash sur un appareil Intelliflash sur cartouche RS-330 Analogix. L'élution se fait dans le dichlorométhane à 10% de méthanol. 2,3 g de (3-{6-Fluoro-2-[4-nitro-1-(tetrahydro-pyran-2-yl)-1H-pyrazol-3-yl]-3H-benzimidazol-5-yloxy}-propyl)-dimethyl-amine sont isolés. RMN ¹H (300 MHz, DMSO-d6, δ en ppm) : 1,50 à 2,23 (m, 8H) ; 2,80 (s, 6H) ; 3,23 (m, 2H) ; 3,68 (m, 1H) ; 3,98 (m, 1 H) ; 4,17 (t, J=6,0 Hz, 2H) ; 5,58 (dd, J=2,5 et 9,5 Hz, 1H) ; 7,34 (d, J=8,0 Hz, 1H) ; 7,49 (d, J=11,0 Hz, 1H) ; 9,11 (s, 1 H).

### Etape IV : 3-[6-(3-diméthylamino-propoxy)-5-fluoro-1H-benzimidazol-2-yl]-1-(tetrahydro-pyran-2-yl)-1H-pyrazol-4-ylamine (intermédiaire 1)

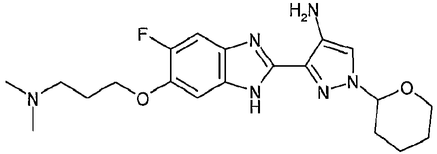

Une suspension de 2,3 g de (3-{6-Fluoro-2-[4-nitro-1-(tetrahydro-pyran-2-yl)-1H-pyrazol-3-yl]-3H-benzimidazol-5-yloxy}-propyl)-diméthyl-amine dans 40 mL de méthanol et 0,2 g de palladium sur charbon est hydrogénée sous 1 bar de pression d'hydrogène à température ambiante pendant 18 heures. Le milieu réactionnel est filtré sur célite et le filtrat concentré sous pression réduite à l'évaporateur rotatif. 2,0 g de 3-[6-(3-Diméthylamino-propoxy)-5-fluoro-1H-benzimidazol-2-yl]-1-(tetrahydro-pyran-2-yl)-1H-pyrazol-4-ylamine sont obtenus sous forme d'un solide brun. RMN ¹H (300 MHz, DMSO-d6, δ en ppm) : 1,46 à 2,21 (m, 8H) ; 2,17 (s, 6H) ; 2,40 (t, J=7,0 Hz, 2H) ; 3,63 (m, 1H) ; 3,95 (m, 1H) ; 4,08 (m, 2H) ; 4,92 (s large, 2H) ; 5,32 (dd, J=2,5 et 10,0 Hz, 1H) ; de 7,01 à 7,47 (m, 2H) ; 7,31 (s, 1H) ; 12,55 (m, 1H).

### Etape V : Préparation du 3-[3-[6-(3-diméthylamino-propoxy)-5-fluoro-1H-benzimidazol-2-yl]-1-(tetrahydro-pyran-2-yl)-1 H-pyrazol-4-yl]-1,1-diethyl-urea

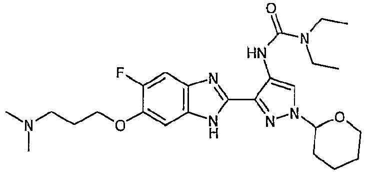

A une solution de 0,9 g d'**intermédiaire 1** dans 30 mL de THF, 1,4 mL de chlorure de diethylcarbonyle et 1,8 mL de N,N-diisopropyléthylamine sont additionnés. Le mélange réactionnel est chauffé à 52°C pendant 24 heures. Le milieu réactionnel est refroidi à température ambiante et lavé avec 30 mL d'une solution saturée de bicarbonate de sodium. La phase aqueuse est extraite avec 3 fois 30 mL d'AcOEt. Les phases organiques sont séchées sur MgSO₄, filtrées et concentrées sous vide à l'évaporateur rotatif. 1,4 g de 3-[3-[6-(3-Dimethylamino-propoxy)-5-fluoro-1H-benzimidazol-2-yl]-1-(tetrahydro-pyran-2-yl)-1H-pyrazol-4-yl]-1,1-diethyl-urea sont obtenus sous forme d'une huile brune. RMN ¹H (300 MHz, DMSO-d6, δ en ppm) : 1,22 (t, J=7,5 Hz, 6H) ; de 1,50 à 2,22 (m, 8H) ; 2,85 (s, 6H) ; 3,27 (m, 2H) ; de 3,31 à 3,48 (m, 4H) ; 3,67 (m, 1H) ; 3,97 (m, 1 H) ; 4,17 (t, J=6,0 Hz, 2H) ; 5,47 (dd, J=2,5 et 10,0 Hz, 1 H) ; 7,22 (d, J=8,0 Hz, 1 H) ; 7,44 (d, J=11,0 Hz, 1 H) ; 8,14 (s, 1 H).

### Etape VI : Trifluoroacétate de 3-{3-[6-(3-Dimethylamino-propoxy)-5-fluoro-1H-benzimidazol-2-yl]-1H-pyrazol-4-yl}-1,1-diethyl-urée

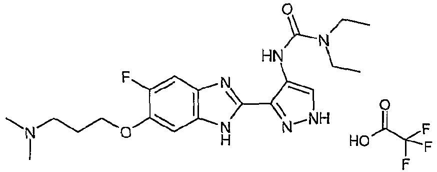

Une solution de 1,5 g de 3-[3-[6-(3-Dimethylamino-propoxy)-5-fluoro-1H-benzimidazol-2-yl]-1-(tetrahydro-pyran-2-yl)-1H-pyrazol-4-yl]-1,1-diethyl-urea en solution dans 30 mL d'une solution d'acide chlorhydrique 4N dans le dioxanne est agitée à 22 °C pendant 4 heures. Un solide précipite. Le solide est filtré, lavé au dioxanne puis séché à l'étuve sous vide à 40°C jusqu'à poids constant. Le précipité obtenu est purifié par HPLC préparative en utilisant des éluants contenant respectivement 0,05 % d'acide acétique. Après concentration des fractions contenant le produit, celui-ci est séché sous vide jusqu'à poids constant. 420 mg de 3-[3-[6-(3-Dimethylamino-propoxy)-5-fluoro-1H-benzimidazol-2-yl]-1-(tetrahydro-pyran-2-yl)-1H-pyrazol-4-yl]-1,1-diethyl-urée sont obtenus sous forme de sel mixte 60% acétate et 40% chlorhydrate.

Pour ce lot, nous avons une salification partielle en acétate soit 2 moles de produit attendu pour une mole d'acétate dans la mesure où nous localisons : RMN ¹H (300 MHz, DMSO-d6, δ en ppm) : 1,19 (t, J=7,0 Hz, 6H) ; 1,90 (s, 1,5H) ; 2,17 (m, 2H) ; 2,84 (s, 6H) ; 3,27 (m, 2H) ; 3,39 (q, J=7,0 Hz, 4H) ; 4,18 (t, J=6,0 Hz, 2H) ; 7,27 (d, J=8,0 Hz, 1H) ; 7,48 (d, J=11,0 Hz, 1H) ; 8,00 (s, 1H).

### Exemple 6: Chlorhydrate de 1,1-Diethyl-3-{3-[5-fluoro-6-(3-piperidin-1-yl-propoxy)-1H-benzimidazol-2-yl]-1H-pyrazol-4-yl}-urée

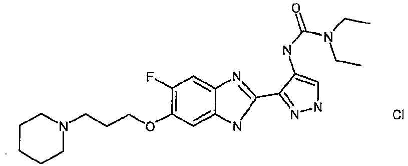

### Etape I : Préparation de 4-Fluoro-2-nitro-5-(3-piperidin-1-yl-propoxy)-phenylamine

Une solution de 1,3 mL de 3-Piperidin-1-yl-propan-1-ol dans 16 ml de N, N-diméthylformamide (DMF) est refroidie à 0°C par un bain de glace. 345 mg d'hydrure de sodium (60% en suspension dans l'huile) sont additionnés par petites portions. La suspension obtenue est additionnée goutte à goutte sur une suspension contenant 500 mg de 4,5-difluoro-2-nitro-phenylamine et 724 mg de bicarbonate de sodium dans 15 ml de DMF. Le milieu réactionnel est agité une heure à température ambiante puis chauffé pendant 1 heure à 90°C. Le milieu réactionnel refroidi est est traité par 70 mL d'acétate d'éthyle et 70 mL d'eau distillée. La phase aqueuse est extraite par 70 mL d'acétate d'éthyle. Les phases organiques réunies sont lavées par 100 mL d'eau distillée, 100 mL d'une solution aqueuse saturée de chlorure de sodium et séchée sur sulfate de magnésium. Le solvant est concentré à sec à l'évaporateur rotatif puis le brut est purifié par chromatographie sur cartouche de 50 g de silice, éluant : dichlorométhane/méthanol 100/0 à 80/20. 300 mg de 4-Fluoro-2-nitro-5-(3-piperidin-1-yl-propoxy)-phénylamine sont isolés sous forme d'huile jaune qui cristallise. LC/MS analytique : Tr=2,26 min, [M+H]⁺ = 298,04 DAD = 87%

### Etape II : Préparation de 4-Fluoro-5-(3-piperidin-1-yl-propoxy)-benzene-1,2-diamine

200 mg de 4-Fluoro-2-nitro-5-(3-piperidin-1-yl-propoxy)-phénylamine en solution dans 15 mL de méthanol sont hydrogénés sous 1 bar de pression d'hydrogène en présence de 20 mg de palladium sur charbon à 25°C pendant 10 heures. Le brut réactionnel est filtré sur célite et le filtrat est concentré sous vide à l'évaporateur rotatif. 180 mg de 4-Fluoro-5-(3-piperidin-1-yl-propoxy)-benzène-1,2-diamine sous forme d'une huile noire. Le produit est utilisé sans autre purification.

### Etape III : Préparation de 5-Fluoro-2-[4-nitro-1-(tetrahydro-pyran-2-yl)-1H-pyrazol-3-yl]-6-(3-piperidin-1-yl-propoxy)-1H-benzimidazole

Une solution de 200 mg 4-fluoro-5-(3-piperidin-1-yl-propoxy)-benzene-1,2-diamine et de 181 mg de 4-Nitro-1-(tetrahydro-pyran-2-yl)-1H-pyrazole-3-carbaldehyde dans 15 mL de méthanol est agitée à température ambiante pendant une nuit. Le milieu réactionnel est concentré à sec sous vide à l'évaporateur rotatif. Le brut réactionnel est purifié par chromatographie flash sur une cartouche de 20 g de silice, éluant : dichlorométhane/méthanol 100/0 à 80/20. 280 mg de 5-Fluoro-2-[4-nitro-1-(tetrahydro-pyran-2-yl)-1H-pyrazol-3-yl]-6-(3-pipéridin-1-yl-propoxy)-1H-benzimidazole sont isolés sous forme de laque rouge. LC/MS analytique : Tr = 2,51 min, [M+H]⁺ = 472,97 DAD = 36%

### Etape IV : Préparation de 3-[5-Fluoro-6-(3-piperidin-1-yl-propoxy)-1H-benzimidazol-2-yl]-1-(tetrahydro-pyran-2-yl)-1H-pyrazol-4-ylamine

Une suspension de 280 mg de 5-Fluoro-2-[4-nitro-1-(tetrahydro-pyran-2-yl)-1H-pyrazol-3-yl]-6-(3-piperidin-1-yl-propoxy)-1H-benzimidazole dans 22 mL d'acétate d'éthyle et 30 mg de palladium sur charbon est hydrogénée sous 1 bar de pression d'hydrogène à 25°C pendant 13 heures. Le milieu réactionnel est filtré sur célite et le filtrat concentré sous pression réduite à l'évaporateur rotatif. 260 mg de 3-[5-Fluoro-6-(3-piperidin-1-yl-propoxy)-1H-benzimidazol-2-yl]-1-(tetrahydro-pyran-2-yl)-1H-pyrazol-4-ylamine sont obtenus sous forme de meringue marron. Le composé est utilisé sans autre purification pour l'étape suivante. LC/MS analytique : Tr=2,30 min, [M+H]⁺ = 443,34 DAD = 64%

### Etape V : Préparation de 1,1-Diethyl-3-[3-[5-fluoro-6-(3-piperidin-1-yl-propoxy)-1H-benzimidazol-2-yl]-1-(tetrahydro-pyran-2-yl)-1H-pyrazol-4-yl]-urea

130 mg de 3-[5-Fluoro-6-(3-piperidin-1-yl-propoxy)-1H-benzimidazol-2-yl]-1-(tetrahydro-pyran-2-yl)-1 H-pyrazol-4-ylamine sont mis en solution dans 10 ml de tétrahydrofuranne puis on ajoute 372 µL de chlorure de diéthylcarbamoyle et 487 µL de N, N-diisopropyléthylamine. Le milieu réactionnel est chauffé à 50°C pendant une nuit. Le solvant est évaporé sous vide à l'évaporateur rotatif et le brut réactionnel est purifié par chromatographie flash sur une cartouche de 4 g de silice, éluant : dichlorométhane/méthanol 100/0 à 80/20. 70 mg de 1,1-diéthyl-3-[3-[5-fluoro-6-(3-pipéridin-1-yl-propoxy)-1H-benzimidazol-2-yl]-1-(tetrahydro-pyran-2-yl)-1H-pyrazol-4-yl]-urea sont isolés sous forme d'huile marron. LC/MS analytique : Tr=2,91 min, [M+H]⁺ = 542,00 DAD = 95%

### Etape VI : Chlorhydrate de 1,1-Diethyl-3-{3-[5-fluoro-6-(3-pipéridin-1-yl-propoxy)-1H-benzimidazol-2-yl]-1H-pyrazol-4-yl}-urée

70 mg de 1,1-Diethyl-3-[3-[5-fluoro-6-(3-piperidin-1-yl-propoxy)-1H-benzimidazol-2-yl]-1-(tetrahydro-pyran-2-yl)-1H-pyrazol-4-yl]-urea sont mis en suspension dans 3 ml d'une solution de HCl 3N dans le dioxanne. Le milieu est agité à température ambiante pendant 48 heures. Le solvant est évaporé sous vide à l'évaporateur rotatif. Le brut réactionnel est purifié par chromatographie flash sur cartouche de 4 g de silice, éluant : dichlorométhane/méthanol 100/0 à 80/20. Les fractions contenant le produit attendu sont concentrées et le solide obtenu est trituré dans l'oxyde d'isopropyle. Après filtration et rinçage du solide, 26.2 mg de chlorhydrate de 1,1-Diethyl-3-{3-[5-fluoro-6-(3-piperidin-1-yl-propoxy)-1H-benzimidazol-2-yl]-1H-pyrazol-4-yl}-urée sous forme de solide couleur sable sont isolés. RMN¹H (400 MHz, DMSO-d6, δ en ppm) après addition d'une goutte de TFA-d1 (CF3COOD-d1) : 1,15 (t, J=7,0 Hz, 6H) ; 1,39 (m, 1H) ; de 1,57 à 1,76 (m, 3H) ; 1,83 (m, 2H) ; 2,22 (m, 2H) ; 2,93 (m, 2H) ; 3,25 (m, 2H) ; 3,37 (q, J=7,0 Hz, 4H) ; 3,51 (m, 2H) ; 4,21 (t, J=6,0 Hz, 2H) ; 7,34 (d, J=7,5 Hz, 1 H) ; 7,56 (d, J=11,0 Hz, 1 H) ; 8,00 (s, 1H).

### Exemple 7 : Trifluoroacétate de N- {3-[6-(3-dimethylamino-propoxy)-5-fluoro-1H-benzimidazol-2-yl]-1H-pyrazol-4-yl}-Piperidine-1-carboxamide

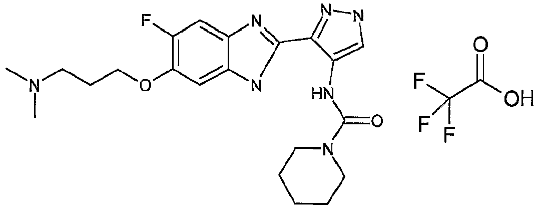

### Etape V : Préparation du Piperidine-1-carboxylic acid [3-[6-(3-diméthylamino-propoxy)-5-fluoro-1H-benzimidazol-2-yl]-1-(tetrahydro-pyran-2-yl)-1H-pyrazol-4-yl]-amide

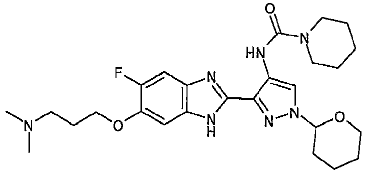

A une solution de 100 mg d'**intermédiaire 1** dans 10 mL de THF, 171,3 µL de chlorure de piperidylcarbonyle et 200 µL de N,N-diisopropyléthylamine sont additionnés. Le mélange réactionnel est chauffé à 52°C pendant 48 heures. Le milieu réactionnel est refroidi à température ambiante puis on ajoute 20mL d'une solution saturée de bicarbonate de sodium. La phase aqueuse est extraite avec 3 fois 10 mL d'AcOEt. Les phases organiques sont séchées sur MgSO₄, filtrées et concentrées sous vide à l'évaporateur rotatif. Le brut réactionnel est purifié par chromatographie flash sur un appareil IIntelliflash sur cartouche RS-4 Analogix. L'élution se fait avec un mélange dichlorométhane / méthanol. 28 mg de Piperidine-1-carboxylic acid [3-[6-(3-dimethylamino-propoxy)-5-fluoro-1H-benzimidazol-2-yl]-1-(tetrahydro-pyran-2-yl)-1 H-pyrazol-4-yl]-amide sont obtenus sous forme d'une résine brune. RMN ¹H (300 MHz, DMSO-d6, δ en ppm) : 1,50 à 2,36 (m, 14H) ; 2,29 (s large, 6H) ; 2,55 (m partiellement masqué, 2H) ; 3,50 (m, 4H) ; 3,66 (m, 1H) ; 3,96 (m, 1H) ; 4,11 (m, 2H) ; 5,46 (d large, J=10,0 Hz, 1H) ; de 7,08 à 7,55 (m, 2H) ; 8,14 (s large, 1 H) ; de 9,66 à 9,93 (m, 1 H) ; 13,05 (m, 1 H).

### Etape VI : Trifluoroacétate de N- {3-[6-(3-dimethylamino-propoxy)-5-fluoro-1H-benzimidazol-2-yl]-1H-pyrazol-4-yl}-Pipéridine-1-carboxamide

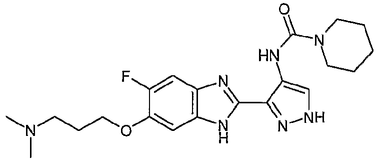

A une solution de 28 mg de Piperidine-1-carboxylic acid [3-[6-(3-dimethylamino-propoxy)-5-fluoro-1H-benzimidazol-2-yl]-1-(tetrahydro-pyran-2-yl)-1H-pyrazol-4-yl]-amide en solution dans 1,5 mL de dichlorométhane, 0,5 mL d'acide trifluoroacétique. Le milieu réactionnel est agité à 22°C pendant 16 heures. Le milieu réactionnel est concentré sous pression réduite à l'évaporateur rotatif, repris plusieurs fois à l'éther isopropylique et concentré sous pression réduite à l'évaporateur rotatif à chaque fois. 40 mg de N-{3-[6-(3-dimethylamino-propoxy)-5-fluoro-1H-benzimidazol-2-yl]-1H-pyrazol-4-yl}-Piperidine-1-carboxamide sont obtenus sous forme de sel de l'acide trifluoroacétique. RMN ¹H (300 MHz, DMSO-d6, δ en ppm) :1,47 à 1,72 (m, 6H) ; 2,19 (m, 2H) ; 2,84 (s, 6H) ; 3,27 (m, 2H) ; 3,42 (m, 4H) ; 4,22 (t, J=6,0 Hz, 2H) ; 7,40 (d, J=7,5 Hz, 1 H) ; 7,65 (d, J=10,0 Hz, 1 H) ; 8,00 (s, 1H).

### Exemple 8: 3-[3-(5-Fluoro-6-perhydro-1,4-oxazepan-4-yl-1H-benzimidazol-2-yl)-1H-pyrazol-4-yl]-1,1-diisopropyl-urée

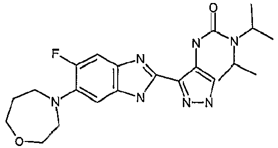

### Etape V : 3-[3-(5-Fluoro-6-perhydro-1,4-oxazepin-4-yl-1H-benzimidazol-2-yl)-1-(tétrahydro-pyran-2-yl)-1H-pyrazol-4-yl]-1,1-diisopropyl-urée

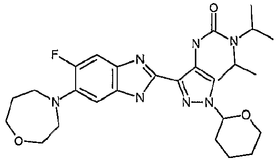

A une solution de 630 mg d'**intermédiaire 6** dans 66,6 mL de THF, 257,4 mg de chlorure de diisoproylcarbamoyle et 1,4 L de N,N-diisopropyléthylamine sont additionnés. Le mélange réactionnel est chauffé à 66°C pendant 7,5 heures, puis agité pendant 16 heures à température ambiante, puis chauffé à 66°C pendant 7,75 heures, puis agité pendant 16 heures à température ambiante. Le milieu réactionnel est lavé avec 20 mL d'eau de solution saturée en NaHCO₃. La phase aqueuse est extraite avec 3 fois 60 mL d'AcOEt. Les phases organiques sont séchées sur MgSO₄, filtrées et concentrées sous vide à l'évaporateur rotatif. Le brut réactionnel est purifié par chromatographie flash en éluant avec un mélange dichlorométhane/ méthanol : 600 mg de 3-[3-(5-Fluoro-6-perhydro-1,4-oxazepin-4-yl-1H-benzimidazol-2-yl)-1-(tetrahydro-pyran-2-yl)-1H-pyrazol-4-yl]-1,1-diisopropyl-urée sont obtenus. LC/MS analytique : Tr=4,57 min; [M+H]⁺= 528,51; ELSD = 59%; DAD = 59%.

### Etape VI : 3-[3-(5-Fluoro-6-perhydro-1,4-oxazepin-4-yl-1H-benzimidazol-2-yl)-1H-pyrazol-4-yl]-1,1-diisopropylurée

Une solution de 600 mg de 3-[3-(5-Fluoro-6-perhydro-1,4-oxazepin-4-yl-1H-benzimidazol-2-yl)-1-(tetrahydro-pyran-2-yl)-1H-pyrazol-4-yl]-1,1-diisopropyl-urée en solution dans 5 mL d'une solution d'acide chlorhydrique 4N dans le dioxanne est agitée à 22 °C pendant 4 heures. Après évaporation, le brut réactionnel est purifié par HPLC préparative puis par LC/MS préparative avec pour éluant un gradient eau, acétonitrile contenant respectivement 0,07 % d'acide trifluoroacétique. 48 mg de 3-[3-(5-Fluoro-6-perhydro-1,4-oxazepin-4-yl-1H-benzimidazol-2-yl)-1H-pyrazol-4-yl]-1,1-diisopropylurée sont obtenus sous forme d'un solide brun. RMN ¹H (400 MHz, DMSO-d6, δ en ppm) : 1,35 (d, J=7,0 Hz, 12H) ; 1,98 (m, 2H) ; 3,33 (m, 4H) ; 3,78 (m, 4H) ; 4,06 (m, 2H) ; 7,08 (d large, J=8,0 Hz, 1H) ; 7,28 (d large, J=12,0 Hz, 1H) ; 8,01 (s, 1 H) ; 9,56 (s, 1 H) ; 12,85 (m étalé, 2H).

### Exemple 9: Trifluoroacétate de 1,1-Diethyl-3-{3-[6-fluoro-5-(4-piperidin-1-yl-butoxy)-1H-benzimidazol-2-yl]-1H-pyrazol-4-yl}-urée :

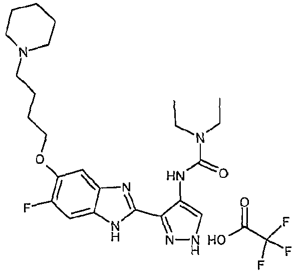

Préparation de 4-Pipéridin-1-yl-butan-1-ol : 11.68 mL d'une solution de d'hydrure de lithium aluminium 1M dans l'éther sont ajoutés à 10 mL de tétrahydrofuranne, puis on ajoute 1 g de chlorhydrate d'acide 4-Piperidin-1-yl-butyrique (synthétisé selon une méthode décrite dans la littérature). Le milieu est porté à ébullition pendant 10 minutes environ. Après retour à température ambiante et refroidissement à 0°C, on ajoute goutte à goutte un mélange 1 mL d'eau distillée-30 mL de tétrahydrofuranne. On observe un fort dégagement gazeux et une forte exothermie. Après retour à température ambiante, le milieu est évaporé à sec sous pression réduite à l'évaporateur rotatif. Le brut réactionnel est trituré dans 150 mL d'acétate d'éthyle, filtré et le filtrat est lavé par 2x20 mL d'eau distillée. La phase organique est séchée sur sulfate de magnésium, filtrée puis concentrée sous vide à l'évaporateur rotatif. 646 mg de 4-Piperidin-1-yl-butan-1-ol sont isolés sous forme d'huile incolore. RMN ¹H (300 MHz, DMSO-d6, δ en ppm) : de 1,30 à 1,53 (m, 10H) ; 2,20 (t, J=6,5 Hz, 2H) ; 2,28 (m, 4H) ; 3,37 (t large, J=6,5 Hz, 2H) ; 4,57 (s large, 1H).

### Etape I : 4-Fluoro-2-nitro-5-(4-piperidin-1-yl-butoxy)-phenylamine

A une solution de 4-Piperidin-1-yl-butan-1-ol dans 10 mL de DMF, refroidie à 0°C par un bain de glace, sont ajoutés 382 mg d'hydrure de sodium (60% en suspension dans l'huile) par petites portions en deux heures. On additionne alors une solution de 4,5-difluoro-2-nitroaniline dans 5 mL de N, N-dimethylformamide goutte à goutte. Le milieu est agité à température ambiante pendant 24 heures. 60 mL d'eau distillée sont ajoutés et le milieu réactionnel est extrait par 3x30 mL d'acétate d'éthyle. Les phases organiques rassemblées sont séchées sur sulfate de magnésium filtrée et concentrées sous vide à l'évaporateur rotatif. Le brut réactionnel est purifié par chromatographie flash sur un appareil Intelliflash avec une cartouche de silice RS-12 analogix avec un gradient d'élution dichlorométhane/méthanol 100% à 85/15. 700 mg de 4-Fluoro-2-nitro-5-(4-piperidin-1-yl-butoxy)-phenylamine sont isolés sous forme d'huile rouge. RMN ¹H (300 MHz, DMSO-d6, δ en ppm) : de 1,29 à 1,62 (m, 8H) ; 1,75 (m, 2H) ; 2,28 (m, 6H) ; 4,07 (t, J=6,5 Hz, 2H) ; 6,62 (d, J=7,5 Hz, 1H) ; 7,48 (s large, 2H) ; 7,74 (d, J=12,0 Hz, 1H).

### Etape II : 4-Fluoro-5-(4-piperidin-1-yl-butoxy)-benzene-1,2-diamine

758 mg de 4-Fluoro-2-nitro-5-(4-piperidin-1-yl-butoxy)-phenylamine en solution dans 40 mL de méthanol sont hydrogénés sous 1 bar de pression d'hydrogène en présence de 75 mg de palladium sur charbon à 25°C pendant 16 heures. Le brut réactionnel est filtré sur célite et le filtrat est concentré sous vide à l'évaporateur rotatif. 670 mg de 4-Fluoro-5-(4-piperidin-1-yl-butoxy)-benzene-1,2-diamine sont isolés sous forme d'huile noire. RMN ¹H (300 MHz, DMSO-d6, δ en ppm) : de 1,29 à 1,56 (m, 8H) ; 1,62 (m, 2H) ; de 2,20 à 2,32 (m, 6H) ; 3,82 (t, J=6,5 Hz, 2H) ; 4,27 (m large, 4H) ; 6,32 (d, J=9,0 Hz, 1 H) ; 6,34 (d, J=13,0 Hz, 1 H).

### Etape III : 6-Fluoro-2-[4-nitro-1-(tetrahydro-pyran-2-yl)-1 H-pyrazol-3-yl]-5-(4-piperidin-1-yl-butoxy)-1H-benzimidazole

Une solution de 74 mg de 4-Fluoro-5-(4-piperidin-1-yl-butoxy)-benzene-1,2-diamine et de 50.7 mg de 4-Nitro-1-(tetrahydro-pyran-2-yl)-1H-pyrazole-3-carbaldehyde dans 4 mL de méthanol est agitée à température ambiante pendant 18 heures. Après évaporation du solvant sous pression réduite à l'évaporateur rotatif, le brut réactionnel est purifié par chromatographie flash sur un appareil Intelliflash sur cartouche RS-4 analogix avec un éluant dichlorométhane/méthanol de 100% à 82/18. 77 mg de 6-Fluoro-2-[4-nitro-1-(tetrahydro-pyran-2-yl)-1H-pyrazol-3-yl]-5-(4-piperidin-1-yl-butoxy)-1 H-benzimidazole sont isolés sous forme de mousse jaune. RMN ¹H (300 MHz, DMSO-d6, δ en ppm) pour ce lot, nous observons un dédoublement 60%- 40% de tautomères avec : de 1,29 à 2,44 (m, 22H) ; 3,70 (m, 1H) ; 4,00 (m, 1 H) ; 4,10 (t, J=6,0 Hz, 2H) ; 5,60 (dd, J=3,0 et 10,0 Hz, 1H) ; 7,21 (d, J=8,5 Hz, 0,6H) ; de 7,36 à 7,46 (m, 0,8H) ; 7,53 (d, J=11,0 Hz, 0,6H) ; 9,17 (s, 1H) ; 12,8 (s large, 0,6H) ; 12,9 (s large, 0,4H).

### Etape IV : Préparation de 3-[6-Fluoro-5-(4-piperidin-1-yl-butoxy)-1H-benzimidazol-2-yl]-1-(tetrahydro-pyran-2-yl)-1H-pyrazol-4-ylamine (Intermédiaire Bêta)

470 mg de 6-Fluoro-2-[4-nitro-1-(tetrahydro-pyran-2-yl)-1H-pyrazol-3-yl]-5-(4-piperidin-1-yl-butoxy)-1 H-benzimidazole dans 30 mL d'acétate d'éthyle et 40 mg de palladium sur charbon est hydrogénée sous 1 bar de pression d'hydrogène à 25°C pendant 16 heures. Le milieu réactionnel est filtré sur célite et le filtrat concentré sous pression réduite à l'évaporateur rotatif. 380 mg de 3-[6-Fluoro-5-(4-piperidin-1-yl-butoxy)-1 H-benzimidazol-2-yl]-1-(tetrahydro-pyran-2-yl)-1H-pyrazol-4-ylamine sont obtenus. Le composé est utilisé sans autre purification pour l'étape suivante. RMN ¹H (400 MHz, DMSO-d6, δ en ppm) Pour ce lot, nous observons un dédoublement 60%-40% de tautomères avec: de 1,31 à 2,40 (m, 16H) ; 2,33 (m, 6H) ; 3,63 (m, 1H) ; 3,92 (m, 1H) ; 4,07 (m, 2H) ; 4,92 (m large, 2H) ; 5,32 (dd, J=2,5 et 10,0 Hz, 1H) ; 7,06 (d, J=8,0 Hz, 0,6H) ; 7,19 (d, J=11,0 Hz, 0,4H) ; 7,32 (s, 1H) ; 7,37 (d, J=8,0 Hz, 0,4H) ; 7,43 (d, J=11,0 Hz, 0,6H) ; 12,55 (s large, 0,4H) ; 12,6 (s large, 0,6H).

### Etape V : 1,1-Diethyl-3-[3-[6-fluoro-5-(4-piperidin-1-yl-butoxy)-1H-benzimidazol-2-yl]-1-(tetrahydro-pyran-2-yl)-1H-pyrazol-4-yl]-urée

Une solution de 110 mg d'intermédiaire Bêta, de 305 µL de chlorure de diéthylcarbamoyle et 400 µL de triéthylamine dans 6 mL de tétrahydrofuranne est agitée pendant 72 heures à 52°C. Après retour à température ambiante, le milieu est traité par 20 mL d'eau distillée et la phase aqueuse est extraite par 3 x 10 mL d'acétate d'éthyle. Les phases organiques rassemblées sont séchées sur sulfate de magnésium, filtrées et le solvant est évaporé sous pression réduite à l'évaporateur rotatif. Le brut réactionnel est purifié par chromatographie Flash sur appareil Intelliflash sur cartouche RS-12 Analogix avec un éluant dichlorométhane/méthanol 100% à 90 / 10. 100 mg de 1,1-Diethyl-3-[3-[6-fluoro-5-(4-piperidin-1-yl-butoxy)-1H-benzimidazol-2-yl]-1-(tetrahydro-pyran-2-yl)-1 H-pyrazol-4-yl]-urée sont isolés sous forme de mousse marron. RMN ¹H (400 MHz, DMSO-d6, δ en ppm) après addition d'une goutte de TFA-d1 (acide trifluoroacétique CF3COOD-d1) : 1,14 (t, J=7,0 Hz, 6H) ; de 1,35 à 2,28 (m, 16H) ; de 2,80 à 2,94 (m, 2H) ; 3,12 (m, 2H) ; 3,35 (q, J=7,0 Hz, 4H) ; 3,44 (m, 2H) ; 3,65 (m, 1H) ; 3,96 (m, 1 H) ; 4,15 (m, 2H) ; 5,49 (dd, J=2,5 et 10,0 Hz, 1 H) ; 7,31 (d, J=7,5 Hz, 1H) ; 7,54 (d, J=10,5 Hz, 1 H) ; 8,13 (s, 1H).

### Etape VI : Trifluoroacétate de 1,1-Diethyl-3-{3-[6-fluoro-5-(4-piperidin-1-yl-butoxy)-1H-benzimidazol-2-yl]-1H-pyrazol-4-yl}-urée

100 mg de 1,1-Diethyl-3-[3-[6-fluoro-5-(4-piperidin-1-yl-butoxy)-1H-benzimidazol-2-yl]-1-(tetrahydro-pyran-2-yl)-1H-pyrazol-4-yl]-urea en solution dans 2 mL de dichlorométhane avec 1 mL d'acide trifluoroacétique sont agités à température ambiante pendant 18 heures. Le solvant est évaporé sous vide par chromatographie Flash sur appareil Intelliflash sur cartouche RS-12 Analogix avec un éluant dichlorométhane 100% à dichlorométhane/méthanol 85/15. 72 mg de 1,1-Diethyl-3-{3-[6-fluoro-5-(4-piperidin-1-yl-butoxy)-1H-benzimidazol-2-yl]-1H-pyrazol-4-yl}-urée sous forme de sel de trifluoroacétate sont isolés sous forme d'un solide jaune. RMN ¹H (400 MHz, DMSO-d6, δ en ppm). Pour ce lot, nous observons un dédoublement 60%-40% de tautomères avec : 1,23 (m, 6H) ; de 1,30 à 1,91 (m étalé, 10H) ; 2,70 à 3,53 (m très étalé, 6H) ; 3,41 (m, 4H) ; 4,12 (m, 2H) ; 7,11 (d, J= 8,0 Hz, 0,6H) ; de 7,24 à 7,30 (m, 0,8H) ; 7,44 (d, J=11,0 Hz, 0,6H) ; 8,00 (s, 1 H) ; 8,94 (m étalé, 1 H) ; 9,77 (s, 0,4H) ; 9,79 (s, 0,6H) ; 12,9 (s large, 0,4H) ; 12,95 (s large, 0,6H) ; 13,0 (s large, 1H).

### Exemple 10 : Trifluoroacétate de N-{3-[6-fluoro-5-(4-piperidin-1-yl-butoxy)-1H-benzimidazol-2-yl]-1H-pyrazol-4-yl}- Piperidine-1-carboxamide

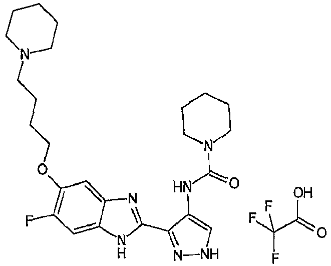

### Etape V : N-{3-[6-fluoro-5-(4-piperidin-1-yl-butoxy)-1H-benzimidazol-2-yl]-1-(tetrahydro-pyran-2-yl)--1 H-pyrazol-4-yl}- Piperidine-1-carboxamide

130 mg d'intermédiaire Bêta sont mis en solution dans 7 mL de Tétrahydrofuranne avec 356 µL de chlorure de pipéridine carbonyle et 472 µL de N, N-diisopropylamine et agités à température ambiante pendant 18 heures. Après évaporation du solvant sous vide à l'évaporateur rotatif, le brut réactionnel est purifié par chromatographie flash sur cartouche RS-12 Analogix, avec un éluant dichlorométhane 100% à dichlorométhane/méthanol 90/10. 130 mg de Piperidine-1-carboxylic acid [3-[6-fluoro-5-(4-piperidin-1-yl-butoxy)-1H-benzimidazol-2-yl]-1-(tetrahydro-pyran-2-yl)-1H-pyrazol-4-yl]-amide sont isolés sous forme de mousse jaune. RMN ¹H (300 MHz, DMSO-d6, δ en ppm) : pour ce lot, nous observons un dédoublement 60% - 40% de tautomères avec : de 1,33 à 2,21 (m étalé, 22H) ; de 2,65 à 3,35 (m très étalé, 6H) ; 3,49 (m, 4H) ; 3,65 (m, 1 H) ; 3,98 (m, 1 H) ; 4,12 (m, 2H) ; 5,46 (d large, J=10,0 Hz, 1 H) ; 7,11 (d, J=7,5 Hz, 0,6H) ; 7,27 (d, J=11,0 Hz, 0,4H) ; 7,36 (d, J=7,5 Hz, 0,4H) ; 7,52 (d, J=11,0 Hz, 0,6H) ; 8,14 (s, 1H) ; 9,86 (s, 0,4H) ; 9,91 (s, 0,6H) ; 13,0 (s large, 0,4H) ; 13,05 (s large, 0,6H).

### Etape VI : Trifluoroacétate de N-{3-[6-fluoro-5-(4-piperidin-1-yl-butoxy)-1H-benzimidazol-2-yl]-1H-pyrazol-4-yl}- Piperidine-1-carboxamide

Une solution de 130 mg de Piperidine-1-carboxylic acid [3-[6-fluoro-5-(4-piperidin-1-yl-butoxy)-1H-benzimidazol-2-yl]-1-(tetrahydro-pyran-2-yl)-1H-pyrazol-4-yl]-amide dans 2 mL de dichlorométhane et 500 µL d'acide trifluoroacétique sont agités pendant 72 heures à température ambiante. Après évaporation du solvant sous vide à l'évaporateur rotatif, le brut réactionnel est purifié par chromatographie Flash sur cartouche RS-12 Analogix avec un éluant dichlorométhane 100% à dichlorométhane/méthanol 90/10. 86 mg de N-{3-[6-fluoro-5-(4-piperidin-1-yl-butoxy)-1H-benzimidazol-2-yl]-1H-pyrazol-4-yl}-Piperidine-1-carboxamide sous forme de sel de trifluoacétate, sous forme de solide jaune. RMN ¹H (300 MHz, DMSO-d6, δ en ppm) : pour ce lot, nous observons un dédoublement 60%-40% de tautomères avec : de 1,20 à 1,94 (m étalé, 16H) ; de 2,70 à 3,43 (m partiellement masqué, 6H) ; 3,50 (m, 4H) ; 4,11 (m, 2H) ; 7,11 (d, J=7,5 Hz, 0,6H) ; 7,27 (d, J=11,0 Hz, 0,4H) ; 7,37 (d, J=7,5 Hz, 0,4H) ; 7,51 (d, J=11,0 Hz, 0,6H) ; 8,01 (s large, 1H) ; 8,94 (m étalé, 1H) ; 9,83 (s, 0,4H) ; 9,87 (s, 0,6H) ; 12,95 (s, 0,4H) ; 13,0 (s, 0,6H) ; 13,05 (s, 1H).

### Exemple 11: 1,1-Diethyl-3-[3-(5-fluoro-6-perhydro-1,4-oxazepin-4-yl-1H-benzimidazol-2-yl)-1 H-pyrazol-4-yl]-urée

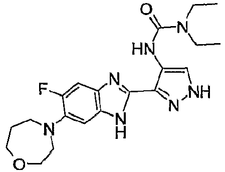

### Etape I : 4-Fluoro-2-nitro-5-perhydro-1,4-oxazepin-4-yl-phenylamine

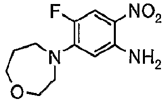

A une solution de 5 g de 4,5-difluoro-2-nitroaniline dans 98 mL de DMF anhydre, 19,4 g de carbonate de potassium et 11,9 g de chlorhydrate d'homomorpholine sont ajoutés. Le milieu réactionnel est chauffé à 80°C à l'aide d'un bain d'huile pendant 2 heures. Le milieu réactionnel est refroidi à température ambiante, puis on y ajoute 300 mL d'eau, il y a précipitation. Le précipité est filtré sur verre fritté. Le solide jaune est rincé avec 3 fois 100 mL d'eau. Le solide est séché à l'étuve à 70°C. 6,5 g de 4-Fluoro-2-nitro-5-perhydro-1,4-oxazepin-4-yl-phenylamine sont obtenus sous forme de solide orange. RMN ¹H (400 MHz, DMSO-d6, δ en ppm) : 1,91 (m, 2H) ; de 3,52 à 3,61 (m, 4H) ; 3,66 (m, 2H) ; 3,74 (m, 2H) ; 6,28 (d, J=8,5 Hz, 1H) ; 7,24 (s large, 2H) ; 7,61 (d, J=15,5 Hz, 1 H).

### Etape II : 4-Fluoro-5-perhydro-1,4-oxazepin-4-yl-benzene-1,2-diamine

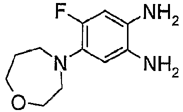

7 g de 4-Fluoro-2-nitro-5-perhydro-1,4-oxazepin-4-yl-phenylamine en solution dans 170 mL de méthanol sont hydrogénés sous 1 bar de pression d'hydrogène en présence de 700 mg de palladium sur charbon à 22°C pendant 10 heures. Le brut réactionnel est filtré sur célite et le filtrat est concentré sous vide à l'évaporateur rotatif. Après évaporation, le brut réactionnel est purifié par chromatographie flash (élution dichlorométhane/méthanol) 3 g de 4-Fluoro-5-perhydro-1,4-oxazepin-4-yl-benzene-1,2-diamine sont isolés. Spectre RMN ¹H (400 MHz, DMSO-d6, δ en ppm) : 1,88 (m, 2H) ; 3,11 (m, 4H) ; de 3,64 à 3,74 (m, 4H) ; 4,20 (s large, 2H) ; 4,26 (s large, 2H) ; de 6,25 à 6,31 (m, 2H).

### Etape III: 5-Fluoro-2-[4-nitro-1-(tetrahydro-pyran-2-yl)-1H-pyrazol-3-yl]-6-perhydro-1,4-oxazepin-4-yl-1H-benzimidazole

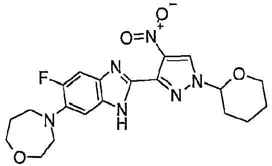

A une solution de 3 g de 4-Fluoro-5-perhydro-1,4-oxazepin-4-yl-benzene-1,2-diamine dans 60 mL de DMF, 3 g de 4-Nitro-1-(tetrahydro-pyran-2-yl)-1H-pyrazole-3-carbaldéhyde et 43 mg de chlorure ferrique sont ajoutés. Le milieu réactionnel est agité à 22°C pendant 16 heures. Après évaporation, le brut réactionnel est purifié par chromatographie flash. L'élution se fait dans un mélange cyclohexane/acétate d'éthyle 1/1. 3 g de 5-Fluoro-2-[4-nitro-1-(tetrahydro-pyran-2-yl)-1H-pyrazol-3-yl]-6-perhydro-1,4-oxazepin-4-yl-1H-benzimidazole sont isolés sous forme d'une huile rouge. RMN ¹H (400 MHz, DMSO-d6, δ en ppm) : De 1,40 à 2,25 (m, 8H) ; de 3,25 à 3,39 (m partiellement masqué, 4H) ; 3,70 (m, 1 H) ; 3,78 (m, 4H) ; 3,99 (m, 1H) ; 5,59 (dd, J = 2,5 et 9,5 Hz, 1 H) ; 7,16 (m étalé, 1 H) ; 7,39 (d large, J = 11,5 Hz, 1H) ; 9,17 (s, 1 H) ; 12,75 (m étalé, 1 H).

### Etape IV : 3-(5-Fluoro-6-perhydro-1,4-oxazepin-4-yl-1H-benzimidazol-2-yl)-1-(tetrahydro-pyran-2-yl)-1 H-pyrazol-4-ylamine (intermédiaire 6)

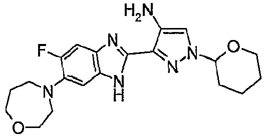

Une suspension de 2,9 g de 5-Fluoro-2-[4-nitro-1-(tetrahydro-pyran-2-yl)-1H-pyrazol-3-yl]-6-perhydro-1,4-oxazepin-4-yl-1H-benzimidazole dans 100 mL de méthanol et 497 mg de palladium sur charbon est hydrogénée sous 1 bar de pression d'hydrogène à température ambiante pendant 16 heures. Le milieu réactionnel est filtré sur célite et le filtrat concentré sous pression réduite à l'évaporateur rotatif. 2,2 g de 3-(5-Fluoro-6-perhydro-1,4-oxazepin-4-yl-1H-benzimidazol-2-yl)-1-(tetrahydro-pyran-2-yl)-1 H-pyrazol-4-ylamine sont obtenus sous forme d'une résine noire. RMN ¹H (400 MHz, DMSO-d6, δ en ppm) : de 1,26 à 2,24 (m, 8H) ; 3,55 (m, 4H) ; 3,68 (m, 1H) ; 3,78 (m, 2H) ; 3,84 (m, 2H) ; 3,97 (m, 1H) ; 5,55 (dd, J=2,5 et 10,5 Hz, 1H) ; 7,38 (d large, J=8,0 Hz, 1H); 7,52 (d, J=12,5 Hz, 1 H) ; 8,17 (s, 1H).

### Etape V : 1,1-Diethyl-3-[3-(5-fluoro-6-perhydro-1,4-oxazepin-4-yl-1H-benzimidazol-2-yl)-1-(tetrahydro-pyran-2-yl)-1H-pyrazol-4-yl]-urée

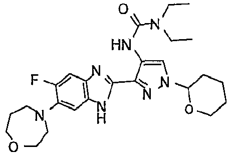

A une solution de 1,8 g d'intermédiaire 6 dans 36 mL de THF, 2,8 mL de chlorure de diethylcarbamoyle et 4 mL de N,N-diisopropyléthylamine sont additionnés. Le mélange réactionnel est chauffé à 80°C pendant 16 heures. Le milieu réactionnel est concentré sous vide à l'évaporateur rotatif, puis purifié par chromatographie flash (élution dichlorométhane / méthanol): Le mélange contenant le produit attendu est concentré puis repurifié sur 200 g de silice (0,2-0,045 µm), sous 0.5 bar de pression en éluant dans un mélange cyclohexane/acétate d'éthyle 30/40 puis 80/20. 380 mg de 1,1-diéthyl-3-[3-(5-fluoro-6-perhydro-1,4-oxazepin-4-yl-1H-benzimidazol-2-yl)-1-(tetrahydro-pyran-2-yl)-1H-pyrazol-4-yl]-urea sont obtenus sous forme d'une mousse beige. RMN ¹H (300 MHz, DMSO-d6, δ en ppm) : 1,14 (t, J=7,0 Hz, 6H) ; de 1,48 à 2,22 (m, 8H) ; 3,36 (q, J=7,0 Hz, 4H) ; 3,54 (m, 4H) ; 3,65 (m, 1 H) ; 3,77 (m, 2H) ; 3,85 (m, 2H) ; 3,96 (m, 1H) ; 5,49 (dd, J=2,0 et 9,5 Hz, 1H) ; 7,42 (d, J=7,5 Hz, 1H) ; 7,52 (d, J=12,0 Hz, 1 H) ; 8,13 (s, 1H).

### Etape VI 1,1-Diethyl-3-[3-(5-fluoro-6-perhydro-1,4-oxazepin-4-yl-1H-benzimidazol-2-yl)-1H-pyrazol-4-yl]-urée

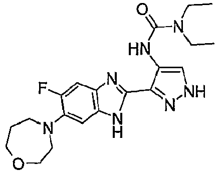

A une solution de 380 mg de 1,1-Diethyl-3-[3-(5-fluoro-6-perhydro-1,4-oxazepin-4-yl-1 H-benzimidazol-2-yl)-1-(tetrahydro-pyran-2-yl)-1 H-pyrazol-4-yl]-urea en solution dans 5 mL d'une solution d'acide chlorhydrique 4N dans le dioxanne est agitée à 22°C pendant 2 heures. Le milieu réactionnel est concentré sous vide à l'évaporateur rotatif, puis remis en réaction à 22°C pendant 16 heures avec 5 mL d'une solution d'acide chlorhydrique 4N dans le dioxanne est agitée à 22°C. Le milieu réactionnel est concentré sous vide à l'évaporateur rotatif. Le produit est dilué dans de l'acétate d'éthyle, lavé avec 2 fois 20 mL d'une solution de soude aqueuse à 2N. Les phases aqueuses sont extraite avec 3 fois 20 mL de d'acétate d'éthyle. Les phases organiques sont séchées sur MgSO₄, filtrées et concentrées sous vide à l'évaporateur rotatif. La mousse jaune obtenue est empâtée dans l'éther isopropylique, triturée, filtrée et séchés sous vide à 40°C. 230 mg de 1,1-Diéthyl-3-[3-(5-fluoro-6-perhydro-1,4-oxazepin-4-yl-1H-benzimidazol-2-yl)-1H-pyrazol-4-yl]-urea sont obtenus sous forme d'un solide jaune pâle. RMN ¹H (500 MHz, DMSO-d6, δ en ppm) : 1,23 (t, J=7,0 Hz, 6H) ; 1,96 (m, 2H) ; 3,35 (m masqué, 4H) ; 3,39 (q partiellement masqué, J=7,0 Hz, 4H) ; 3,79 (m, 4H) ; de 6,98 à 7,39 (m étalé, 2H) ; 7,98 (s, 1 H) ; 9,87 (s large, 1 H).

### Exemple 12: Trifluoroacétate de N-{3-[5-fluoro-6-(4-methyl-piperazin-1-yl)-1H-benzimidazol-2-yl]-1H-pyrazol-4-yl}-Piperidine-1-carboxamide

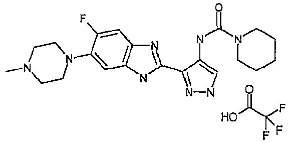

### Etape I : 4-Fluoro-5-(4-methyl-piperazin-1-yl)-2-nitro-phénylamine

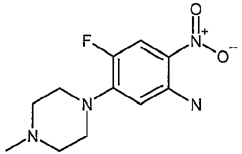

A une solution de 15 g de 4,5-difluoro-2-nitroaniline dans 120 mL de DMF anhydre, 36,2 g de bicarbonate de sodium et 28,7 mL N-methylhomopiperazine sont ajoutés. Le milieu réactionnel est chauffé à 80°C à l'aide d'un bain d'huile pendant 2H30. Le milieu réactionnel est refroidi à température ambiante, puis versé dans 500 mL d'eau. On refroidit à l'aide d'un bain de glace et on agite, il y a précipitation. Le précipité est filtré sur verre fritté. Le solide jaune est rincé à l'eau. Le solide est séché à l'étuve à 40°C. 21,2 g de 4-fluoro-5-(4-methyl-piperazin-1-yl)-2-nitro-phenylamine sont obtenus sous forme de solide jaune. RMN ¹H (300 MHz, DMSO-d6, δ en ppm) : 2,22 (s, 3H) ; 2,45 (m, 4H) ; 3,17 (m, 4H) ; 6,42 (d, J=8,0 Hz, 1 H) ; 7,34 (s large, 2H) ; 7,63 (d, J=14,5 Hz, 1 H). Spectre de masse en IE : m/z=254 : M+. (pic de base) - m/z=239 :(M - CH3)+ - m/z=234 : (M - HF)+. - m/z=183 : (M-C4H9N)+ - m/z=70 : C4H8N+ - m/z=43 : C2H5N+.

### Etape II : 4-Fluoro-5-(4-methyl-piperazin-1-yl)-benzene-1,2-dismine

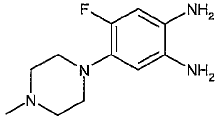

21,2 g de 4-Fluoro-5-(4-méthyl-pipérazin-1-yl)-2-nitro-phenylamine en solution dans 800 mL de méthanol sont hydrogénés sous 1 bar de pression d'hydrogène en présence de 2,1 g de palladium sur charbon à 25°C pendant 16 heures. Le brut réactionnel est filtré sur célite et le filtrat est concentré sous vide à l'évaporateur rotatif. 18,7 g de 4-Fluoro-5-(4-méthyl-pipérazin-1-yl)-benzene-1,2-diamine sous forme d'une huile noire sont isolés. RMN ¹H (400 MHz, DMSO-d6, δ en ppm), pour ce lot, tous les signaux sont larges avec : 2,19 (s, 3H) ; 2,40 (m, 4H) ; 2,79 (m, 4H) ; 4,22 (s, 2H) ; 4,31 (s, 2H) ; de 6,24 à 6,33 (m, 2H). Spectre de masse en IE : m/z=224 : M+. (pic de base) - m/z=209 : (M - CH3)+ - m/z=153 : (M - C4H9N)+ - m/z=70 : C4H8N+ - m/z=43 : C2H5N+.

### Etape III : 5-Fluoro-6-(4-methyl-piperazin-1-yl)-2-[4-nitro-1-(tetrahydro-pyran-2-yl)-1H-pyrazol-3-yl]-1H-benzimidazole

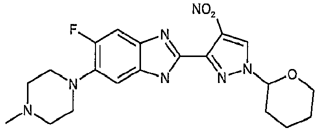

A une solution de 25,3 g de 4-Fluoro-5-(4-methyl-piperazin-1-yl)-benzene-1,2-diamine dans 320 mL de méthanol, 25,4 g de 4-Nitro-1-(tetrahydro-pyran-2-yl)-1 H-pyrazole-3-carbaldehyde sont ajoutés. Le milieu réactionnel est agité à 22°C pendant 16 heures. Après évaporation, le brut réactionnel est purifié sur une colonne de diamètre 8 cm, avec 3,5 kg de silice de porosité 0,063-0,2 mm. L'élution se fait dans avec 2 L de dichlorométhane pur puis 5 L de dichlorométhane à 5% de méthanol, puis avec du dichlorométhane à 10% de méthanol. 18,1 g de 5-Fluoro-6-(4-methyl-piperazin-1-yl)-2-[4-nitro-1-(tetrahydro-pyran-2-yl)-1H-pyrazol-3-yl]-1H-benzimidazole sont isolés. RMN ¹H (400 MHz, DMSO-d6, δ en ppm) : De 1,36 à 2,29 (m, 6H) ; 2,92 (s, 3H) ; 3,10 (m, 2H) ; 3,31 (m, 2H) ; 3,58 (m, 4H) ; 3,72 (m, 1H) ; 4,00 (m, 1 H) ; 5,68 (dd, J = 2,0 et 10,0 Hz, 1H) ; 7,48 (d, J = 7,5 Hz, 1 H) ; 7,75 (d, J = 11,5 Hz, 1 H) ; 9,38 (s, 1 H). Spectre de masse en ES : m/z=857 : (2M - H) pic de base - m/z=428 : (M - H) -

### Etape IV : 3-(5-Fluoro-6-morpholin-4-yl-1H-benzimidazoi-2-yl)-1-(tetrahydro-pyran-2-yl)-1H-pyrazol-4-ylamine (intermédiaire 5)

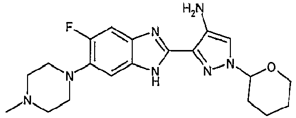

Une suspension de 18,1 g de 5-Fluoro-6-(4-methyl-piperazin-1-yl)-2-[4-nitro-1-(tetrahydro-pyran-2-yl)-1H-pyrazol-3-yl]-1H-benzimidazole dans 425 mL de méthanol et 1,8 g de palladium sur charbon est hydrogénée sous 1 bar de pression d'hydrogène à température ambiante pendant 18 heures. Le milieu réactionnel est filtré sur célite et le filtrat concentré sous pression réduite à l'évaporateur rotatif. 15,9 g de 3-(5-Fluoro-6-morpholin-4-yl-1H-benzimidazol-2-yl)-1-(tetrahydro-pyran-2-yl)-1 H-pyrazol-4-ylamine sont obtenus sous forme d'une poudre brune. Le composé sera utilisé tel quel pour l'étape suivante. RMN ¹H (300 MHz, DMSO-d6, δ en ppm) : de 1,48 à 2,24 (m, 6H) ; 2,91 (s, 3H) ; 3,05 (m, 2H) ; 3,28 (m, 2H) ; 3,54 (m, 4H) ; 3,67 (m, 1H) ; 3,97 (m, 1 H) ; 5,55 (dd, J=2,0 et 10,5 Hz, 1 H) ; 7,22 (d, J=8,0 Hz, 1H) ; 7,48 (d, J=12,0 Hz, 1H) ; 8,18 (s, 1H). Spectre de masse en IE : m/z=399 : M+.(pic de base) - m/z=315: (M - C5H80)+ - m/z=85: C5H9O+ - m/z=43: C2H5N+.

### Etape V : N-{3-[5-fluoro-6-(4-methyl-piperazin-1-yl)-1H-benzimidazol-2-yl]-1-(tetrahydro-pyran-2-yl)-1H-pyrazol-4-yl}-Pipéridine-1-carboxamide

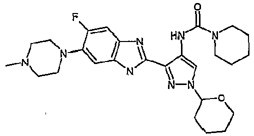

A une solution de 8 g d'intermédiaire 5 dans 268 mL de THF, 12,5 mL de chlorure de 1-piperidinecarbonyle et 17,4 mL de N,N-diisopropyléthylamine sont additionnés. Le mélange réactionnel est chauffé à 66°C pendant 5 heures puis agité 16 heures à 22°C. Le milieu réactionnel est lavé avec 115 mL d'eau. La phase aqueuse est extraite avec 3 fois 215 mL d'AcOEt. Les phases organiques sont séchées sur MgSO₄, filtrées et concentrées sous vide à l'évaporateur rotatif. Le brut réactionnel est purifié par chromatographie flash (élution dichlorométhane/méthanol): 8,22 g de Piperidine-1-carboxylic acid [3-[5-fluoro-6-(4-methyl-piperazin-1-yl)-1H-benzimidazol-2-yl]-1-(tetrahydro-pyran-2-yl)-1H-pyrazol-4-yl]-amide sont obtenus sous forme d'une poudre jaune pâle. Il s'agit du sel de TFA de la structure attendue dans la mesure où nous observons : RMN ¹H (400 MHz, DMSO-d6, δ en ppm) : De 1,40 à 2,26 (m, 12H) ; 2,89 (s, 3H) ; 3,09 (m, 2H) ; 3,28 (m, 2H) ; 3,42 (m, 4H) ; 3,55 (m, 4H) ; 3,67 (m, 1 H) ; 3,96 (m, 1 H) ; 5,50 (dd, J=2,5 et 10,5 Hz, 1H) ; 7,33 (d, J=7,5 Hz, 1H) ; 7,57 (d, J= 11,5 Hz, 1H) ; 8,15 (s, 1H). Spectre de masse en IE : m/z=510 (M+) ; m/z=425 (M - C5H11N)+. ; m/z=341 (m/z=425 - C5H8O)+. m/z=85 C5H9O+. ; m/z=84 (C5H10N+) ; m/z=43 (C2H5N+. ; m/z=41 C3H5+ pic de base

### Etape VI Trifluoroacétate de N-{3-[5-fluoro-6-(4-methyl-piperazin-1-yl)-1H-benzimidazol-2-yl]-1H-pyrazol-4-yl}-Piperidine-1-carboxamide

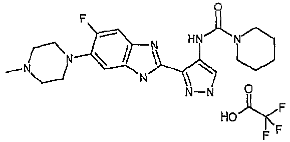

A une solution de 260 mg de Piperidine-1-carboxylic acid [3-[5-fluoro-6-(4-methyl-piperazin-1-yl)-1H-benzimidazol-2-yl]-1-(tétrahydro-pyran-2-yl)-1H-pyrazol-4-yl]-amide en solution dans 1,5 mL de dichlorométhane, 0,6 mL d'acide trifluoroacétique sont ajoutés. Le milieu réactionnel est agité à 22°C pendant 48 heures. Le milieu réactionnel est concentré sous vide à l'évaporateur rotatif. Après évaporation, le brut réactionnel est purifié par chromatographie flash sur un appareil Intelliflash sur cartouche RS-12 Analogix. L'élution se fait dans le dichlorométhane pur pendant 20 min puis dans le dichlorométhane contenant 10% de méthanol ammoniacal. 120 mg de N-{3-[5-fluoro-6-(4-methyl-piperazin-1-yl)-1H-benzimidazol-2-yl]-1H-pyrazol-4-yl}-Piperidine-1-carboxamide sont obtenus sous forme d'un solide beige. RMN ¹H (400 MHz, DMSO-d6, δ en ppm) : de 1,45 à 1,70 (m, 6H) ; 2,90 (s, 3H) ; 3,07 (m, 2H) ; 3,29 (m, 2H) ; 3,45 (m, 4H) ; 3,55 (m, 4H) ; 7,31 (d, J=8,0 Hz, 1H) ; 7,57 (d, J=11,5 Hz, 1 H) ; 8,02 (s, 1H). Spectre de masse en IE : m/z=426, M+., m/z=341 (M - C₅H₁₁N)+. ; m/z=114 CF₃CO₂H+. relatif au TFA ; m/z=84 C₅H₁₀N+ ; m/z=69 CF₃+ relatif au TFA ; m/z=45 CO₂H+ relatif au TFA pic de base, m/z=43 C₂H₅N+. ; m/z=41 C₃H₅+

### Exemple 13: 1,1-Diethyl-3-{3-[5-fluoro-6-(4-methyl-piperazin-1-yl)-1H-benzimidazol-2-yl]-1H-pyrazol-4-yl}-urée:

### Etape V : 1,1-Diethyl-3-[3-[5-fluoro-6-(4-methyl-piperazin-1-yl)-1H-benzimidazol-2-yl]-1-(tetrahydro-pyran-2-yl)-1H-pyrazol-4-yl]-urée

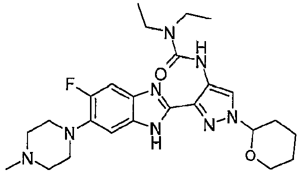

A une solution de 166 mg d'intermédiaire 5 dans 10 mL de THF, 506 µL de chlorure de diethylcarbamoyle et 662 µL de N,N-diisopropyléthylamine sont additionnés. Le mélange réactionnel est chauffé à 55°C pendant 40 heures, puis 2 heures à 80°C. Le milieu réactionnel est refroidi à température ambiante et concentré sous vide à l'évaporateur rotatif. Le brut réactionnel est purifié par chromatographie flash (élution dichlorométhane/méthanol): 250 mg de 1,1-Diethyl-3-[3-[5-fluoro-6-(4-methyl-piperazin-1-yl)-1H-benzimidazol-2-yl]-1-(tetrahydro-pyran-2-yl)-1H-pyrazol-4-yl]-urée sont obtenus sous forme d'une huile jaune. RMN ¹H (300 MHz, DMSO-d6, δ en ppm) : 1,18 (t, J=7,0 Hz, 6H) ; de 1,50 à 2,21 (m, 6H) ; 2,90 (s, 3H) ; 3,05 (m, 2H) ; 3,28 (m, 2H) ; 3,38 (q, J=7,0 Hz, 4H) ; 3,54 (m, 4H) ; 3,68 (m, 1H) ; 3,98 (m, 1H) ; 5,48 (dd, J=2,0 et 10,0 Hz, 1H) ; 7,23 (d, J=8,0 Hz, 1H) ; 7,46 (d, J=11,5 Hz, 1H) ; 8,14 (s, 1 H). Spectre de masse en ES : m/z=497 (M - H) - (pic de base)

### Etape VI : Trifluoroacétate de 1,1-Diethyl-3-{3-[5-fluoro-6-(4-methyl-piperazin-1-yl)-1H-benzimidazol-2-yl]-1H-pyrazol-4-yl}-urée

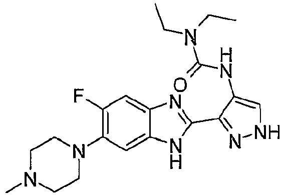

A une solution de 250 mg de 1,1-Diethyl-3-[3-[5-fluoro-6-(4-methyl-piperazin-1-yl)-1H-benzimidazol-2-yl]-1-(tetrahydro-pyran-2-yl)-1H-pyrazol-4-yl]-urea en solution dans 2 mL de dichlorométhane, 0,7 mL d'acide trifluoroacétique sont ajoutés. Le milieu réactionnel est agité à 22°C pendant 72 heures. Le milieu réactionnel est concentré sous vide à l'évaporateur rotatif. Après évaporation, le brut réactionnel est purifié par chromatographie flash sur un appareil Intelliflash sur cartouche RS-12 Analogix. L'élution se fait dans le dichlorométhane pur pendant 15 min puis dans le dichlorométhane contenant 10% de méthanol ammoniacal. 100 mg de 1,1-Diethyl-3-{3-[5-fluoro-6-(4-methyl-piperazin-1-yl)-1H-benzimidazol-2-yl]-1H-pyrazol-4-yl}-urea sont obtenus sous forme d'un solide beige. RMN ¹H (300 MHz, DMSO-d6, δ en ppm) : 1,18 (t, J=7,0 Hz, 6H) ; 2,90 (s, 3H) ; 3,05 (m, 2H) ; 3,28 (m, 2H) ; 3,38 (q, J= 7,0 Hz, 4H) ; 3,54 (m, 4H) ; 7,24 (d, J=8,0 Hz, 1 H) ; 7,46 (d, J=11,5 Hz, 1 H) ; 8,00 (s, 1 H). Spectre de masse en IE : m/z=414 (M+. ; m/z=341 (M - C4H11 N)+. ; m/z=114 CF3CO2H+. relatif au TFA ; m/z=72 C4H10N+; m/z=69 CF3+ relatif au TFA; m/z=45 CO2H+ relatif au TFA, pic de base ; m/z=43 C2H5N+.

### Exemple de référence 14: trifluoroacétate de 1-{2-[4-(3,3-Diethyl-ureido)-1H-pyrazol-3-yl]-6-fluoro-3H-benzimidazol-5-yl}-1-(2-dimethylamino-ethyl)-3,3-diethyl-urée

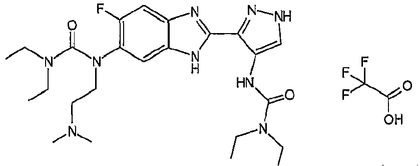

De la même manière que pour l'étape VI de l'exemple 15 (voir plus bas), on déprotège 100 mg de 1-{2-[4-(3,3-Diethyl-ureido)-1-(tetrahydro-pyran-2-yl)-1H-pyrazol-3-yl]-8-fluoro-3H-benzimidazol-5-yl}-1-(2-dimethylamino-ethyl)-3,3-diethyl-urea (étape 5, exemple 15), et on isole 54 mg de 1-{2-[4-(3,3-Diethyl-ureido)-1H-pyrazol-3-yl]-6-fluoro-3H-benzimidazol-5-yl}-1-(2-dimethylamino-ethyl)-3,3-diethyl-urée sous forme de sel de trifluoroacétate. RMN ¹H (300 MHz, DMSO-d6, δ en ppm), pour ce lot, nous observons un dédoublement 60% - 40% de tautomères avec : 0,75 (m, 6H) ; 1,22 (m, 6H) ; de 2,30 à 2,86 (m étalé partiellement masqué, 8H) ; 3,02 (q, J=7,0 Hz, 4H) ; 3,42 (q, J=7,0 Hz, 4H) ; 3,64 (m large, 2H) ; 7,36 (m, 1H) ; 7,52 (m, 1H) ; 8,02 (s, 1 H) ; 9,35 (m étalé, 1 H) ; 9,71 (s, 0,4H) ; 9,74 (s, 0,6H) ; 13,1 (s large, 1H) ; 13,15 (s large, 0,4H) ; 13,2 (s large, 0,6H). Spectre de masse en ES : 502(+)=(M+H)(+)

### Exemple 15: Trifluoroacétate de 3-{3-[6-(2-Dimethylamino-ethylamino)-5-fluoro-1H-benzimidazol-2-yl]-1H-pyrazol-4-yl}-1,1-diethyl-urée

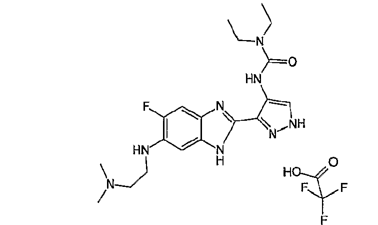

### Etape I : N3-(2-Dimethylamino-ethyl)-4-fluoro-6-nitro-benzene-1,3-diamine

Une solution de 4,5-Difluoro-2-nitroaniline dans 10 mL de DMF est agité à température ambiante et 2,53 g de bicarbonate de sodium et 1,67 g de N, N-diméthylènediamine sont additionnés. Le milieu réactionnel est chauffé au reflux pendant deux heures. On ajoute 30 mL d'eau puis la phase aqueuse est extraite par 4 x 15 ml d'acétate d'éthyle. Les phases organiques sont séchées sur sulfate de magnésium, filtrées et concentrées sous vide à l'évaporateur rotatif. Le brut réactionnel est repris dans l'éther isopropylique et le solide est trituré, puis filtré sur verre fritté. Le solide est séché sous vide à 40°C jusqu'à poids constant. 1,23 g de N3-(2-Dimethylamino-ethyl)-4-fluoro-6-nitro-benzene-1,3-diamine sont recueillis sous forme de solide jaune. RMN ¹H (300 MHz, DMSO-d6, δ en ppm) : 2,19 (s, 6H) ; 2,45 (t, J=6,5 Hz, 2H) ; 3,19 (m, 2H) ; 6,03 (d, J=8,0 Hz, 1H) ; 6,58 (t large, J = 5,5 Hz, 1H) ; 7,37 (s large, 2H) ; 7,58 (d, J=13,0 Hz, 1H).

### Etape II : N4-(2-Dimethylamino-ethyl)-5-fluoro-benzene-1,2,4-triamine

1.29 g de N3-(2-Dimethylamino-ethyl)-4-fluoro-6-nitro-benzene-1,3-diamine en solution dans 30 mL de méthanol sont hydrogénés sous 1 bar de pression d'hydrogène en présence de 130 mg de palladium sur charbon à 25°C pendant 16 heures. Le brut réactionnel est filtré sur célite et le filtrat est concentré sous vide à l'évaporateur rotatif. 1 g de N4-(2-Dimethylamino-ethyl)-5-fluoro-benzene-1,2,4-triamine sont recueillis sous forme de résine noire. Le produit est utilisé comme tel pour l'étape suivante.

### Etape III: N1-{6-Fluoro-2-[4-nitro-1-(tetrahydro-pyran-2-yl)-1H-pyrazol-3-yl]-3H-benzimidazol-5-yl}-N2,N2-dimethyl-ethane-1,2-diamine

Une solution de 1 g de N4-(2-Dimethylamino-ethyl)-5-fluoro-benzene-1,2,4-triamine dans 30 mL de méthanol avec 1,06 g de 4-Nitro-1-(tetrahydro-pyran-2-yl)-1H-pyrazole-3-carbaldehyde est agitée à température ambiante pendant 72 heures. Après évaporation du solvant sous vide à l'évaporateur rotatif, le brut réactionnel est purifié par chromatographie flash, sur un appareil Intelliflash sur cartouche RS-90 Analogix avec un éluant dichlorométhane 100% à dichlorométhane/méthanol 85 /15. 350 mg de N1-{6-Fluoro-2-[4-nitro-1-(tetrahydro-pyran-2-yl)-1H-pyrazol-3-yl]-3H-benzimidazol-5-yl}-N2,N2-dimethyl-ethane-1,2-diamine sont isolés sous forme de mousse orange. Spectre de masse en ES: 418(+)=(M+H)(+); 334(+)=(M+H)(+)-THP+H, 209,6=(M+2H)(2+)

### Etape IV : N1-{2-[4-Amino-1-(tetrahydro-pyran-2-yl)-1 H-pyrazol-3-yl]-6-fluoro-3H-benzimidazol-5-yl}-N2,N2-dimethyl-ethane-1,2-diamine

350 mg de N1-{6-Fluoro-2-[4-nitro-1-(tetrahydro-pyran-2-yl)-1H-pyrazol-3-yl]-3H-benzimidazol-5-yl}-N2,N2-dimethyl-ethane-1,2-diamine dans 15 mL de méthanol et 40 mg de palladium sur charbon est hydrogénée sous 1 bar de pression d'hydrogène à 22°C pendant 16 heures. Le milieu réactionnel est filtré sur célite et le filtrat concentré sous pression réduite à l'évaporateur rotatif. 300 mg de N1-{2-[4-Amino-1-(tetrahydro-pyran-2-yl)-1H-pyrazol-3-yl]-6-fluoro-3H-benzimidazol-5-yl}-N2,N2-dimethyl-ethane-1,2-diamine. Le composé est utilisé tel quel pour l'étape suivante.

### Etape V : 3-[3-[6-(2-Dimethylamino-ethylamino)-5-fluoro-1H-benzimidazol-2-yl]-1-(tetrahydro-pyran-2-yl)-1H-pyrazol-4-yl]-1,1-diethyl-urée

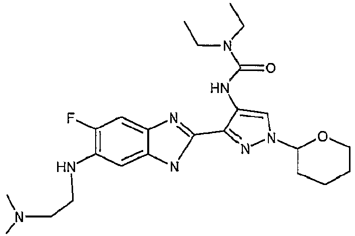

Une solution de 132 mg de N1-{2-[4-Amino-1-(tetrahydro-pyran-2-yl)-1H-pyrazol-3-yl]-6-fluoro-3H-benzimidazol-5-yl}-N2,N2-dimethyl-ethane-1,2-diamine dans 8 mL de tétrahydrofuranne avec 428 µL de chlorure de diéthylcarbamoyle et 560 µL de N, N-diisopropyléthylamine est chauffée à 55°C pendant 48 heures. On ajoute 15 mL d'eau puis la phase aqueuse est extraite par 3x15 mL d'acétate d'éthyle. Les phases organiques sont lavées par 2x15 mL d'eau distillée, séchées sur sulfate de magnésium, filtrées et concentrées sous vide à l'évaporateur rotatif. Le brut réactionnel est purifié par chromatographie Flash sur un appareil Intelliflash sur cartouche RS-12 Analogix, avec un éluant dichlorométhane/méthanol 95/5. 18 mg de 3-[3-[6-(2-Dimethylamino-ethylamino)-5-fluoro-1H-benzimidazol-2-yl]-1-(tetrahydro-pyran-2-yl)-1H-pyrazol-4-yl]-1,1-diethyl-urea sont isolés sous forme de résine jaune. RMN ¹H (400 MHz, DMSO-d6, δ en ppm), après addition d'une goutte de TFA-d1 (acide trifluoroacétique CF3COOD-d1) et d'une goutte d'acide acétique-d4-(CD3OD-d4) : 1,16 (t, J=7,5 Hz, 6H) ; 1,56 (m, 2H) ; 1,70 (m, 1 H) ; 1,97 (m, 2H) ; 2,14 (m, 1 H) ; 2,86 (s, 6H) ; 3,37 (m, 6H) ; 3,50 (t, J=6,5 Hz, 2H) ; 3,68 (m, 1 H) ; 3,96 (m, 1 H) ; 5,48 (d large, J=10,0 Hz, 1 H) ; 6,88 (d, J=7,5 Hz, 1H) ; 7,42 (d, J=11,0 Hz, 1 H) ; 8,11 (s, 1 H) .

On recueille aussi 100 mg de 1-{2-[4-(3,3-Diethyl-ureido)-1-(tetrahydro-pyran-2-yl)-1H-pyrazol-3-yl]-6-fluoro-3H-benzimidazol-5-yl}-1-(2-dimethylamino-ethyl)-3,3-diethyl-urée 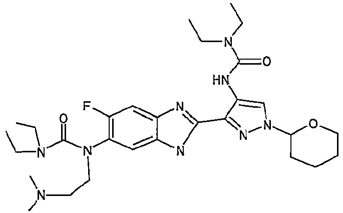 qui est utilisé pour l'exemple 14. RMN ¹H (400 MHz, DMSO-d6, δ en ppm), après addition d'une goutte de TFA-d1 (CF3COOD-d1) et d'une goutte d'acide acetique-d4-(CD₃OD-d4) :0,73 (t, J=7,0 Hz, 6H) ; 1,21 (t, J=7,0 Hz, 6H); 1,56 (m, 2H) ; 1,70 (m, 1H) ; 1,97 (m, 2H) ; 2,14 (m, 1H) ; 2,83 (s, 6H) ; 3,04 (q, J=7,0 Hz, 4H) ; 3,21 (t, J=6,5 Hz, 2H) ; 3,40 (q, J=7,0 Hz, 4H) ; 3,66 (m, 1H) ; 3,78 (t, J=6,5 Hz, 2H) ; 3,95 (m, 1H) ; 5,47 (dd, J=2,0 et 10,0 Hz, 1H) ; 7,49 (d, J=10,5 Hz, 1H) ; 7,51 (d, J= 7,0 Hz, 1H) ; 8,16 (s, 1H).

### Etape VI 3-{3-[6-(2-Dimethylamino-ethylamino)-5-fluoro-1H-benzimidazol-2-yl]-1H-pyrazol-4-yl}-1,1-diethyl-urée

Une solution de 18 mg de 3-[3-[6-(2-Dimethylamino-ethylamino)-5-fluoro-1H-benzimidazol-2-yl]-1-(tetrahydro-pyran-2-yl)-1H-pyrazol-4-yl]-1,1-diethyl-urea dans 1 mL de dichlorométhane et 500 µL d'acide trifluoroacétique sont agités pendant 72 heures à température ambiante. Après évaporation du solvant sous vide à l'évaporateur rotatif, le brut réactionnel est purifié par chromatographie Flash sur cartouche RS-4 Analogix avec un éluant dichlorométhane 100% à dichlorométhane/ méthanol ammoniacal 90/10. 10 mg de 3-{3-[6-(2-dimethylamino-ethylamino)-5-fluoro-1H-benzimidazol-2-yl]-1H-pyrazol-4-yl}-1,1-diethyl-urée sous forme de trifluoroacétate sont isolés. RMN ¹H (300 MHz, DMSO-d6, δ en ppm), après addition D'une goutte de TFA-d1 (CF3COOD-d1) et d'une goutte d'acide acetique-d4-(CD3OD-d4) : 1,16 (t, J=7,0 Hz, 6H) ; 2,87 (s, 6H) ; 3,38 (m, 6H) ; 3,51 (t, J=6,0 Hz, 2H) ; 6,89 (d, J=7,5 Hz, 1 H) ; 7,44 (d, J=11,0 Hz, 1 H) ; 7,98 (s, 1H).

### Exemple 16 : 3-[3-(5-Fluoro-6-morpholin-4-yl-1H-benzimidazol-2-yl)-1H-pyrazol-4-yl]-1,1-diisopropyl-urée sous forme de sel de trifluoroacétate

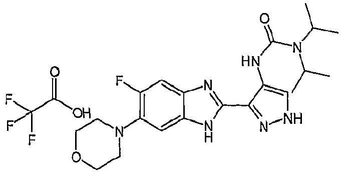

### Etape V : 3-[3-(5-Fluoro-6-morpholin-4-yl-1H-benzimidazol-2-yl)-1-(tetrahydro-pyran-2-yl)-1H-pyrazol-4-yl]-1,1-diisopropylurée

Une solution de 300 mg d'intermédiaire Gamma dans 33 mL de Tétrahydrofuranne est agitée à température ambiante, puis 676 µL de N,N-diisopropylethylamine et 127 mg de chlorure de diisopropylcarbamoyle sont additionnés. La solution est chauffée au reflux. On ajoute du chlorure de diisopropylcarbamoyle jusqu'à ce que la réaction soit complète. Le milieu réactionnel est traité par 20 mL d'une solution saturée de chlorure de sodium puis la phase aqueuse est extraite par 3x30 mL d'acétate d'éthyle. Les phases organiques sont séchées sur sulfate de magnésium, et concentrées sous vide à l'évaporateur rotatif. 550 mg de 3-[3-(5-Fluoro-6-morpholin-4-yl-1H-benzimidazol-2-yl)-1-(tetrahydro-pyran-2-yl)-1H-pyrazol-4-yl]-1,1-diisopropyl-urea sont isolés sous forme de solide brun. LC/MS : Tr=4,53 min, [M+H]+ = 514,24, UV254 nm = 59%

### Etape VI 3-[3-(5-Fluoro-6-morpholin-4-yl-1H-benzimidazol-2-yl)-1H-pyrazol-4-yl]-1,1-diisopropyl-urée sous forme de sel de trifluoroacétate

Une solution de 550 mg de 3-[3-(5-Fluoro-6-morpholin-4-yl-1H-benzimidazol-2-yl)-1-(tetrahydro-pyran-2-yl)-1H-pyrazol-4-yl]-1,1-diisopropyl-urea dans 8,5 mL d'acide chlorhydrique concentré est agitée à température ambiante pendant 12 heures et le milieu est concentré sous vide à l'évaporateur rotatif. Le brut réactionnel est purifié par LC/MS préparative en éluant avec un mélange eau et acétonitrile contenant respectivement 0,07% d'acide trifluoroacétique. 183 mg de 3-[3-(5-Fluoro-6-morpholin-4-yl-1H-benzimidazol-2-yl)-1H-pyrazol-4-yl]-1,1-diisopropyl-urée sous forme de sel de trifluoroacétate sont isolés. RMN ¹H (400 MHz, DMSO-d6, δ en ppm) : 1,35 (d, J=7,0 Hz, 12H) ; 3,00 (m, 4H) ; 3,78 (m, 4H) ; 4,07 (m, 2H) ; 7,08 (d large, J = 7,5 Hz, 1H) ; 7,32 (d large, J=12,0 Hz, 1H) ; 8,03 (s, 1 H) ; 9,57 (s, 1H) ; 12,95 (m étalé, 2H).

### Exemple 17: Trifluoroacétate de N-{3-[5-fluoro-6-(4-methyl-perhydro-1,4-diazepin-1-yl)-1H-benzimidazol-2-yl]-1H-pyrazol-4-yl}-Piperidine-1-carboxamide

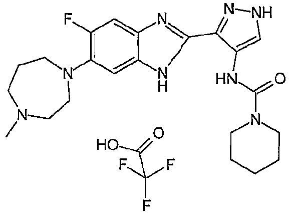

### Etape I : 4-Fluoro-5-(4-methyl-perhydro-1,4-diazepin-1-yl)-2-nitro-phenylamine

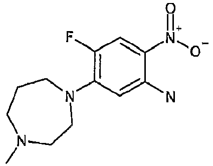

A une solution de 15 g de 4,5-difluoro-2-nitroaniline dans 120 mL de DMF anhydre, 36,2 g de bicarbonate de sodium et 29,5 g N-méthylhomopipérazine sont ajoutés. Le milieu réactionnel est chauffé à 80°C à l'aide d'un bain d'huile pendant 2H30. Le milieu réactionnel est refroidi à température ambiante, puis versé dans 400 mL d'eau. On refroidit à l'aide d'un bain de glace et on agite, il y a précipitation. Le précipité est filtré sur verre fritté. Le solide jaune est rincé à l'eau. Le solide est séché à l'étuve à 40°C. 21 g de 4-Fluoro-5-(4-methyl-perhydro-1,4-diazepin-1-yl)-2-nitro-phenylamine sont obtenus sous forme de solide jaune. RMN ¹H (300 MHz, DMSO-d6, δ en ppm) : 1,90 (m, 2H) ; 2,27 (s, 3H) ; 2,50 (m masqué, 2H) ; 2,64 (m, 2H) ; 3,46 (m, 2H) ; 3,52 (m, 2H) ; 6,21 (d, J=9,0 Hz, 1 H) ; 7,24 (s large, 2H) ; 7,59 (d, J=16,0 Hz, 1H). Spectre de masse en IE : m/z=268: M+. (pic de base) - m/z=253 : (M - CH3)+ - m/z=198 : (M - C4H8N)+ - m/z=70 : C4H8N+ - m/z=57 : C3H7N+.

### Etape II : 4-Fluoro-5-(4-methyl-perhydro-1,4-diazepin-1-yl)-benzene-1,2-diamine

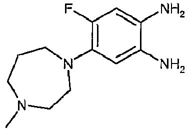

20,9 g de 4-Fluoro-5-(4-methyl-perhydro-1,4-diazepin-1-yl)-2-nitro-phenylamine en solution dans 750 mL de méthanol sont hydrogénés sous 1 bar de pression d'hydrogène en présence de 2 g de palladium sur charbon à 25°C pendant 16 heures. Le brut réactionnel est filtré sur célite et le filtrat est concentré sous vide à l'évaporateur rotatif. 19,1 g de 4-Fluoro-5-(4-methyl-perhydro-1,4-diazepin-1-yl)-benzene-1,2-diamine sous forme d'une huile noire sont isolés. RMN ¹H (300 MHz, DMSO-d6, δ en ppm) : 1,82 (m, 2H) ; 2,26 (s, 3H) ; 2,56 (m, 4H) ; 3,11 (m, 4H) ; 4,19 (m large, 4H) ; de 6,22 à 6,31 (m, 2H). IE m/z=238 : M+. (pic de base) - m/z=223 : (M - CH3)+ - m/z=168 : (M - C4H8N)+ - m/z=70 : C4H8N+ - m/z=57 : C3H7N+.

### Etape III : 5-Fluoro-6-(4-methyl-parhydro-1,4-diazepin-1-yl)-2-[4-nitro-1-(tetrahydro-pyran-2-yl)-1H-pyrazol-3-yl]-1H-benzimidazole

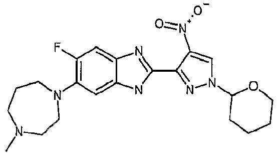

A une solution de 19,1 g de 4-Fluoro-5-(4-methyl-perhydro-1,4-diazepin-1-yl)-benzene-1,2-diamine dans 406 mL de DMF, 18 g de 4-nitro-1-(tetrahydro-pyran-2-yl)-1H-pyrazole-3-carbaldehyde et 664 mg de chlorure ferrique sont ajoutés. Le milieu réactionnel est agité à 22°C pendant 4 heures. Après évaporation, le brut réactionnel est purifié sur une colonne de diamètre 8 cm, avec 3 kg de silice de porosité 0,063-0,2 mm. L'élution se fait avec 2 L de dichlorométhane pur puis 4 L de dichlorométhane à 10% de méthanol, puis avec du dichlorométhane à 15% de méthanol. 12 g de 5-Fluoro-6-(4-methyl-perhydro-1,4-diazepin-1-yl)-2-[4-nitro-1-(tetrahydro-pyran-2-yl)-1H-pyrazol-3-yl]-1H-benzimidazole sont isolés. RMN ¹H (400 MHz, DMSO-d6, 8 en ppm) : 1,41 à 2,31 (m, 8H) ; 2,91 (s, 3H) ; de 3,22 à 3,67 (m, 8H) ; 3,72 (m, 1H) ; 3,99 (m, 1H) ; 5,69 (dd, J=2,5 et 9,5 Hz, 1H) ; 7,32 (d, J=8,0 Hz, 1H) ; 7,75 (d, J=12,0 Hz, 1H); 9,40 (s, 1H). Spectre de masse en IE : m/z=443 : M+. - m/z=373 : (M-C4H8N)+ - m/z=289 : (m/z=373 - C5H8O)+ - m/z=84 : C5H8O+.(pic de base) - m/z=57 : C3H7N+.

### Etape IV : 3-[5-Fluoro-6-(4-methyl-perhydro-1,4-diazepin-1-yl)-1H-benzimidazol-2-yl]-1-(tetrahydro-pyran-2-yl)-1H-pyrazol-4-ylamine (Intermédiaire 4)

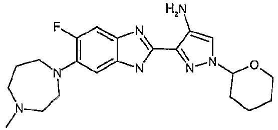

Une suspension de 11,9 g de 5-Fluoro-6-(4-methyl-perhydro-1,4-diazepin-1-yl)-2-[4-nitro-1-(tetrahydro-pyran-2-yl)-1H-pyrazol-3-yl]-1H-benzimidazole dans 260 mL de méthanol et 1,2 g de palladium sur charbon est hydrogénée sous 1 bar de pression d'hydrogène à température ambiante pendant 18 heures. Le milieu réactionnel est filtré sur célite et le filtrat concentré sous pression réduite à l'évaporateur rotatif. 10,9 g de 3-[5-Fluoro-6-(4-methyl-perhydro-1,4-diazepin-1-yl)-1H-benzimidazol-2-yl]-1-(tetrahydro-pyran-2-yl)-1 H-pyrazol-4-ylamine sont obtenus sous forme d'une poudre brune. RMN ¹H (300 MHz, DMSO-d6, δ en ppm) : 1,43 à 2,17 (m, 8H) ; 2,33 (s, 3H) ; 2,64 (m, 2H) ; 2,73 (m, 2H) ; de 3,20 à 3,40 (m masqué, 4H) ; 3,62 (m, 1 H) ; 3,95 (m, 1H) ; 4,92 (s large, 2H) ; 5,31 (dd, J=2,5 et 10,0 Hz, 1H) ; de 6,91 à 7,36 (m, 2H) ; 7,31 (s, 1H) ; 12,4 (m étalé, 1H). Spectre de masse en ES : m/z=414 : MH+ (pic de base) - m/z=330 : (MH - C5H8O)+

### Etape V : N-[3-[5-fluoro-6-(4-methyl-perhydro-1,4-diazepin-1-yl)-1H-benzimidazol-2-yl]-1-(tetrahydro-pyran-2-yl)-1H-pyrazol-4-yl]-Piperidine 1-carboxamide

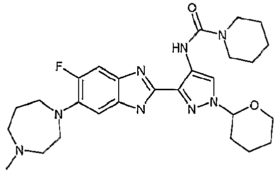

A une solution de 8 g d'intermédiaire 4 dans 260 mL de THF, 12,1 mL de chlorure de 1-piperidinecarbonyle et 16,8 mL de N,N-diiopropyléthylamine sont additionnés. Le mélange réactionnel est chauffé à 66°C pendant 5 heures. Le milieu réactionnel est refroidi à température ambiante et on ajoute 100 mL d'eau. La phase aqueuse est extraite avec 3 fois 250 mL d'AcOEt. Les phases organiques sont séchées sur MgSO₄, filtrées et concentrées sous vide à l'évaporateur rotatif. Le brut réactionnel est purifié par chromatographie flash en éluant avec un mélange dichlorométhane / méthanol : 5,6 g de Piperidine-1-carboxylic acid [3-[5-fluoro-6-(4-methyl-perhydro-1,4-diazepin-1-yl)-1 H-benzimidazol-2-yl]-1-(tetrahydro-pyran-2-yl)-1H-pyrazol-4-yl]-amide sont obtenus sous forme d'une résine jaune. RMN ¹H (400 MHz, DMSO-d6, δ en ppm) : 1,43 à 2,17 (m, 14H) ; 2,30 (s, 3H) ; 2,60 (m, 2H) ; 2,70 (m, 2H) ; 3,30 (m masqué, 4H) ; 3,49 (m, 4H) ; 3,66 (m, 1H) ; 3,93 (m, 1H) ; 5,46 (dd, J=2,5 et 10,0 Hz, 1 H) ; de 6,92 à 7,42 (m étalé, 2H) ; 8,12 (s, 1 H) ; 9,96 (s, 1 H) ; 12,85 (m étalé, 1H). Spectre de masse en ES : m/z=525 : MH+ - m/z=441 : (MH - C5H8O)+ - m/z=85 : C5H9O+ (pic de base)

### Etape VI : Trifluoroacétate de N-{3-[5-fluoro-6-(4-methyl-perhydro-1,4-diazepin-1-yl)-1H-benzimidazol-2-yl]-1H-pyrazol-4-yl}-Piperidine-1-carboxamide

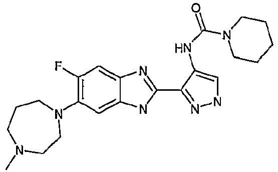

A une solution de 60 mg de N-[3-[5-fluoro-6-(4-methyl-perhydro-1,4-diazepin-1-yl)-1H-benzimidazol-2-yl]-1-(tetrahydro-pyran-2-yl)-1H-pyrazol-4-yl]-Piperidine 1-carboxamide en solution dans 1 mL de dichlorométhane, 0,5 mL d'acide trifluoroacétique. Le milieu réactionnel est agité à 22°C pendant 16 heures. Le milieu réactionnel est concentré sous vide à l'évaporateur rotatif. Après évaporation, le brut réactionnel est purifié par chromatographie flash. L'élution se fait avec du dichlorométhane pur puis avec un mélange de dichlorométhane contenant 5% de méthanol ammoniacal. 29 mg de N-{3-[5-fluoro-6-(4-methyl-perhydro-1,4-diazepin-1-yl)-1H-benzimidazol-2-yl]-1H-pyrazol-4-yl}-Piperidine-1-carboxamide sont obtenus sous forme d'un solide jaune. RMN ¹H (400 MHz, DMSO-d6, δ en ppm) : 1,42 à 2,27 (m, 8H) ; 2,90 (s, 3H) ; de 3,18 à 3,65 (m, 12H) ; 7,20 (d, J=7,0 Hz, 1H) ; 7,56 (d, J = 12,0 Hz, 1 H) ; 8,00 (s, 1 H). Spectre de masse en ES : m/z=439 (M - H) - pic de base

### Exemple 18: 1,1-Diethyl-3-{3-[5-fluoro-6-(4-methyl-perhydro-1,4-diazepin-1-yl)-1H-benzimidazol-2-yl]-1H-pyrazol-4-yl-urée (2 étapes à partir de l'intermédiaire 4)

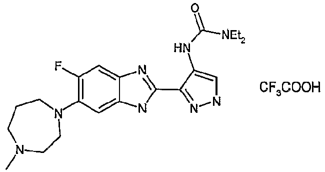

### Etape V : 1,1-Diethyl-3-[3-[5-fluoro-6-(4-methyl-perhydro-1,4-diazepin-1-yl)-1H-benzimidazol-2-yl]-1-(tetrahydro-pyran-2-yl)-1 H-pyrazol-4-yl]-urée

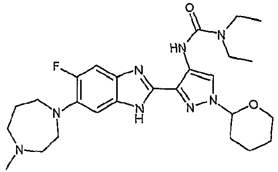

A une solution de 118 mg d'**intermédiaire 4** dans 7 mL de THF, 362 µL de chlorure de diethylcarbamoyle et 500 µL de N,N-diisopropyléthylamine sont additionnés. Le mélange réactionnel est chauffé à 55°C pendant 16 heures, puis 2 heures à 80°C. Le milieu réactionnel est refroidi à température ambiante et on ajoute 20 mL d'eau distillée. La phase aqueuse est extraite avec 3 fois 10 mL d'AcOEt. Les phases organiques sont séchées sur MgSO₄, filtrées et concentrées sous vide à l'évaporateur rotatif. Le brut réactionnel est purifié par chromatographie flash en éluant avec un mélange dichlorométhane/méthanol. 39 mg de 1,1-Diethyl-3-[3-[5-fluoro-6-(4-methyl-perhydro-1,4-diazepin-1-yl)-1H-benzimidazol-2-yl]-1-(tetrahydro-pyran-2-yl)-1 H-pyrazol-4-yl]-urea sont obtenus sous forme d'une résine jaune. RMN ¹H (400 MHz, DMSO-d6, δ en ppm) : 1,12 (t, J=7,0 Hz, 6H) ; de 1,50 à 2,26 (m, 8H) ; 2,90 (s, 3H) ; de 3,18 à 3,63 (m, 8H); 3,34 (q, J=7,0 Hz, 4H) ; 3,68 (m, 1H) ; 3,96 (m, 1H); 5,49 (dd, J=2,5 et 10,0 Hz, 1H) ; 7,19 (d, J=7,5 Hz, 1H) ; 7,52 (d, J=12,0 Hz, 1 H) ; 8,12 (s, 1H). Spectre de masse en ES:m/z=513: MH+ - m/z=429 (MH - C5H8O)+(pic de base) - m/z=356 : (m/z=429 - C4H11 N)+ - m/z=85 : C5H9O+

### Etape VI : 1,1-Diethyl-3-{3-[5-fluoro-6-(4-methyl-perhydro-1,4-diazepin-1-yl)-1H-benzimidazol-2-yl]-1H-pyrazol-4-yl}-urée sous forme de sel de trifluoroacétate

A une solution de 39 mg de 1,1-Diethyl-3-[3-[5-fluoro-6-(4-methyl-perhydro-1,4-diazepin-1-yl)-1H-benzimidazol-2-yl]-1-(tetrahydro-pyran-2-yl)-1H-pyrazol-4-yl]-urea en solution dans 1 mL de dichlorométhane, 0,5 mL d'acide trifluoroacétique. Le milieu réactionnel est agité à 22°C pendant 16 heures. Le milieu réactionnel est concentré sous vide à l'évaporateur rotatif. Après évaporation, le brut réactionnel est purifié par chromatographie flash sur un appareil INTELLIFLASH sur cartouche RS-4 Analogix. L'élution se fait avec du dichlorométhane pur puis avec du dichlorométhane contenant 5% de méthanol ammoniacal. 12 mg de sel de trifluoroacétate de 1,1-Diethyl-3-{3-[5-fluoro-6-(4-methyl-perhydro-1,4-diazepin-1-yl)-1H-benzimidazol-2-yl]-1H-pyrazol-4-yl}-urée sont obtenus sous forme d'une résine noire. RMN ¹H (300 MHz, DMSO-d6, δ en ppm): 1,10 (t, J=7,0 Hz, 6H) ; 2,20 (m large, 2H) ; 2,89 (s, 3H) ; de 3,07 à 3,68 (m, 8H) ; 3,34 (q, J=7,0 Hz, 4H) ; 7,22 (d, J=7,5 Hz, 1 H) ; 7,55 (d, J=12,0 Hz, 1 H) ; 7,96 (s, 1 H). Spectre de masse en IE : m/z=428 M+., m/z=355 (M - C4H11 N)+, m/z=69 CF3+ (relatif au TFA), m/z=58 C3H8N+(pic de base), m/z=45 CO2H+ (relatif au TFA).

### Exemple de référence 19: 1,1-Diethyl-3-{3-[6-(4-methyl-piperazin-1-yl)-1H-benzimidazol-2-yl]-1H-pyrazol-4-yl}-urée

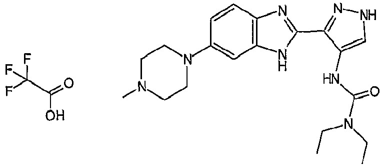

### Etape I : 6-(4-méthyl-pipérazin-1-yl)-2-[4-nitro-1-(tetrahydro-pyran-2-yl)-1H-pyrazol-3-yl]-1H-benzimidazole

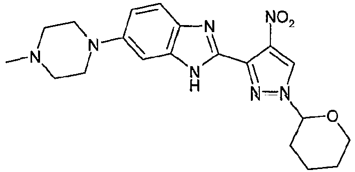

A une solution de 680 mg de 4-(4-Methylpiperazino)-1,2-benzenediamine dans 30 mL de méthanol, 742 mg de 4-Nitro-1-(tetrahydro-pyran-2-yl)-1 H-pyrazole-3-carbaldehyde sont ajoutés. Le milieu réactionnel est agité à 22°C pendant 3 heures. Le milieu réactionnel est chauffé à 60°C à l'aide d'un bain d'huile pendant 1H30, puis agité à 22°C pendant 64 heures. Après évaporation, le brut réactionnel est purifié par chromatographie flash sur un appareil INTELLIFLASH sur cartouche RS-12 Analogix. L'élution se fait dans le dichlorométhane à 10% de méthanol. 370 mg de 6-(4-Methyl-piperazin-1-yl)-2-[4-nitro-1-(tetrahydro-pyran-2-yl)-1H-pyrazol-3-yl]-1H-benzimidazole sont isolés. LC/MS analytique : tr = 1,88 min; [M+H]⁺ = 412,32; ELSD = 97% ; DAD=93%. RMN ¹H (400 MHz, DMSO-d6, δ en ppm) : 1,50 à 2,23 (m, 6H) ; 2,25 (s, 3H) ; 2,50 (m masqué, 4H) ; 3,14 (m, 4H) ; 3,70 (m, 1H) ; 4,00 (m, 1H) ; 5,59 (d large, J=9,5 Hz, 1 H) ; de 6,90 à 7,15 (m, 2H) ; de 7,39 à 7,54 (m, 1 H) ; 9,17 (s, 1 H) ; de 12,5 à 12,7 (m, 1H).

### Etape II : Préparation du 3-[6-(4-méthyl-pipérazin-1-yl)-1H-benzimidazol-2-yl]-1-(tétrahydro-pyran-2-yl-1H-pyrazol-4-ylamine (intermédiaire 3)

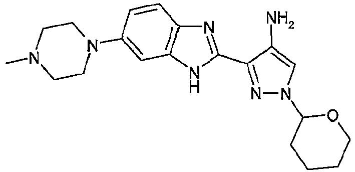

Une suspension de 370 mg de 6-(4-méthyl-pipérazin-1-yl)-2-[4-nitro-1-(tetrahydro-pyran-2-yl)-1H-pyrazol-3-yl]-1H-benzimidazole dans 10 mL de méthanol et 37 mg de palladium sur charbon est hydrogénée sous 1 bar de pression d'hydrogène à température ambiante pendant 18 heures. Le milieu réactionnel est filtré sur célite et le filtrat concentré sous pression réduite à l'évaporateur rotatif. 360 mg de 3-[6-(4-Methyl-piperazin-1-yl)-1H-benzimidazol-2-yl]-1-(tetrahydro-pyran-2-yl)-1H-pyrazol-4-ylamine sont obtenus sous forme d'une huile noire. RMN ¹H (300 MHz, DMSO-d6, δ en ppm) : 1,50 à 2,21 (m, 6H) ; 2,23 (s, 3H) ; 2,50 (m masqué, 4H) ; 3,08 (m, 4H) ; 3,61 (m, 1 H) ; 3,94 (m, 1 H) ; de 4,89 à 4,98 (m, 2H) ; 5,30 (d large, J=10,0 Hz, 1H) ; de 6,85 à 7,12 (m, 2H) ; de 7,25 à 7,46 (m, 2H) ; de 12,2 à 12,35 (m, 1H).

### Procédure A pour la préparation des dérivés urées du type décrit ci-dessous

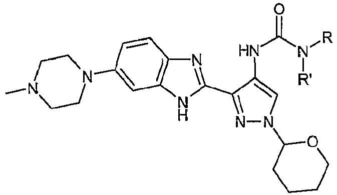

A une solution de 180 mg d'**intermédiaire 3** dans 10 mL de THF, 5 éq de chlorure de carbamoyle et 5 éq de N,N-diisopropyléthylamine sont additionnés. Le mélange réactionnel est chauffé à 52°C pendant 48 heures dans un tube RADLEY. Le milieu réactionnel est refroidi à température ambiante et on additionne 10 mL d'une solution saturée de bicarbonate de sodium. La phase aqueuse est extraite avec 3 fois 10 mL d'AcOEt. Les phases organiques sont séchées sur MgSO₄, filtrées et concentrées sous vide à l'évaporateur rotatif. Le concentrat est utilisé immédiatement dans l'étape suivante.

### Etape III: Préparation du 1,1-Diethyl-3-[3-[6-(4-methyl-piperazin-1-yl)-1H-benzimidazol-2-yl]-1-(tetrahydro-pyran-2-yl)-1 H-pyrazol-4-yl]-urée

Selon la **procédure A,** en présence de chlorure de diéthylcarbamoyle, 210 mg de 1-Diethyl-3-[3-[6-(4-methyl-piperazin-1-yl)-1H-benzimidazol-2-yl]-1-(tetrahydro-pyran-2-yl)-1 H-pyrazol-4-yl]-urée sont obtenus.

### Etape IV: Préparation du 1,1-diéthyl-3-{3-[6-(4-méthyl-pipérazin-1-yl)-1H-benzimidazol-2-yl]-1H-pyrazol-4-yl}-urée

A une solution de 210 mg de 1-diéthyl-3-[3-[6-(4-méthyl-pipérazin-1-yl)-1H-benzimidazol-2-yl]-1-(tétrahydro-pyran-2-yl)-1 H-pyrazol-4-yl]-urea en solution dans 2,22 mL de dichlorométhane, 742 µL d'acide trifluoroacétique et 100 µL d'eau sont ajoutés. Le milieu réactionnel est agité à 22°C pendant 48 heures. Le mélange réactionnel est chauffé à 50°C pendant 10 minutes. Après évaporation, le brut réactionnel est purifié par chromatographie flash. L'élution se fait dans le dichlorométhane à 15% de méthanol. 80 mg de 1,1-Diethyl-3-{3-[6-(4-methyl-piperazin-1-yl)-1H-benzimidazol-2-yl]-1H-pyrazol-4-yl}-urée sont isolés. 61 mg de 1,1-Diethyl-3-[3-[6-(4-methyl-piperazin-1-yl)-1H-benzimidazol-2-yl]-1-(tetrahydro-pyran-2-yl)-1 H-pyrazol-4-yl]-urée sont isolés et remis en réaction avec 2,2 mL de TFA et 550 µL d'eau. Le milieu réactionnel est agité à 22°C pendant 120 heures. Après évaporation, le brut réactionnel est purifié par chromatographie flash sur un appareil Intelliflash sur cartouche RS-4 Analogix. L'élution se fait dans le dichlorométhane à 15% de méthanol. 17 mg de 1,1-diéthyl-3-{3-[6-(4-methyl-piperazin-1-yl)-1H-benzimidazol-2-yl]-1H-pyrazol-4-yl}-urée sont isolés. Soit 92 mg de 1,1-diéthyl-3-{3-[6-(4-méthyl-pipérazin-1-yl)-1H-benzimidazol-2-yl]-1H-pyrazol-4-yl}-urée sont isolés au total. RMN ¹H (400 MHz, DMSO-d6, δ en ppm) : 1,16 (t, J=7,0 Hz, 6H) ; 2,93 (s, 3H) ; 3,11 (m, 2H) ; 3,26 (m, 2H) ; 3,39 (q, J=7,0 Hz, 4H) ; 3,60 (m, 2H) ; 3,89 (m, 2H) ; 7,20 (d, J=1,5 Hz, 1 H) ; 7,31 (dd, J=1,5 et 9,0 Hz, 1H) ; 7,66 (d, J=9,0 Hz, 1 H) ; 7,89 (s, 1H).

### Exemple de référence 20: N-{3-[6-(4-methyl-piperazin-1-yl)-1H-benzimidazol-2-yl]-1H-pyrazol-4-yl}- Piperidine-1-carboxamide (en 2 étapes à partir de l'intermédiaire 3)

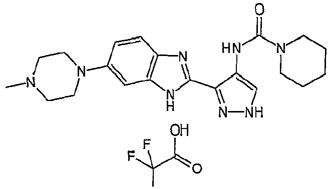

### Etape III : Préparation du N-[3-[6-(4-methyl-piperazin-1-yl)-1H-benzimidazol-2-yl]-1-(tetrahydro-pyran-2-yl)-1H-pyrazol-4-yl]-piperidine-1-carbox amide

Selon la **Procédure A,** en présence de chlorure de Piperidine-1-carbonyle, 290 mg de N-[3-[6-(4-methyl-piperazin-1-yl)-1H-benzimidazol-2-yl]-1-(tetrahydro-pyran-2-yl)-1H-pyrazol-4-yl]-Piperidine-1-carboxamide sont obtenus.

### Etape IV : Préparation du N-{3-[6-(4-methyl-piperazin-1-yl)-1H-benzimidazol-2-yl]-1H-pyrazol-4-yl}-Piperidine-1-carboxamide

A une solution de 290 mg de N-[3-[6-(4-methyl-piperazin-1-yl)-1H-benzimidazol-2-yl]-1-(tetrahydro-pyran-2-yl)-1 H-pyrazol-4-yl]-Piperidine-1-carboxamide en solution dans 3 mL de dichlorométhane, 1 mL d'acide trifluoroacétique et 100 µL d'eau sont ajoutés. Le milieu réactionnel est agité à 22°C pendant 24 heures. Le milieu réactionnel est concentré sous vide à l'évaporateur rotatif. On rajoute 0,5 mL de dichlorométhane, 0,5 mL d'acide trifluoroacétique et 100 µL d'eau. Le milieu réactionnel est agité à 22°C pendant 4 heures. Après évaporation, le brut réactionnel est purifié par chromatographie flash sur un appareil Intelliflash sur cartouche RS-12 Analogix. L'élution se fait dans le dichlorométhane à 15% de méthanol. 40 mg de N-{3-[6-(4-methyl-piperazin-1-yl)-1H-benzimidazol-2-yl]-1H-pyrazol-4-yl}-Piperidine-1-carboxamide sont isolés. 123 mg de N-[3-[6-(4-methyl-piperazin-1-yl)-1 H-benzimidazol-2-yl]-1-(tétrahydro-pyran-2-yl)-1H-pyrazol-4-yl]-pipéridine-1 carboxamide sont isolés et remis en réaction avec 2,8 mL de TFA et 700 µL d'eau. Le milieu réactionnel est agité à 22°C pendant 72 heures. Après évaporation, le brut réactionnel est purifié par chromatographie flash sur un appareil Intelliflash sur cartouche RS-12 Analogix. L'élution se fait dans le dichlorométhane à 15% de méthanol. 37 mg de N {3-[6-(4-methyl-piperazin-1-yl)-1H-benzimidazol-2-yl]-1H-pyrazol-4-yl}-Piperidine-1-carboxamide sont isolés. Soit 77 mg de N-{3-[6-(4-méthyl-pipérazin-1-yl)-1H-benzimidazol-2-yl]-1H-pyrazol-4-yl}-pipéridine-1-carboxamide sont isolés au total. RMN ¹H (400 MHz, DMSO-d6, δ en ppm) : 1,43 à 1,63 (m, 6H) ; 2,90 (s, 3H) ; 3,05 (m, 2H) ; 3,22 (m, 2H) ; 3,43 (m, 4H) ; 3,58 (m, 2H) ; 3,89 (m, 2H) ; 7,17 (s large, 1H) ; 7,31 (d large, J=9,0 Hz, 1H) ; 7,65 (d, J=9,0 Hz, 1H) ; 8,02 (s, 1H).

### Exemple 21 : N-[3-(5-fluoro-6-piperazin-1-yl-1H-benzimidazol-2-yl)-1H-pyrazol-4-yl]-pipéridine-1-carboxamide

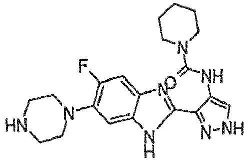

### Etape I : Tert-butyl ester de l'acide 4-(5-amino-2-fluoro-4-nitro-phényl)-pipérazine-1-carboxylique

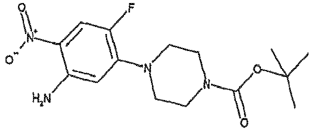

A une solution de 3,3 g de tert-butyl ester de l'acide piperazine-1-carboxylique dans 30 mL de DMF anhydre, 2,4 g de bicarbonate de potassium et 1 g 4,5-difluoro-2-nitroaniline sont ajoutés. Le milieu réactionnel est chauffé à 60°C à l'aide d'un bain d'huile pendant 30 minutes. Le milieu réactionnel est refroidi à température ambiante, puis on y ajoute 150 mL d'eau, il y a précipitation. Le précipité est filtré sur verre fritté. Le solide est séché à l'étuve à 50°C. 1,4 g de tert-butyl ester de l'acide 4-(5-Amino-2-fluoro-4-nitro-phenyl)-pipérazine-1-carboxylique sont obtenus sous forme de solide jaune. RMN ¹H (300 MHz, DMSO-d6, δ en ppm) : 1,42 (s, 9H) ; 3,14 (m, 4H) ; 3,46 (m, 4H) ; 6,43 (d, J = 8,0 Hz, 1 H) ; 7,36 (s large, 2H) ; 7,66 (d, J = 14,5 Hz, 1H). Spectre de masse en IE : m/z=340 M+., m/z=284 (M - C4H8)+., m/z=264 (m/z=284 - HF)+., m/z=57 C4H9+(pic de base)

### Etape II : Tert-butyl ester de l'acide 4-(4,5-Diamino-2-fluoro-phenyl)-piperazine-1-carboxylique

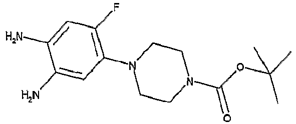

1,4 g de 4-(5-Amino-2-fluoro-4-nitro-phenyl)-piperazine-1-carboxylic acid tert-butyl ester en solution dans 20 mL de méthanol sont hydrogénés sous 1 bar de pression d'hydrogène en présence de 139 mg de palladium sur charbon à 22°C pendant 16 heures. Le brut réactionnel est filtré sur célite et le filtrat est concentré sous vide à l'évaporateur rotatif. 1,4 g de tert butyl ester de l'acide 4-(4,5-diamino-2-fluoro-phényl)-pipérazine-1-carboxylique sont isolés sous forme d'une résine brune. RMN ¹H (300 MHz, DMSO-d6, δ en ppm) : 1,41 (s, 9H) ; 2,73 (m, 4H) ; 3,40 (m, 4H) ; 4,23 (s large, 2H) ; 4,37 (s large, 2H) ; 6,27 (d, J = 8,5 Hz, 1H) : 6,31 (d, J=13,5 Hz, 1 H). Spectre de masse en IE :m/z=310 M+. (pic de base), m/z=254 (M - C4H8)+., m/z=57 C4H9+

### Etape III : tert butyl ester de l'acide 4-{6-Fluoro-2-[4-nitro-1-(tetrahydro-pyran-2-yl)-1H-pyrazol-3-yl]-3H-benzimidazol-5-yl}-piperazine-1-carboxylique

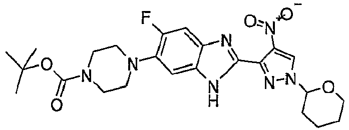

A une solution de 1,2 g de tert butyl ester de l'acide 4-(4,5-diamino-2-fluoro-phenyl)-piperazine-1-carboxylique dans 30 mL de méthanol, 0,9 g de 4-Nitro-1-(tetrahydro-pyran-2-yl)-1H-pyrazole-3-carbaldéhyde sont ajoutés. Le milieu réactionnel est agité à 22°C pendant 16 heures. Après évaporation, le brut réactionnel est purifié par chromatographie flash en éluant dans un mélange dichloromethane/acétone. 530 mg de tert butyl ester de l'acide 4-{6-Fluoro-2-[4-nitro-1-(tetrahydro-pyran-2-yl)-1H-pyrazol-3-yl]-3H-benzimidazol-5-yl}-pipérazine-1-carboxylique sont isolés sous forme d'une mousse rouge. RMN ¹H (400 MHz, DMSO-d6, δ en ppm) : de 1,35 à 2,25 (m, 6H) ; 1,43 (s, 9H) ; 3,03 (m, 4H) ; 3,52 (m, 4H) ; 3,72 (m, 1H) ; 4,00 (m, 1H) ; 5,65 (dd, J=2,0 et 10,0 Hz, 1H) ; 7,31 (d, J=8,0 Hz, 1H) ; 7,54 (d, J=12,0 Hz, 1H) ; 9,17 (s, 1 H). Spectre de masse en ES : m/z=516 MH+ (pic de base).

### Etape IV : Tert-butyl ester de l'acide 4-{2-[4-Amino-1-(tetrahydro-pyran-2-yl)-1H-pyrazol-3-yl]-6-fluoro-3H-benzimidazol-5-yl}-piperazine-1-carboxylique

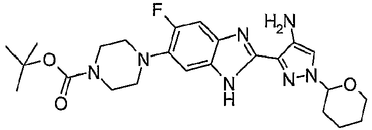

Une suspension de 540 mg de tert-butyl ester de l'acide 4-{6-fluoro-2-[4-nitro-1-(tetrahydro-pyran-2-yl)-1H-pyrazol-3-yl]-3H-benzimidazol-5-yl}-pipérazine-1-carboxylic dans 15 mL de méthanol et 54 mg de palladium sur charbon est hydrogénée sous 1 bar de pression d'hydrogène à température ambiante pendant 16 heures. Le milieu réactionnel est filtré sur célite et le filtrat concentré sous pression réduite à l'évaporateur rotatif. 900 mg de tert butyl ester de l'acide 4-{2-[4-amino-1-(tetrahydro-pyran-2-yl)-1H-pyrazol-3-yl]-6-fluoro-3H-benzimidazol-5-yl}-pipérazine-1-carboxylique sont obtenus sous forme d'un solide noir. RMN ¹H (400 MHz, DMSO-d6, δ en ppm) : de 1,30 à 2,20 (m, 6H) ; 1,42 (s, 9H) ; 3,00 (m, 4H) ; 3,52 (m, 4H) ; 3,67 (m, 1H) ; 3,96 (m, 1 H) ; 5,55 (d large, J=10,0 Hz, 1 H) ; 7,22 (d, J=7,5 Hz, 1 H) ; 7,46 (d, J=12,5 Hz, 1 H) ; 8,17 (s, 1H). Spectre de masse en ES : m/z=486 MH+ (pic de base).

### Etape V : Tert butyl ester de l'acide 4-{6-fluoro-2-[4-[(pipéridine-1-carbonyl)-amino]-1-(tétrahydro-pyran-2-yl)-1H-pyrazol-3-yl]-3H-benzimidazol-5-yl}-pipérazine-1-carboxylique

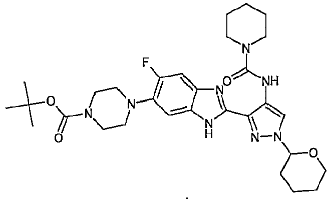

A une solution de 480 mg de 4-{2-[4-amino-1-(tétrahydro-pyran-2-yl)-1H-pyrazol-3-yl]-6-fluoro-3H-benzimidazol-5-yl}-piperazine-1-carboxylic acid tert-butyl ester dans 10 mL de THF, 1,2 mL de chlorure de 1-pipéridinecarbonyle et 1,7 mL de N,N-diisopropyléthylamine sont additionnés. Le mélange réactionnel est chauffé à 80°C pendant 16 heures. Le milieu réactionnel est refroidi à température ambiante et on ajoute 20 mL de solution saturée de bicarbonate de sodium. Les phases aqueuses sont extraites avec 3 fois 10 mL d'AcOEt. Les phases organiques sont séchées sur MgSO₄, filtrées et concentrées sous vide à l'évaporateur rotatif. Le brut réactionnel est purifié par chromatographie flash en éluant dans un mélange cyclohexane/acétate d'éthyle. 350 mg de tert-butyl ester de l'acide 4-{6-fluoro-2-[4-[(pipéridine-1-carbonyl)-amino]-1-(tétrahydro-pyran-2-yl)-1H-pyrazol-3-yl]-3H-benzimidazol-5-yl}-piperazine-1-carboxylique sont obtenus sous forme d'une mousse brune. RMN ¹H (400 MHz, DMSO-d6, δ en ppm) : de 1,37 à 2,22 (m, 12H) ; 1,42 (s, 9H) ; 3,04 (m, 4H) ; 3,44 (m, 4H) ; 3,53 (m, 4H) ; 3,67 (m, 1 H) ; 3,96 (m, 1 H) ; 5,52 (dd, J=2,0 et 10,0 Hz, 1H) ; 7,29 (d, J=7,5 Hz, 1 H) ; 7,56 (d, J= 11,5 Hz, 1H) ; 8,15 (s, 1 H). Spectre de masse en ES : m/z=597 MH+ (pic de base)

### Etape VI: N-[3-(5-fluoro-6-piperazin-1-yl-1H-benzimidazol-2-yl)-1H-pyrazol-4-yl]-Piperidine-1-carboxamide

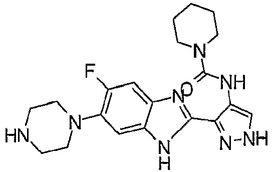

A une solution de 380 mg de tert-butyl ester de l'acide 4-{6-fluoro-2-[4-[(piperidine-1-carbonyl)-amino]-1-(tetrahydro-pyran-2-yl)-1H-pyrazol-3-yl]-3H-benzimidazol-5-yl}-piperazine-1-carboxylique en solution dans 2 mL d'acide trifluoroacétique, 0,5 mL d'eau sont ajoutés. Le milieu réactionnel est agité à 22°C pendant 48 heures. Le milieu réactionnel est concentré sous vide à l'évaporateur rotatif. Le brut réactionnel est purifié par chromatographie flash sur un appareil Intelliflash sur cartouche RS-40 Analogix. L'élution se fait avec du dichlorométhane pur puis avec du dichlorométhane contenant 5% de méthanol ammoniacal. 195 mg de N-[3-(5-fluoro-6-piperazin-1-yl-1H-benzimidazol-2-yl)-1H-pyrazol-4-yl]-piperidine-1-carboxamide sont obtenus sous forme d'un solide jaune. RMN ¹H (400 MHz, DMSO-d6, δ en ppm) : de 1,48 à 1,68 (m, 6H) ; 3,23 (m, 4H) ; 3,30 (m, 4H) ; 3,47 (m, 4H) ; 7,24 (d, J=8,0 Hz, 1 H) ; 7,48 (d, J=11,5 Hz, 1 H) ; 8,01 (s, 1 H). Spectre de masse en ES : m/z=413 MH+, m/z=328 (M + H - C5H11 N)+, m/z=227,9 (M + CH3CN + 2H)2+ / 2 (pic de base). remarque : l'acétonitrile explique le pic m/z=227,9 est dans la phase mobile.

Les exemples 22 à 30, 33 et 36 sont réalisés selon le schéma 4 :

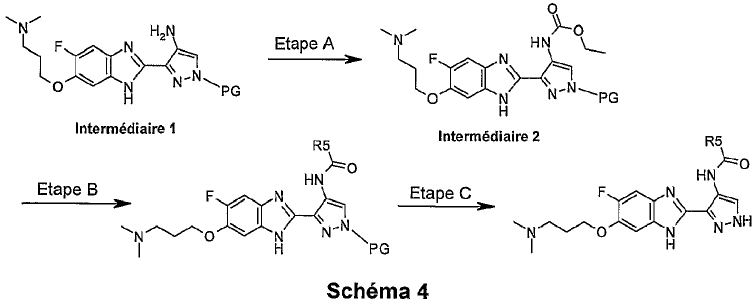

De manière préférée, le groupement protecteur utilisé est le suivant :

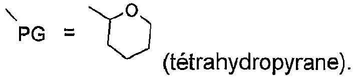

### Etape A : Ethyl ester de l'acide [3-[6-(3-Dimethylamino-propoxy)-5-fluoro-1 H-benzimidazol-2-yl]-1-(tétrahydro-pyran-2-yl)-1 H-pyrazol-4-yl]-carbamique (intermédiaire 2)

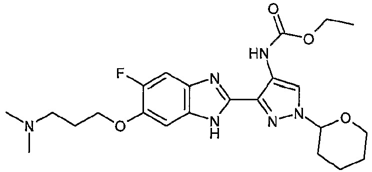

A une solution de 0,3 g d'intermédiaire 1 dans 5 mL de dichlorométhane, 71 µL chloroformate d'ethyle et 103,8 µL de triéthylamine sont additionnés. Le mélange réactionnel est agité à 22°C pendant 2,5 heures. Le milieu réactionnel est lavé avec 5 mL d'eau. La phase aqueuse est extraite avec 3 fois 5 mL de dichlorométhane. Les phases organiques sont séchées sur MgSO₄, filtrées et concentrées sous vide à l'évaporateur rotatif. 307 mg de l'ethyl ester de l'acide [3-[6-(3-diméthylamino-propoxy)-5-fluoro-1H-benzimidazol-2-yl]-1-(tétrahydro-pyran-2-yl)-1H-pyrazol-4-yl]-carbamique sont obtenus sous forme d'une poudre brune. RMN ¹H (300 MHz, DMSO-d6, δ en ppm) : 1,25 (m, 3H) ; 1,60 à 2,18 (m, 6H) ; 2,2 (m, 2H) ; 2,86 (s, 6H) ; 3,3 (t, J=8 Hz, 2H) ; 3,7 (m, 1H) ; 3,9 (m, 1 H) ; 4,2 (m, 4H) ; 5,4 (m, 1 H) ; 7,4 (d, J=8 Hz, 1 H) ; 7,61 (d, J=10,0 Hz, 1H) ; 8,20 (s, 1H).

### Etape B méthode générale :

A une solution de 30 ou 50 mg d'**intermédiaire 2** dans 1 mL de DMF, 50 éq d'amine à substituer sont additionnés. Le mélange réactionnel est irradié pendant 40 min à 200°C au micro-onde sur Personal Synthetiser. Le milieu réactionnel est concentré sous vide à l'évaporateur rotatif. Le concentrat est utilisé immédiatement dans l'étape suivante.

### Etape C méthode générale :

Une solution du concentrat en solution dans 40 éq d'une solution d'acide chlorhydrique 4N dans le dioxanne est agitée à 22 °C pendant environ 4 heures (jusqu'à disparition du produit de départ). Le milieu réactionnel est concentré sous vide à l'évaporateur rotatif. Le concentrât est purifié par LC/MS préparative avec pour éluant un gradient eau, acétonitrile contenant respectivement 0,07 % d'acide trifluoroacétique

### Exemple 22 : Cyclopropyl-3-{3-[6-(3-dimethylamino-propoxy)-5-fluoro-1H-benzimidazol-2-yl]-1H-pyrazol-4-yl-urée

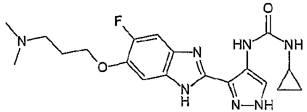

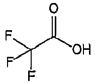

### Etape B : Préparation du 1-Cyclopropyl-3-[3-[6-(3-dimethylamino-propoxy)-5-fluoro-1H-benzimidazol-2-yl]-1-(tetrahydro-pyran-2-yl)-1H-pyrazol-4-yl]-urée

A partir de 30 mg de l'**intermédiaire 2** et de cyclopropylamine selon la procédure générale. LC/MS analytique : Tr=2,46 min; [M+H]+ = 486,19 ; ELSD = 60 %; DAD = 56 %

### Etape C: 1-Cyclopropyl-3-{3-[6-(3-dimethylamino-propoxy)-5-fluoro-1H-benzimidazol-2-yl]-1H-pyrazol-4-yl}-urée

Le brut obtenu selon la méthode générale est purifié par LC/MS préparative avec le gradient standard. 13 mg de 1-cyclopropyl-3-{3-[6-(3-diméthylamino-propoxy)-5-fluoro-1H-benzimidazol-2-yl]-1H-pyrazol-4-yl}-urée sont obtenus sous forme d'une poudre brune. RMN ¹H (400 MHz, DMSO-d6, δ en ppm) : 0,32 (m, 2H) ; 0,53 (m, 2H) ; 0,78 (m, 1H) ; 2,18 (m, 2H) ; 2,85 (s, 6H) ; 3,27 (m, 2H) ; 4,17 (t, J= 6,0 Hz, 2H) ; 7,27 (d, J=7,5 Hz, 1 H) ; 7,48 (d, J=10,5 Hz, 1H) ; 8,05 (s, 1H).

### Exemple 23 N-{3-[6-(3-dimethylamino-propoxy)-5-fluoro-1H-benzimidazol-2-yl]-1H-pyrazol-4-yl}-pyrrolidine-1-carboxamide

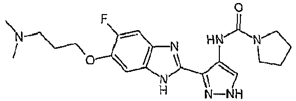

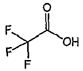

### Etape B : N-[3-[6-(3-diméthylamino-propoxy)-5-fluoro-1H-benzimidazol-2-yl]-1-(tétrahydro-pyran-2-yl)-1H-pyrazol-4-yl]-pyrrolidine-1-carboxamide

A partir de 30 mg de l'**intermédiaire 2** et de pyrolidine selon la procédure générale LC/MS analytique : Tr = 2,53 min; [M+H]⁺ = 500,58; ELSD = 66 %; DAD = 71 %

### Etape C: Préparation du N- {3-[6-(3-dimethylamino-propoxy)-5-fluoro-1H-benzimidazol-2-yl]-1H-pyrazol-4-yl}-Pyrrolidine-1-carboxamide

Le brut obtenu selon la méthode générale est purifié par LC/MS préparative avec le gradient standard. 22,5 mg de N-{3-[6-(3-dimethylamino-propoxy)-5-fluoro-1H-benzimidazol-2-yl]-1H-pyrazol-4-yl}-Pyrrolidine-1-carboxamide sont obtenus sous forme d'une poudre brune. RMN ¹H (400 MHz, DMSO-d6, δ en ppm) : 1,89 (m, 4H) ; 2,19 (m, 2H) ; 2,85 (s, 6H) ; 3,28 (m, 2H) ; 3,41 (m, 4H) ; 4,22 (t, J= 6,0 Hz, 2H) ; 7,39 (d, J=7,5 Hz, 1 H) ; 7,63 (d, J=10,5 Hz, 1 H) ; 8,00 (s, 1 H).

### Exemple 24 1-{3-[6-(3-Dimethylamino-propoxy)-5-fluoro-1H-benzimidazol-2-yl]-1H-pyrazol-4-yl}-3-isopropyl-urée

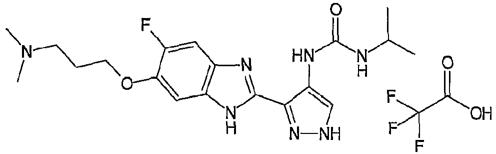

### Etape B : Préparation du 1-[3-[6-(3-Dimethylamino-propoxy)-5-fluoro-1H-benzimidazol-2-yl]-1-(tetrahydro-pyran-2-yl)-1 H-pyrazol-4-yl]-3-isopropyl-urée

A partir de 30 mg de l'**intermédiaire 2** et de diisopropylamine selon la méthode générale LC/MS analytique : Tr=2,53 min; [M+H]⁺=488,35; ELSD=55 %; DAD=41 %

### Etape C : Préparation du 1-{3-[6-(3-Dimethylamino-propoxy)-5-fluoro-1H-benzimidazol-2-yl]-1H-pyrazol-4-yl}-3-isopropyl-urée

Le brut obtenu selon la méthode générale est purifié par LC/MS préparative avec le gradient standard. 12,5 mg de 1-{3-[6-(3-diméthylamino-propoxy)-5-fluoro-1H-benzimidazol-2-yl]-1H-pyrazol-4-yl}-3-isopropyl-urée sont obtenus sous forme d'une poudre brune. RMN ¹H (400 MHz, DMSO-d6, δ en ppm) : 1,11 (d, J=6,5 Hz, 6H) ; 2,18 (m, 2H) ; 2,85 (s, 6H) ; 3,28 (m, 2H) ; 3,78 (m, 1H) ; 4,18 (t, J=6,0 Hz, 2H) ; 7,29 (d large, J=8,0 Hz, 1H) ; 7,48 (d large, J=10,5 Hz, 1H) ; 8,05 (s, 1H).

### Exemple 25 : N-{3-[6-(3-dimethylamino-propoxy)-5-fluoro-1H-benzimidazol-2-yl]-1H-pyrazol-4-yl}-Morpholine-4-carboxamide

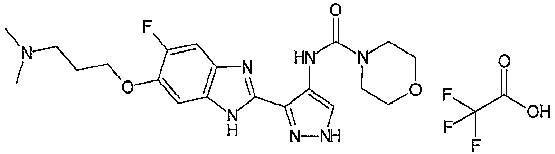

### Etape B : Préparation du N- [3-[6-(3-dimethylamino-propoxy)-5-fluoro-1H-benzimidazol-2-yl]-1-(tetrahydro-pyran-2-yl)-1H-pyrazol-4-yl]-Morpholine-4-carbox amide

A partir de 30 mg de l**'intermédiaire 2** et de morpholine selon la méthode générale LC/MS analytique : Tr = 2,50 min; [M+H]⁺ = 516,35; ELSD=90%; DAD=81%

### Etape C: Préparation du N- {3-[6-(3-dimethylamino-propoxy)-5-fluoro-1H-benzimidazol-2-yl]-1H-pyrazol-4-yl}- Morpholine-4-carboxamide

Le brut obtenu selon la méthode générale est purifié par LC/MS préparative avec le gradient standard. 25,1 mg de N- {3-[6-(3-diméthylamino-propoxy)-5-fluoro-1H-benzimidazol-2-yl]-1H-pyrazol-4-yl}-morpholine-4-carboxamide sont obtenus sous forme d'une poudre brune. RMN ¹H (400 MHz, DMSO-d6, δ en ppm) : 2,18 (m, 2H) ; 2,85 (s, 6H) ; 3,27 (m, 2H) ; 3,45 (m, 4H) ; 3,65 (m, 4H) ; 4,20 (t, J=6,0 Hz, 2H) ; 7,34 (d, J=8,0 Hz, 1 H) ; 7,62 (d, J=11,0 Hz, 1 H) ; 8,05 (s, 1 H).

### Exemple 26 3-{3-[6-(3-Dimethylamino-propoxy)-5-fluoro-1H-benzimidazol-2-yl]-1H-pyrazol-4-yl}-1-isopropyl-1-methyl-urée

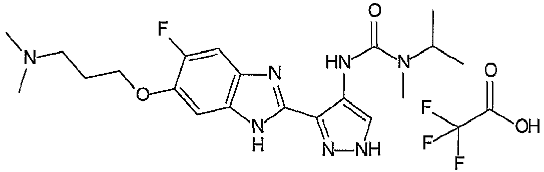

### Etape B: Préparation du 3-[3-[6-(3-Dimethylamino-propoxy)-5-fluoro-1 H-benzimidazol-2-yl]-1-(tétrahydro-pyran-2-yl)-1H-pyrazol-4-yl]-1-isopropyl-1-methyl-urée

A partir de 50 mg de l'**intermédiaire 2** et de N-methyl-N-isopropylamine selon la méthode générale. LC/MS analytique :Tr=2,70 min; [M+H]⁺ =502,26; ELSD=73%; DAD = 43%

### Etape C : Préparation du 3-{3-[6-(3-Dimethylamino-propoxy)-5-fluoro-1H-benzimidazol-2-yl]-1H-pyrazol-4-yl}-1-isopropyl-1-méthyl-urée

Le produit brut obtenu selon la méthode générale est purifié par LC/MS préparative avec le gradient standard. 34,3 mg de 3-{3-[6-(3-diméthylamino-propoxy)-5-fluoro-1H-benzimidazol-2-yl]-1H-pyrazol-4-yl}-1-isopropyl-1-methyl-urée sont obtenus sous forme d'une poudre brune. RMN ¹H (400 MHz, DMSO-d6, δ en ppm) : 1,11 (d, J=6,5 Hz, 6H) ; 2,18 (m, 2H); 2,85 (s, 6H) ; 2,87 (s, 3H); 3,27 (m, 2H) ; 4,20 (t, J=6,0 Hz, 2H) ; 4,45 (m, 1 H) ; 7,34 (d, J=8,0 Hz, 1 H) ; 7,57 (d, J=11,0 Hz, 1 H) ; 8,01 (s, 1 H).

### Exemple 27 1-tert-Butyl-3-{3-[6-(3-dimethylamino-propoxy)-5-fluoro-1H-benzimidazol-2-yl]-1H-pyrazol-4-yl}-urée

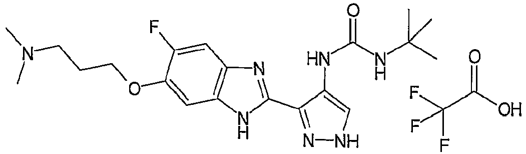

### Etape B : Préparation du 1-tert-Butyl-3-[3-[6-(3-dimethylamino-propoxy)-5-fluoro-1H-benzimidazol-2-yl]-1-(tetrahydro-pyran-2-yl)-1 H-pyrazol-4-yl]-urée

A partir de 50 mg de l**'intermédiaire 2** et de ter-butylamine selon la méthode générale. LC/MS analytique : Tr=2,85 min; [M+H]⁺ =502,52; ELSD=34 %; DAD= 31 %

### Etape C : 1-tert-Butyl-3-{3-[6-(3-dimethylamino-propoxy)-5-fluoro-1H-benzimidazol-2-yl]-1H-pyrazol-4-yl}-urée

Le produit brut obtenu selon la méthode générale est purifié par LC/MS préparative avec le gradient standard. 8,9 mg de 1-tert-Butyl-3-{3-[6-(3-dimethylamino-propoxy)-5-fluoro-1H-benzimidazol-2-yl]-1H-pyrazol-4-yl}-urea sont obtenus sous forme d'une poudre brune. RMN ¹H (400 MHz, DMSO-d6, δ en ppm) : 1,28 (s, 9H) ; 2,19 (m, 2H) ; 2,85 (s, 6H) ; 3,28 (m, 2H) ; 4,21 (t, J=6,0 Hz, 2H) ; 7,38 (d, J=7,0 Hz, 1 H) ; 7,63 (d, J=10,0 Hz, 1H) ; 8,10 (s, 1H).

### Exemple 28 1-{3-[6-(3-diméthylamino-propoxy)-5-fluoro-1H-benzimidazol-2-yl]-1H-pyrazol-4-yl}-3-isobutyl-urée

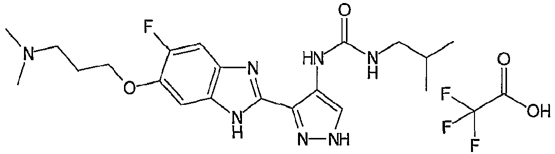

### Etape B : Préparation du 1-[3-[6-(3-dimethylamino-propoxy)-5-fluoro-1H-benzimidazol-2-yl]-1-(tétrahydro-pyran-2-yl)-1H-pyrazol-4-yl]-3-isobutyl-urée

A partir de 50 mg de l**'intermédiaire 2** et de s-butylamine selon la méthode générale LC/MS analytique : Tr=2,78 min; [M+H]⁺=502,25; ELSD = 58%; DAD = 34%.

### Etape C : Préparation du 1-{3-[6-(3-Dimethylamino-propoxy)-5-fluoro-1H-benzimidazol-2-yl]-1H-pyrazol-4-yl}-3-isobutyl-urée

Le produit brut obtenu selon la méthode générale est purifié par LC/MS préparative avec le gradient standard. 14,4 mg de 1-{3-[6-(3-dimethylamino-propoxy)-5-fluoro-1H-benzimidazol-2-yl]-1H-pyrazol-4-yl}-3-isobutyl-urée sont obtenus sous forme d'une poudre brune. RMN ¹H (400 MHz, DMSO-d6, δ en ppm) : 0,86 (d, J= 6,5 Hz, 6H) ; 1,68 (m, 1 H) ; 2,19 (m, 2H) ; 2,85 (s, 6H) ; 2,93 (d, J=7,0 Hz, 2H) ; 3,28 (m, 2H) ; 4,21 (t, J=6,5 Hz, 2H) ; 7,39 (d, J=8,0 Hz, 1H) ; 7,62 (d, J=10,5 Hz, 1H) ; 8,07 (s, 1H).

### Exemple 29 3-{3-[6-(3-dimethylamino-propoxy)-5-fluoro-1H-benzimidazol-2-yl]-1 H-pyrazol-4-yl}-1,1-diisopropyl-urée

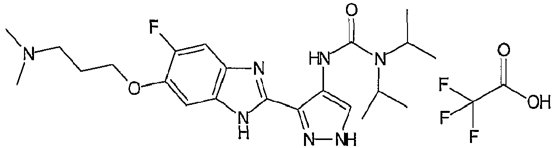

### Etape B : Préparation du 3-[3-[6-(3-diméthylamino-propoxy)-5-fluoro-1H-benzimidazol-2-yl]-1-(tetrahydro-pyran-2-yl)-1H-pyrazol-4-yl]-1,1-diisopropyl-urea

A partir de 50 mg de **l'intermédiaire 2** et de N,N-diisopropylamine LC/MS analytique : Tr = 3,18 min; [M+H]⁺ = 530,25 ; ELSD = 21 %; DAD = 25%

### Etape C : Préparation du 3-{3-[6-(3-Dimethylamino-propoxy)-5-fluoro-1H-benzimidazol-2-yl]-1H-pyrazol-4-yl}-1,1-diisopropyl-urée

Le produit brut obtenu selon la méthode générale est purifié par LC/MS préparative avec le gradient standard. 2,4 mg de 3-{3-[6-(3-diméthylamino-propoxy)-5-fluoro-1 H-benzimidazol-2-yl]-1H-pyrazol-4-yl}-1,1-diisopropyl-urée sont obtenus sous forme d'une poudre brune. LC/MS analytique : Tr=2,8 min; [M+H]⁺ = 446,42; ELSD = 100 %; DAD = 94 %

### Exemple 30 : 3-{3-[6-(3-diméthylamino-propoxy)-5-fluoro-1H-benzimidazol-2-yl]-1H-pyrazol-4-yl}-1,1-dipropyl-urée

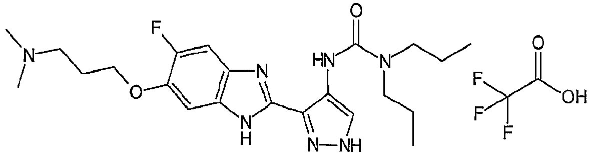

### Etape B : 3-[3-[6-(3-diméthylamino-propoxy)-5-fluoro-1H-benzimidazol-2-yl]-1-(tetrahydro-pyran-2-yl)-1H-pyrazol-4-yl]-1,1-dipropyl-urée

A partir de 50 mg de l'**intermédiaire 2** et N, N-dipropylamine selon la méthode générale. LC/MS analytique : Tr=3,04 min; [M+H]⁺=530,91; ELSD=92%; DAD = 77%

### Etape C : 3-{3-[6-(3-Dimethylamino-propoxy)-5-fluoro-1H-benzimidazol-2-yl]-1H-pyrazol-4-yl}-1,1-dipropyl-urée

Le produit brut obtenu selon la méthode générale est purifié par LC/MS préparative avec le gradient standard. 35,4 mg de 3-{3-[6-(3-Dimethylamino-propoxy)-5-fluoro-1H-benzimidazol-2-yl]-1H-pyrazol-4-yl}-1,1-dipropyl-urée sont obtenus sous forme d'une poudre brune. RMN ¹H (400 MHz, DMSO-d6, δ en ppm) : 0,95 (t, J= 7,0 Hz, 6H) ; 1,64 (m, 4H) ; 2,17 (m, 2H) ; 2,85 (s, 6H) ; 3,30 (m, 6H) ; 4,16 (t, J=6,5 Hz, 2H) ; 7,22 (d, J=7,5 Hz, 1 H) ; 7,39 (d, J=11,0 Hz, 1 H) ; 8,02 (s, 1 H).

### Exemple 31 N-{3-[6-(3-dimethylamino-propoxy)-5-fluoro-1H-benzimidazol-2-yl]-1H-pyrazol-4-yl}-Piperazine-1-carboxamide

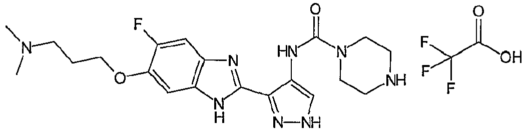

### Etape B : Tert-butyl ester de l'acide 4-[3-[6-(3-Dimethylamino-propoxy)-5-fluoro-1H-benzimidazol-2-yl]-1-(tetrahydro-pyran-2-yl)-1H-pyrazol-4-ylcarbamoyl]-piperazine-1-carboxylique

A une solution de 50 mg d'**intermédiaire 2** dans 1 mL de DMF, 10 éq de 1-Boc-piperazine sont additionnés. Le mélange réactionnel est irradié pendant 40 min à 200°C au micro-onde sur PERSONAL SYNTHETISER. Le milieu réactionnel est concentré sous vide à l'évaporateur rotatif. Le concentrat est utilisé immédiatement dans l'étape suivante. LC/MS analytique : Tr = 3,11 min; [M+H]⁺ = 615,41; ELSD = 18%; DAD = 18%

### Etape C : N-{3-[6-(3-dimethylamino-propoxy)-5-fluoro-1H-benzimidazol-2-yl]-1H-pyrazol-4-yl}-Piperazine-1-carboxamide

Le produit brut obtenu selon la méthode générale est purifié par LC/MS préparative avec le gradient standard. 28,2 mg de N-{3-[6-(3-dimethylamino-propoxy)-5-fluoro-1H-benzimidazol-2-yl]-1H-pyrazol-4-yl}-pipérazine-1-carboxamide sont obtenus sous forme d'une poudre brune. RMN ¹H (400 MHz, DMSO-d6, δ en ppm) : 2,18 (m, 2H) ; 2,85 (s, 6H) ; de 3,17 à 3,33 (m, 6H) ; 3,70 (m, 4H) ; 4,18 (t, J=6,0 Hz, 2H) ; 7,31 (d, J=7,5 Hz, 1H) : 7,61 (d, J=10,5 Hz, 1 H) ; 8,04 (s, 1H).

### Exemple 32 : 1-{3-[6-(3-diméthylamino-propoxy)-5-fluoro-1H-benzimidazol-2-yl]-1H-pyrazol-4-yl}-3-éthyl-urée

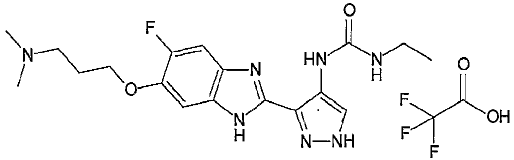

### Etape B : 1-[3-[6-(3-Dimethylamino-propoxy)-5-fluoro-1H-benzimidazol-2-yl]-1-(tetrahydro-pyran-2-yl)-1H-pyrazol-4-yl]-3-ethyl-urée

A 50 mg d'**intermédiaire 2,** 50 éq d'éthylamine dans le THF à 2N sont additionnés. Le mélange réactionnel est irradié pendant 40 min à 160°C au micro-onde sur Personal Synthetiser. On rajoute 10 éq. d'éthylamine. Le mélange réactionnel est irradié pendant 20 min à 80°C au micro-onde. Le milieu réactionnel est concentré sous vide à l'évaporateur rotatif. On rajoute 50 éq d'éthylamine et 0,5 mL de DMF. Le mélange réactionnel est irradié pendant 10 min à 160°C, puis 20 min à 160°C, puis 60 min à 160°C, puis 60 min à 160°C au micro-onde. Le milieu réactionnel est concentré sous vide à l'évaporateur rotatif. On rajoute 50 éq d'éthylamine et 0,5 mL de DMF. Le mélange réactionnel est irradié pendant 60 min à 160°C. Le milieu réactionnel est concentré sous vide à l'évaporateur rotatif. Le concentrat est utilisé immédiatement dans l'étape suivante. LC/MS analytique :Tr=2,34 min; [M+H]⁺ = 474,34 ; ELSD = 31%; DAD = 22%.

### Etape C : Préparation du 1-{3-[6-(3-diméthylamino-propoxy)-5-fluoro-1H-benzimidazol-2-yl]-1H-pyrazol-4-yl}-3-éthyl-urée

Le produit brut obtenu selon la méthode générale est purifié par LC/MS préparative avec le gradient standard. 14,2 mg de 1-{3-[6-(3-diméthylamino-propoxy)-5-fluoro-1H-benzimidazol-2-yl]-1H-pyrazol-4-yl}-3-ethyl-urée sont obtenus sous forme d'une poudre brune. RMN ¹H (400 MHz, DMSO-d6, δ en ppm) : 1,07 (t, J=7,5 Hz, 3H) ; 2,19 (m, 2H) ; 2,85 (s, 6H) ; 3,13 (q, J=7,5 Hz, 2H) ; 3,28 (m, 2H) ; 4,20 (t, J=6,0 Hz, 2H) ; 7,35 (d, J = 7,5 Hz, 1 H) ; 7,59 (d, J=10,5 Hz, 1 H) ; 8,05 (s, 1H).

### Exemple 33 : 3-{3-[6-(3-Dimethylamino-propoxy)-5-fluoro-1H-benzimidazol-2-yl]-1H-pyrazol-4-yl}-1-ethyl-1-isopropyl-urée

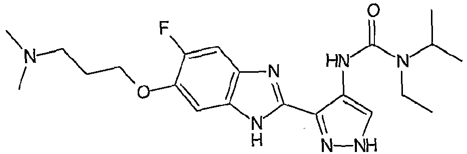

### Etape B : 3-[3-[6-(3-Dimethylamino-propoxy)-5-fluoro-1H-benzimidazol-2-yl]-1-(tetrahydro-pyran-2-yl)-1 H-pyrazol-4-yl]-1-ethyl-1-isopropyl-urée

A partir de 47 mg de l'**intermédiaire 2** N-éthyl-N-isopropylamine selon la méthode générale. LC/MS analytique : Tr=3,08 min; [M+H]⁺ = 516,30; ELSD = 82%; DAD = 67%

### Etape C : Préparation du 3-{3-[6-(3-diméthylamino-propoxy)-5-fluoro-1 H-benzimidazol-2-yl]-1H-pyrazol-4-yl}-1-éthyl-1-isopropyl-urée

Le produit brut obtenu selon la méthode générale est purifié par LC/MS préparative avec le gradient standard. 15,7 mg de 3-{3-[6-(3-Dimethylamino-propoxy)-5-fluoro-1H-benzimidazol-2-yl]-1H-pyrazol-4-yl}-1-ethyl-1-isopropyl-urée sont obtenus sous forme d'une poudre brune. RMN ¹H (400 MHz, DMSO-d6, δ en ppm) : 1,17 (d, J=7,0 Hz, 6H) ; 1,28 (t, J=7,5 Hz, 3H) ; 2,17 (m, 2H) ; 2,85 (s, 6H) ; 3,27 (m, 2H) ; 3,33 (q, J=7,5 Hz, 2H) ; 4,18 (t, J=6,0 Hz, 2H) ; 4,41 (m, 1H) ; 7,26 (d, J=7,5 Hz, 1H) ; 7,47 (d, J=10,5 Hz, 1 H) ; 8,01 (s, 1H).

### Exemple de référence 34: 1,1-Diisopropyl-3-{3-[6-(4-methyl-piperazin-1-yl)-1H-benzimidazol-2-yl]-1H-pyrazol-4-yl}-urée

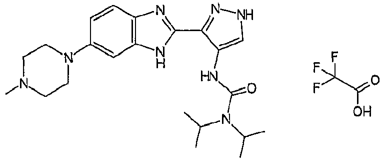

### Etape III: 1,1-Diisopropyl-3-[3-[6-(4-methyl-piperazin-1-yl)-1H-benzimidazol-2-yl]-1-(tetrahydro-pyran-2-yl)-1H-pyrazol-4-yl]-urée

A une solution de 200 mg d'**intermédiaire 3** dans 22 mL de THF, 5 éq de chlorure de diisopropylcarbamoyle et 1 éq de N,N-diisopropyléthylamine sont additionnés. Le mélange réactionnel est chauffé à 52°C pendant 4 heures. Le milieu réactionnel est refroidi à température ambiante et on ajoute 20 mL d'une solution saturée de bicarbonate de sodium. La phase aqueuse est extraite avec 3 fois 20 mL d'AcOEt. Les phases organiques sont séchées sur MgSO₄, filtrées et concentrées sous vide à l'évaporateur rotatif. 230 mg de 1,1-Diisopropyl-3-[3-[6-(4-methyl-piperazin-1-yl)-1H-benzimidazol-2-yl]-1-(tetrahydro-pyran-2-yl)-1H-pyrazol-4-yl]-urée. LC/MS analytique : Tr = 3,04 min; [M+H]⁺ = 509,32; ELSD = 82 %; DAD = 100 %

### Etape IV : Préparation du 1,1-diisopropyl-3-{3-[6-(4-methyl-pipérazin-1-yl)-1H-benzimidazol-2-yl]-1H-pyrazol-4-yl}-urée

Une solution de 230 mg de 1,1-Diisopropyl-3-[3-[6-(4-methyl-piperazin-1-yl)-1H-benzimidazol-2-yl]-1-(tetrahydro-pyran-2-yl)-1H-pyrazol-4-yl]-urée en solution dans 40 éq d'une solution d'acide chlorhydrique 4N dans le dioxanne est agitée à 22 °C pendant environ 4 heures, il y a précipitation. La suspension est filtrée sur verre fritté et le solide est rincé au dioxanne et à l'éther isopropylique. Le précipité est séché à l'étuve. Le précipité est dissous dans 5 mL de NaOH à 2 N, puis on extrait avec 2 fois 5 mL d'acétate d'éthyle puis 2 fois 10 mL d'acétate d'éthyle. Les phases organiques sont séchées sur MgSO₄, filtrées et concentrées sous vide à l'évaporateur rotatif. Le concentrat est purifié par HPLC préparative avec pour éluant un gradient eau, acétonitrile contenant respectivement 0,07 % d'acide trifluoroacétique. 99 mg de 1,1-Diisopropyl-3-{3-[6-(4-methyl-piperazin-1-yl)-1H-benzimidazol-2-yl]-1H-pyrazol-4-yl}-urée sont isolés sous forme d'une poudre jaune pâle. RMN ¹H (400 MHz, DMSO-d6, δ en ppm) : 1,25 (d, J=7,0 Hz, 12H) ; 2,90 (s, 3H) ; 3,03 (m, 2H) ; 3,23 (m, 2H) ; 3,58 (m, 2H) ; 3,87 (m, 4H) ; 7,12 (d, J = 2,0 Hz, 1H) ; 7,24 (dd, J=2,0 et 9,0 Hz, 1H) ; 7,58 (d, J = 9,0 Hz, 1H) ; 7,98 (s, 1 H).

### Exemple de référence 35: 3-{3-[6-(4-Methyl-piperazin-1-yl)-1H-benzimidazol-2-yl]-1H-pyrazol-4-yl}-1,1-dipropyl-urée

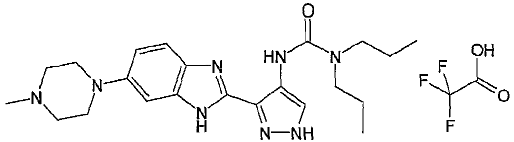

### Etape III: Ethyl ester de l'acide [3-[6-(4-Methyl-piperazin-1-yl)-1H-benzimidazol-2-yl]-1-(tetrahydro-pyran-2-yl)-1H-pyrazol-4-yl]-carbamique

A une solution de 200 mg **d'intermédiaire 3** dans 3,6 mL de dichlorométhane, 50 µL chloroformate d'éthyle et 73 µL de triéthylamine sont additionnés. Le mélange réactionnel est agité à 22°C pendant 72 heures. Le milieu réactionnel est lavé avec 4 mL d'eau. La phase aqueuse est extraite avec 3 fois 4 mL de dichlorométhane. Les phases organiques sont séchées sur MgSO₄ filtrées et concentrées sous vide à l'évaporateur rotatif. 168 mg d'ester éthylique de l'acide [[3-[6-(4-Methyl-piperazin-1-yl)-1H-benzimidazol-2-yl]-1-(tetrahydro-pyran-2-yl)-1H-pyrazol-4-yl]-carbamique sont obtenus sous forme d'un solide jaune pâle. RMN ¹H (400 MHz, DMSO-d6, δ en ppm) : 1,24 (m, 3H) ; 1,60 à 2,21 (m, 6H) ; 2,90 (s, 3H) ; 3,03 (t, 2H) ; 3,23 (t, 2H) ; 3,58 (d, 2H) ; 3,70 (m, 1 H) ; 3,89 (d, 2H) ; 3,98 (m, 1H) ; 4,15 (q, 2H) ; 5,55 (m, 1H) ; 7,16 (d, J=2,0 Hz, 1H) ; 7,28 (dd, J=2,0 et 9,0 Hz, 1H) ; 7,64 (d, J=9,0 Hz, 1 H) ; 8,21 (s, 1H). LC/MS analytique : Tr=2,38 min; [M+H]⁺ = 454,32; ELSD=100 %; DAD=91 %

### Etape IV : 3-[3-[6-(4-Methyl-piperazin-1-yl)-1H-benzimidazol-2-yl]-1-(tetrahydro-pyran-2-yl)-1H-pyrazol-4-yl]-1,1-dipropyl-urée

A une solution de 118 mg du [3-[6-(4-Methyl-piperazin-1-yl)-1H-benzimidazol-2-yl]-1-(tetrahydro-pyran-2-yl)-1H-pyrazol-4-yl]-carbamic acid ethyl ester dans 4,1 mL de DMF, 50 éq de dipropylamine sont additionnés. Le mélange réactionnel est irradié pendant 40 min à 200°C aux micro-ondes sur Persona Synthetiser. Le milieu réactionnel est concentré sous vide à l'évaporateur rotatif. Le concentrat est utilisé immédiatement dans l'étape suivante. LC/MS analytique : Tr = 3,23 min; [M+H]⁺= 509,92; ELSD = 91 %; DAD = 70 %

### Etape V : 3-{3-[6-(4-Methyl-piperazin-1-yl)-1H-benzimidazol-2-yl]-1H-pyrazol-4-yl}-1,1-dipropyl-urée

Une solution de 190 mg de 3-[3-[6-(4-Methyl-piperazin-1-yl)-1H-benzimidazol-2-yl]-1-(tetrahydro-pyran-2-yl)-1H-pyrazol-4-yl]-1,1-dipropyl-urée en solution dans 3,5 mL d'une solution d'acide chlorhydrique 4N dans le dioxanne est agitée à 22 °C pendant 4 heures. Un solide précipite. Le solide est filtré, lavé au dioxanne et à l'éther isopropylique. Le solide devient pâteux. On regroupe filtrat et précipité avec du méthanol et on concentre. On dissout le brut réactionnel dans une solution aqueuse de NaOH 2N, il y a formation d'une pâte collante qui ne se dissout pas. On récupère avec du méthanol et on concentre. Le concentrat est dissout avec 10 mL d'eau et on extrait par 2 fois 10 mL et 2 fois 15 mL d'acétate d'éthyle. Les phases organiques sont séchées sur MgSO₄ filtrées et concentrées sous vide à l'évaporateur rotatif. Le précipité obtenu est purifié par HPLC préparative avec pour éluant un gradient eau, acétonitrile contenant respectivement 0,07% d'acide trifluoroacétique. 65 mg de 3-{3-[6-(4-Methyl-piperazin-1-yl)-1H-benzimidazol-2-yl]-1H-pyrazol-4-yl}-1,1-dipropyl-urée sont obtenus sous forme d'un solide jaune. RMN ¹H (400 MHz, DMSO-d6, δ en ppm) : 0,89 (t, J=7,5 Hz, 6H) ; 1,57 (m, 4H) ; 2,89 (s, 3H) ; 3,03 (m, 2H) ; 3,23 (m partiellement masqué, 2H) ; 3,29 (m, 4H) ; 3,57 (m, 2H) ; 3,87 (m, 2H) ; 7,13 (d, J=2,0 Hz, 1 H) ; 7,24 (d large, J=9,0 Hz, 1H) ; 7,59 (d, J=9,0 Hz, 1 H) ; 7,98 (s, 1 H).

### Exemple 36 : N-{3-[6-(3-dimethylamino-propoxy)-5-fluoro-1H-benzimidazol-2-yl]-1H-pyrazol-4-yl}-4-méthyl-piperazine-1-carboxamide

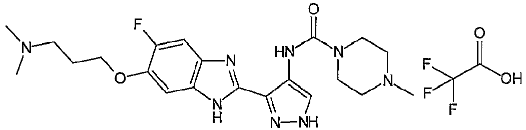

### Etape B : N- [3-[6-(3-dimethylamino-propoxy)-5-fluoro-1H-benzimidazol-2-yl]-1-(tetrahydro-pyran-2-yl)-1 H-pyrazol-4-yl]-4-Methyl-piperazine-1-carboxamide

A partir de 47 mg de l'**intermédiaire 2** et de N-méthylpipérazine selon la méthode générale. LC/MS analytique : Tr=1,92 min; [M+H]⁺ =528,99; ELSD=93%; DAD=81 %

### Etape C : Préparation du N- {3-[6-(3-dimethylamino-propoxy)-5-fluoro-1H-benzimidazol-2-yl]-1H-pyrazol-4-yl}-4-Methyl-pipérazine-1-carboxamide

Le produit brut obtenu selon la méthode générale obtenu est purifié par HPLC préparative avec pour éluant un gradient eau, acétonitrile contenant respectivement 0,07 % d'acide trifluoroacétique. 16,9 mg de N-{3-[6-(3-dimethylamino-propoxy)-5-fluoro-1H-benzimidazol-2-yl]-1H-pyrazol-4-yl}-4-Methyl-piperazine-1-carboxamide sont obtenus sous forme d'une poudre brune. RMN ¹H (400 MHz, DMSO-d6, δ en ppm) : 2,19 (m, 2H) ; 2,85 (s, 6H) ; 2,87 (s, 3H) ; 3,06 (m, 2H) ; 3,27 (m, 4H) ; 3,52 (m, 2H) ; 4,20 (m, 4H) ; 7,35 (d, J = 7,5 Hz, 1H) ; 7,63 (d, J = 10,5 Hz, 1 H) ; 8,04 (s, 1H).

### Exemple de référence 37: 1-Ethyl-1-isopropyl-3-{3-[6-(4-methyl-piperazin-1-yl)-1H-benzimidazol-2-yl]-1H-pyrazol-4-yl}-urée

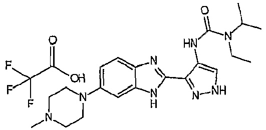

### Etape III : 1-Ethyl-1-isopropyl-3-[3-[6-(4-methyl-piperazin-1-yl)-1H-benzimidazol-2-yl]-1-(tetrahydro-pyran-2-yl)-1H-pyrazol-4-yl]-urée

A une solution de 16 mg d'imidazole et de 192 mg de N,N'-carbonyldiimidazole dans 4,5 mL d'acétonitrile refroidi à 0°C on additionne une solution de 300 mg d'**intermédiaire 3** en solution dans 1 mL d'acétonitrile et 2 mL de DMF. Le mélange réactionnel est agité à 22°C pendant 2 heures. On rajoute 685 µL de N-ethylisopropylamine. Le mélange réactionnel est irradié pendant 45 min à 150°C au micro-onde sur Personal Synthetiser. Le milieu réactionnel est concentré sous vide à l'évaporateur rotatif. Le concentrat est utilisé immédiatement dans l'étape suivante. LC/MS analytique : Tr = 2,9 min; [M+H]⁺ = 495,29; ELSD = 90%; UV254nm = 58%

### Etape IV : Préparation du 1-Ethyl-1-isopropyl-3-{3-[6-(4-methyl-piperazin-1-yl)-1H-benzimidazol-2-yl]-1H-pyrazol-4-yl}-urée

Une solution du concentrat en solution dans 40 éq d'une solution d'acide chlorhydrique 4N dans le dioxanne est agitée à 22 °C pendant environ 4 heures (jusqu'à disparition du produit de départ). Le milieu réactionnel est concentré sous vide à l'évaporateur rotatif. Le concentrat est purifié par LC/MS préparative avec pour éluant un gradient eau, acétonitrile contenant respectivement 0,07 % d'acide trifluoroacétique. 160 mg de 1-Ethyl-1-isopropyl-3-{3-[6-(4-methyl-piperazin-1-yl)-1H-benzimidazol-2-yl]-1H-pyrazol-4-yl}-urée sont obtenus sous forme d'une poudre brune. RMN ¹H (300 MHz, DMSO-d6, δ en ppm) : 1,13 (d, J=6,5 Hz, 6H) ; 1,16 (t partiellement masqué, J=7,0 Hz, 3H) ; 2,90 (s, 3H) ; 3,04 (m, 2H) ; 3,24 (m, 2H) ; 3,28 (q partiellement masqué, J=7,0 Hz, 2H) ; 3,57 (m, 2H) ; 3,88 (m, 2H) ; 4,34 (m, 1H) ; 7,16 (d, J=2,0 Hz, 1H) ; 7,27 (dd, J=2,0 et 9,0 Hz, 1H) ; 7,62 (d, J= 9,0 Hz, 1H) ; 8,00 (s, 1H).

### Exemple 38: N-[3-(5-fluoro-6-(1,4-oxazepan-4-yl-1H-benzimidazol-2-yl)-1H-pyrazol-4-yl]-Piperidine-1-carboxamide

### Etape V : Préparation du N-[3-(5-fluoro-6-perhydro-1,4-oxazepin-4-yl-1H-benzimidazol-2-yl)-1-(tetrahydro-pyran-2-yl)-1H-pyrazol-4-yl]-piperidine-1-carboxamide

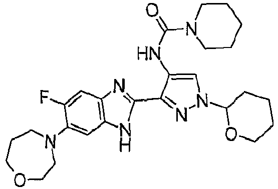

A une solution de 367 mg d**'intermédiaire 6** dans 5 mL de THF, 570 µL de chlorure de 1-piperidinecarbonyle et 800 µL de N,N-diisopropyléthylamine sont additionnés. Le mélange réactionnel est chauffé à 80°C pendant 5 heures. Le milieu réactionnel est refroidi à température ambiante et concentré sous vide à l'évaporateur rotatif. Le brut réactionnel est purifié par chromatographie flash en éluant avec un mélange dichlorométhane/méthanol. Le mélange contenant le produit attendu est concentré puis repurifié par chromatographie flash en éluant avec un mélange cyclohexane/ acétate d'éthyle. 80 mg de N-[3-(5-fluoro-6-perhydro-1,4-oxazepin-4-yl-1H-benzimidazol-2-yl)-1-(tetrahydro-pyran-2-yl)-1H-pyrazol-4-yl]-piperidine-1-carboxamide sont obtenus sous forme d'une mousse beige. RMN ¹H (400 MHz, DMSO-d6, δ en ppm) : de 1,30 à 2,24 (m, 14H) ; 3,43 (m, 4H) ; 3,51 (m, 4H) ; 3,66 (m, 1 H) ; 3,76 (m, 2H) ; 3,82 (m, 2H) ; 3,96 (m, 1H) ; 5,51 (d large, J=10,0 Hz, 1H) ; 7,34 (d, J=7,5 Hz, 1H) ; 7,55 (d, J=12,0 Hz, 1 H) ; 8,15 (s, 1H).

### Etape VI : N- [3-(5-fluoro-6-perhydro-1,4-oxazepin-4-yl-1H-benzimidazol-2-yl)-1H-pyrazol-4-yl]-Piperidine-1-carboxamide

A une solution de 80 mg de N-[3-(5-fluoro-6-perhydro-1,4-oxazepin-4-yl-1 H-benzimidazol-2-yl)-1-(tetrahydro-pyran-2-yl)-1 H-pyrazol-4-yl]-piperidine-1-carboxamide en solution dans 4 mL d'une solution d'acide chlorhydrique 4N dans le dioxanne est agitée à 22 °C pendant 1 heures. Le milieu réactionnel est concentré sous vide à l'évaporateur rotatif, puis remis en réaction à 22 °C pendant 16 heures avec 4 mL d'une solution d'acide chlorhydrique 4N dans le dioxanne est agitée à 22 °C. Le milieu réactionnel est concentré sous vide à l'évaporateur rotatif. Le produit est empâté dans l'éther isopropylique mais le produit est hydroscopique. Le brut réactionnel est purifié par chromatographie flash en éluant avec un mélange cyclohexane/acétate d'éthyle puis dichlorométhane/méthanol. Le produit est dilué dans de l'acétate d'éthyle, lavé avec 2 fois 5 mL d'une solution de soude aqueuse à 2N. Les phases aqueuses sont extraites avec 3 fois 10 mL de d'acétate d'éthyle. Les phases organiques sont séchées sur MgSO₄, filtrées et concentrées sous vide à l'évaporateur rotatif. 65 mg de N-[3-(5-fluoro-6-perhydro-1,4-oxazepin-4-yl-1 H-benzimidazol-2-yl)-1H-pyrazol-4-yl]-Piperidine-1-carbox amide sont obtenus sous forme d'un solide gris

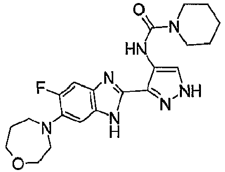

RMN ¹H (400 MHz, DMSO-d6, δ en ppm) : 1,58 (m, 6H) ; 1,99 (m, 2H) ; 3,30 (m partiellement masqué, 4H) ; 3,49 (m, 4H) ; 3,78 (m, 4H) ; de 7,03 à 7,40 (m étalé, 2H) ; 7,99 (s, 1 H) ; 9,93 (s, 1 H) ; 12,9 (m étalé, 2H).

### Exemple 39: Trifluoroacétate de 3-{3-[5-Fluoro-6-(4-methyl-piperazin-1-yl)-1H-benzimidazol-2-yl]-1H-pyrazol-4-yl}-1,1-diisopropyl-urée

### Etape V : 3-[3-[5-Fluoro-6-(4-methyl-piperazin-1-yl)-1H-banzimidazol-2-yl]-1-(tetrahydro-pyran-2-yl)-1H-pyrazol-4-yl]-1,1-diisopropyl-urée

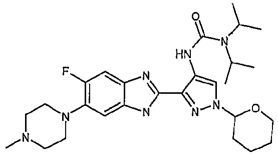

A une solution de 200 mg d'**intermédiaire 5** dans 21,2 mL de THF, 82 mg de chlorure de diisoproylcarbamoyle et 436 µL de N,N-diisopropyléthylamine sont additionnés. Le mélange réactionnel est chauffé à 66°C pendant 1,7 heures, puis agité pendant 16 heures à température ambiante, puis chauffé à 66°C pendant 7,75 heures, puis agité pendant 16 heures à température ambiante, puis chauffé à 66°C pendant 8 heures, puis agité pendant 16 heures. Le milieu réactionnel est lavé avec 20 mL d'eau de solution saturée en NaHCO₃. La phase aqueuse est extraite avec 3 fois 20 mL d'AcOEt. Les phases organiques sont séchées sur MgSO₄, filtrées et concentrées sous vide à l'évaporateur rotatif. 364 mg de 3-[3-[5-Fluoro-6-(4-methyl-piperazin-1-yl)-1H-benzimidazol-2-yl]-1-(tetrahydro-pyran-2-yl)-1H-pyrazol-4-yl]-1,1-diisopropyl-urée sont obtenus sous forme d'un solide orange. RMN ¹H (400 MHz, DMSO-d6, δ en ppm) : 1,32 (d, J=7,0 Hz, 12H); de 1,51 à 2,20 (m, 6H) ; 2,89 (s, 3H) ; 3,05 (m, 2H) ; 3,28 (m, 2H) ; 3,53 (m, 4H); 3,66 (m, 1H) ; 3,96 (m, 1H) ; 4,02 (m, 2H); 5,46 (dd, J=2,0 et 10,0 Hz, 1H) ; 7,16 (d, J=8,0 Hz, 1H) ; 7,40 (d, J= 11,5 Hz, 1H) ; 8,16 (s, 1H). Spectre de masse en ES : m/z=527 : MH+ (pic de base) - m/z=426 : (M + H - C6H15N)+ - m/z=342 : (m/z=426 - C5H8O)+

### Etape VI : 3-{3-[5-Fluoro-6-(4-methyl-piperazin-1-yl)-1H-benzimidazol-2-yl]-1H-pyrazol-4-yl}-1,1-diisopropyl-urée sous forme d'un sel de trifluoroacétate

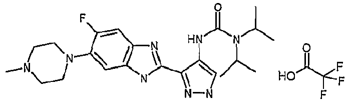

Une solution de 364 mg de 3-[3-[5-Fluoro-6-(4-methyl-piperazin-1-yl)-1H-benzimidazol-2-yl]-1-(tetrahydro-pyran-2-yl)-1H-pyrazol-4-yl]-1,1-diisopropyl-urée en solution dans 6,9 mL d'une solution d'acide chlorhydrique 4N dans le dioxanne est agitée à 22 °C pendant 4 heures. Après évaporation, le brut réactionnel est purifié par HPLC préparative avec pour éluant un gradient eau, acétonitrile contenant respectivement 0,07 % d'acide trifluoroacétique. 123 mg de 3-{3-[5-Fluoro-6-(4-methyl-piperazin-1-yl)-1H-benzimidazol-2-yl]-1H-pyrazol-4-yl}-1,1-diisopropyl-urée sous forme d'un sel de trifluoroacétate sont obtenus sous forme d'un solide rose. RMN ¹H (400 MHz, DMSO-d6, δ en ppm) : 1,32 (d, J=7,0 Hz, 12H) ; 2,91 (s, 3H) ; 3,04 (m, 2H) ; 3,28 (m, 2H) ; 3,53 (m, 4H) ; 4,01 (m, 2H) ; 7,18 (d, J=8,0 Hz, 1H) ; 7,41 (d, J=11,5 Hz, 1H) ; 8,03 (s, 1H). ES m/z=443 :MH+ (pic de base) - m/z=342 : (M + H - C6H15N)+

### Exemple 40: 3-{3-[5-fluoro-6-(4-methyl-perhydro-1,4-diazepin-1-yl)-1H-benzimidazol-2-yl]-1H-pyrazol-4-yl}-1,1-diisopropyl-urée

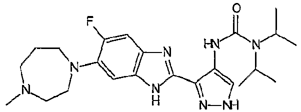

### Etape V: 3-[3-[5-Fluoro-6-(4-methyl-perhydro-1,4-diazepin-1-yl)-1H-benzimidazol-2-yl]-1-(tetrahydro-pyran-2-yl)-1H-pyrazol-4-yl]-1,1-diisopropyl-urée

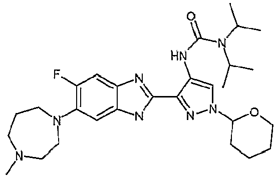

A une solution de 177 mg d'**intermédiaire 4** dans 20 mL de THF, 70 mg de chlorure de diisoproylcarbamoyle et 373 µL de N,N-diisopropyléthylamine sont additionnés. Le mélange réactionnel est chauffé à 66°C pendant 3 heures, puis agité pendant 65 heures à température ambiante, puis chauffé à 66°C pendant 7,75 heures, puis agité pendant 16 heures à température ambiante, puis chauffé à 66°C pendant 7,75 heures, puis agité pendant 16 heures à température ambiante, puis chauffé à 66°C pendant 3,5 heures, puis agité pendant 16 heures à température ambiante. On ajoute 20 mL d'une solution saturée en NaHCO₃. La phase aqueuse est extraite avec 3 fois 20 mL d'AcOEt. Les phases organiques sont séchées sur MgSO₄, filtrées et concentrées sous vide à l'évaporateur rotatif. 159 mg de 3-[3-[5-Fluoro-6-(4-methyl-perhydro-1,4-diazepin-1-yl)-1H-benzimidazol-2-yl]-1-(tetrahydro-pyran-2-yl)-1H-pyrazol-4-yl]-1,1-diisopropyl-urée sont obtenus sous forme d'un solide orange. LC/MS analytique : Tr=3,24 min; [M+H]⁺ = 541,67; ELSD = 87%; DAD = 37

### Etape VI: 3-{3-[5-Fluoro-6-(4-methyl-perhydro-1,4-diazepin-1-yl)-1H-benzimidazol-2-yl]-1H-pyrazol-4-yl}-1,1-diisopropyl-urée

Une solution de 278,5 mg de 3-[3-[5-Fluoro-6-(4-methyl-perhydro-1,4-diazepin-1-yl)-1H-benzimidazol-2-yl]-1-(tetrahydro-pyran-2-yl)-1H-pyrazol-4-yl]-1,1-diisopropyl-urée en solution dans 5,1 mL d'une solution d'acide chlorhydrique 4N dans le dioxanne est agitée à 22°C pendant 4 heures. Après évaporation, le brut réactionnel est purifié par HPLC préparative avec pour éluant un gradient eau, acétonitrile contenant respectivement 0,07 % d'acide trifluoroacétique. 15.2 mg de 3-{3-[5-Fluoro-6-(4-methyl-perhydro-1,4-diazepin-1-yl)-1H-benzimidazol-2-yl]-1H-pyrazol-4-yl}-1,1-diisopropyl-urée sont isolés sous forme d'un sel de trifluoroacétate. RMN ¹H (300 MHz, DMSO-d6, δ en ppm) : 1,26 (d, J=7,0 Hz, 12H) ; 2,21 (m large, 2H) ; 2,90 (s, 3H) ; de 3,14 à 3,68 (m, 8H) ; 3,90 (m, 2H) ; 7,16 (d, J=8,0 Hz, 1H) ; 7,48 (d, J=12,0 Hz, 1H) ; 7,98 (s, 1H). Spectre de masse en ES : m/z=457 : MH+ (pic de base) - m/z=330 (MH - C₇H₁₃NO)+.

Dans le tableau 1 les exemples 14, 19-20, 34,35 et 37 sont des exemples de référence.

**Tableau I (composés de formule (I) avec R₁=R₄=H)**

| **Ex** | **R₂** | **R₃** | **R₅** |
|---|---|---|---|
| **1** | F | 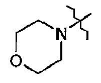 | 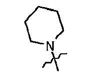 |
| **2** | F | 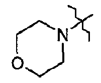 | -NEt₂ |
| **3** | F | 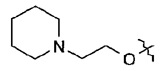 | 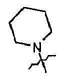 |
| **4** | F | 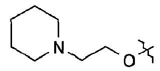 | -NEt₂ |
| **5** | F | Me₂N(CH₂)₃O- | -NEt₂ |
| **6** | F | 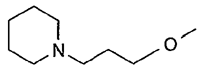 | -NEt₂ |
| **7** | F | Me₂N(CH₂)₃O- | 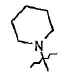 |
| **8** | F | 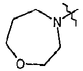 | -N(^{I}Pr)₂ |
| **9** | 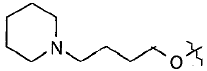 | F | -NEt₂ |
| **10** | 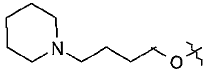 | F | 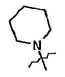 |
| **11** | F | 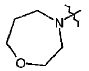 | -NEt₂ |
| **12** | F | 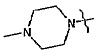 | 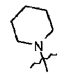 |
| **13** | F | 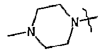 | -NEt₂ |
| **14** | F | (Et₂N-CO)(Me₂NCH₂CH₂)N- | -NEt₂ |
| **15** | F | NMe₂CH₂CH₂NH- | -NEt₂ |
| **16** | F | 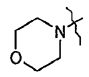 | -N(^{I}Pr)₂ |
| **17** | F | 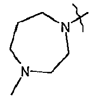 | 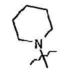 |
| **18** | F | 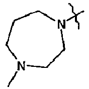 | -NEt₂ |
| **19** | H | 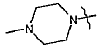 | -NEt₂ |
| **20** | H | 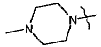 | 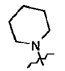 |
| **21** | F | 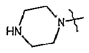 | 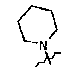 |
| **22** | F | NMe₂(CH₂)₃O- | -NHCy |
| **23** | F | NMe₂(CH₂)₃O- | 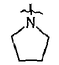 |
| **24** | F | NMe₂(CH₂)₃O- | -NH(^{I}Pr) |
| **25** | F | NMe₂(CH₂)₃O- | 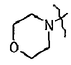 |
| **26** | F | NMe₂(CH₂)₃O- | -NMe(^{I}Pr) |
| **27** | F | NMe₂(CH₂)₃O- | -NH(ⁱBu) |
| **28** | F | NMe₂(CH₂)₃O- | -NH(ⁱBu) |
| **29** | F | NMe₂(CH₂)₃O- | -N(ⁱPr)₂ |
| **30** | F | NMe₂(CH₂)₃O- | -N(ⁿPr)₂ |
| **31** | F | NMe₂(CH₂)₃O- | 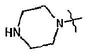 |
| **32** | F | NMe₂(CH₂)₃O- | -NHEt |
| **33** | F | NMe₂(CH₂)₃O- | -NEt(ⁱPr) |
| **34** | H | 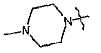 | -N(ⁱPr)₂ |
| **35** | H | 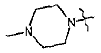 | N(ⁿPr2) |
| **36** | F | NMe₂(CH₂)₃O- | 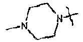 |
| **37** | H | 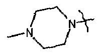 | -NEt(ⁱPr) |
| **38** | F | 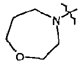 | 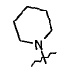 |
| **39** | F | 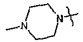 | -N(ⁱPr)₂ |
| **40** | F | 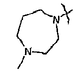 | -N(ⁱPr)₂ |

### Protocoles expérimentaux des tests cellulaires

Les composés ont été testés dans un test de viabilité/prolifération permettant de comparer, dans des conditions similaires, leur activité sur les cellules tumorales proliférantes et sur les cellules normales quiescentes.

### Origine des cellules :

Les cellules HeLa (lignée cellulaire tumorale) et les PBLs (cellules quiescentes, lymphocytes du sang périphérique) proviennent respectivement de l'ATCC et de Cambrex.

### Principe du test de viabilité/prolifération en FDA-IP :

Les cellules HeLa (lignée cellulaire tumorale) sont ensemencées à 0,25x10⁶ cellules/puits dans 2 mL de milieu de culture. Les produits sont ajoutés 24 h après ensemencement. Après 48 d'incubation en présence des produits, la totalité des cellules HeLa (surnageant et cellules adhérentes) est récupérée, centrifugée et reprise en PBS contenant une concentration finale de 0,25 µg/mL de fluorescéine diacétate (FDA), 10 µg/mL de iodure de propidium (IP) et 3 µg/mL de billes de 10 µm. Les suspensions cellulaires sont filtrées immédiatement avant analyse sur le Facscalibur. Les PBLs sont ensemencés à 10⁶ cellules/puits dans 2 mL de milieu de culture. Les produits sont ajoutés 1-4h après ensemencement. Après 48 d'incubation en présence des produits, la moitié de la suspension cellulaire est récupérée et incubée en présence d'une concentration finale de 0,25 µg/mL fluorescéine diacétate (FDA), 10 µg/mL iodure de propidium (IP) et 2 µg/mL de billes de 10 µm.

### Principe de lecture :

Le FDA qui entre dans les cellules est clivé par des estérases uniquement dans les cellules vivantes, libérant la fluorescéine qui émet un rayonnement fluorescent dans le vert lorsqu'elle est excitée (généralement par un rayonnement UV). L'IP n'entre que dans les cellules mortes dont la membrane est perméabilisée, et qui émet un rayonnement fluorescent dans le rouge lorsqu'il est excité. Les billes sont utilisées comme traceurs des cellules mortes ayant déjà disparu au moment de la lecture.

Calcul : le % de cellules viables par rapport au contrôle non-traité est donné par : (nombre de cellules vivantes/nombre cellules vivantes dans le contrôle)/(nombre de billes/nombre de billes dans le contrôle)x100 pour les cellules HeLa, et par : (nombre de cellules vivantes/nombre cellules vivantes dans le contrôle)x100 pour les PBLs quiescents. Les produits selon l'invention sont testés à plusieurs concentrations et on calcule une IC50 correspondant à la concentration provoquant 50% de l'inhibition maximale.

Les produits selon l'invention présentent une activité cellulaire dans le test d'inhibition de viabilité/prolifération des cellules tumorales (lignée HeLa) et une grande sélectivité vis-à-vis des lymphocytes du sang périphérique (PBLs) quiescents, comme modèle de cellules quiescentes. Le ratio d'IC50 d'inhibition de viabilité PBLs/IC50 d'inhibition de viabilité des cellules HeLa des produits selon l'invention, est supérieur à 10, et généralement supérieur à 50 ; avec une IC50 d'inhibition de viabilité des cellules HeLa inférieure à 1 µM.

Les IC50 sur les cellules tumorales des composés selon l'invention s'échelonnent de 5 à 50 µM. Notamment, les IC50 des composés des exemples 2, 4, 5, 6, 8, 9, 11, 13, 15, 16, 19, 20, 33, 34, 37, 39 et 40 sont inférieures à 100 nM. En particulier, celle du composé de l'exemple 31 est comprise entre 5 et 30 µM.

**Tableau II**

| **Exemple** | **Viability PBL IC50 (nM)** | **Viability HeLa IC50 (nM)** | **ratio** |
|---|---|---|---|
| 1 | 7552 | 107 | 70 |
| 12 | 3487 | 56 | 62 |
| 17 | 4246 | 100 | 42 |
| 18 | 3764 | 39 | 96 |
| 38 | 9166 | 85 | 108 |

## Revendications

1. Composé de formule générale (I) suivante : 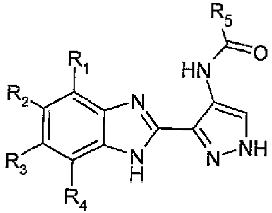 dans laquelle :
- R₁ et R₄ sont indépendamment sélectionnés dans le groupe constitué par : H, Me, Et, CO₂R_{c}, CH₂OR_{c}, OR_{c}, F, Cl, C(=O)NHR_{d} ; dans lequel R_{c} est choisi parmi H, (C₁-C₆)alkyle, (C₁-C₆)alkyle substitué, (C₃-C₇)cycloalkyle, (C₃-C₇)cycloalkyle substitué, aryle, aryle substitué, hétéroaryle et hétéroaryle substitué ; et dans lequel R_{d} est choisi parmi H, (C₁-C₆)alkyle, (C₁-C₆)alkyle substitué, (C₃-C₇)cycloalkyle, (C₃-C₇)cycloalkyle substitué et (C₃-C₇)hétérocycloalkyle comprenant de 1 à 3 hétéroatomes choisis parmi N, O et S, éventuellement substitué,
- R₂ est F,
- R₃ représente un groupe choisi parmi les groupes morpholinyle, homomorpholinyle, -O(CH₂)ₙpipéridinyle avec n = 2, 3 ou 4, -O(CH₂)ₙN(Me)₂ avec n=2, 3 ou 4, N-méthylpipérazinyle et N- méthylhomopipérazinyle, -NH(CH₂)₂NMe₂, et
- R₅ est sélectionné dans le groupe constitué par NMeEt, NH(ⁱPr), NEt₂, N(ⁱPr)₂, NEt(ⁱPr), pyrrolidinyle, pipéridinyle, morpholinyle, N-méthylpipérazinyle, NHCy, NCy₂, NMe(ⁱPr), NH(^{t}Bu), NH(ⁱBu), N(ⁿBu)₂, pipérazinyle, NH(Et), N(ⁿPr)₂, NEt(ⁱPr), où Cy désigne un radical cyclopropyle,
où « substitué » fait référence à la présence d'un ou plusieurs substituant(s) choisis parmi halogène, C₁-C₆ alkyle, C₃-C₁₀ cycloalkyle, hydroxy, C₁-C₆ alkyloxy, aryloxy, amino, C₁-C₆alkylamino, C₁-C₆dialkylamino, acyle ou phényle.

2. Composé selon la revendication 1 **caractérisé en ce que** R₅ est sélectionné dans le groupe constitué par NMeEt, NH(ⁱPr), NEt₂, N(ⁱPr)₂, NEt(ⁱPr), pyrrolidinyle, pipéridinyle, morpholinyle, N-méthylpipérazinyle, NHCy et NCy₂, où Cy désigne un radical cyclopropyle.

3. Composé selon l'une des revendications 1 ou 2 **caractérisé en ce que** R₁ et R₄ désignent tous les deux H.

4. Composé selon la revendication 1 répondant à la formule (Ia) : 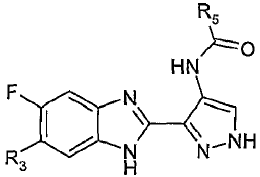 dans laquelle R₃ est tel que défini à la revendication 1 et R₅ représente NH(ⁱPr), NHEt, NEt₂, N(ⁱPr)₂, NEt(ⁱPr), pyrrolidinyle, pipéridinyle ou morpholinyle.

5. Composé selon la revendication 4 **caractérisé en ce que** R₃ représente morpholinyle, homomorpholinyle, -O(CH₂)ₙpipéridinyle avec n = 2, 3 ou 4, -O(CH₂)₃NMe₂, N-méthylpipérazinyle, N-méthylhomopipérazinyle et R₅ représente NEt₂ ou pipéridinyle.

6. Composé selon l'une quelconque des revendications 4 et 5 dans laquelle R₃ est choisi parmi -O(CH₂)₃NMe₂, N-méthylpipérazinyle, N-méthylhomopipérazinyle, morpholin-4-yle et homomorpholinyle.

7. Composé selon l'une des revendications 1 à 6 **caractérisé en ce que** R₅ est pipéridin-1-yle.

8. Composé selon la revendication 1 dont le nom suit :
N-[3-(5-fluoro-6-morpholin-4-yl-1H-benzimidazol-2-yl)-1H-pyrazol-4-yl]-pipéridine-1-carboxamide ;
1,1-diéthyl-3-[3-(5-fluoro-6-morpholin-4-yl-1H-benzimidazol-2-yl)-1H-pyrazol-4-yl]-urée ;
N-{3-[5-fluoro-6-(2-pipéridin-1-yl-éthoxy)-1H-benzimidazol-2-yl]-1H-pyrazol-4-yl}-pipéridine-1-carboxamide ;
1,1-diéthyl-3-{3-[5-fluoro-6-(2-pipéridin-1-yl-éthoxy)-1H-benzimidazol-2-yl]-1H-pyrazol-4-yl}-urée ;
3-{3-[6-(3-diméthylamiono-propoxy)-5-fluoro-1H-benzimidazol-2-yl]-1H-pyrazol-4-yl}-1,1-diéthyl-urée ;
1,1-diéthyl-3-{3-[5-fluoro-6-(3-pipéridin-1-yl-propoxy)-1H-benzimidazol-2-yl]-1H-pyrazol-4-yl}-urée ;
N-{3-[6-(3-diméthylamino-propoxy)-5-fluoro-1H-benzimidazol-2-yl]-1H-pyrazol-4-yl}-pipéridine-1-carboxamide ;
3-[3-(5-fluoro-6-perhydro-1,4-oxazépan-4-yl-1 H-benzimidazol-2-yl)-1 H-pyrazol-4-yl]-1,1-diisopropyl-urée ;
1,1-diéthyl-3-[3-(5-fluoro-6-perhydro-1,4-oxazépin-4-yl-1 H-benzimidazol-2-yl)-1 H-pyrazol-4-yl]-urée ;
N-{3-[5-fluoro-6-(4-méthyl-pipérazin-1-yl)-1H-benzimidazol-2-yl]-1H-pyrazol-4-yl}-pipéridine-1-carboxamide ;
1,1-diéthyl-3-{3-[5-fluoro-6-(4-méthyl-pipérazin-1-yl)-1H-benzimidazol-2-yl]-1H-pyrazol-4-yl}-urée ;
3-[3-(5-fluoro-6-morpholin-4-yl-1 H-benzimidazol-2-yl)-1H-pyrazol-4-yl]-1,1-diisopropyl-urée ;
3-{3-[6-(2-diméthylamino-éthylamino)-5-fluoro-1H-benzimidazol-2-yl]-1H-pyrazol-4-yl}-1,1-diéthyl-urée ;
N-{3-[5-fluoro-6-(4-méthyl-perhydro-1,4-diazépin-1-yl)-1H-benzimidazol-2-yl]-1H-pyrazol-4-yl}-pipéridine-1-carboxamide ;
1,1-diéthyl-3-{3-[5-fluoro-6-(4-méthyl-perhydro-1,4-diazépin-1-yl)-1H-benzimidazol-2-yl]-1H-pyrazol-4-yl}-urée ;
cyclopropyl-3-{3-[6-(3-diméthylamino-propoxy)-5-fluoro-1H-benzimidazol-2-yl]-1H-pyrazol-4-yl}-urée ;
N-{3-[6-(3-diméthylamino-propoxy)-5-fluoro-1H-benzimidazol-2-yl]-1H-pyrazol-4-yl}-pyrrolidine-1-carboxamide ;
1-{3-[6-(3-diméthylamino-propoxy)-5-fluoro-1H-benzimidazol-2-yl]-1H-pyrazol-4-yl}-3-isopropyl-urée ;
N-{3-[6-(3-diméthylamino-propoxy)-5-fluoro-1H-benzimidazol-2-yl]-1H-pyrazol-4-yl}-morpholine-4-carboxamide ;
3-{3-[6-(3-diméthylamino-propoxy)-5-fluoro-1H-benzimidazol-2-yl]-1H-pyrazol-4-yl}-1-isopropyl-1-méthyl-urée ;
1-tert-butyl-3-{3-[6-(3-diméthylamino-propoxy)-5-fluoro-1H-benzimidazol-2-yl]-1H-pyrazol-4-yl}-urée ;
1-{3-[6-(3-diméthylamino-propoxy)-5-fluoro-1H-benzimidazol-2-yl]-1H-pyrazol-4-yl}-3-isobutyl-urée ;
3-{3-[6-(3-diméthylamino-propoxy)-5-fluoro-1H-benzimidazol-2-yl]-1H-pyrazol-4-yl}-1,1-diisopropyl-urée ;
3-{3-[6-(3-diméthylamino-propoxy)-5-fluoro-1H-benzimidazol-2-yl]-1H-pyrazol-4-yl}-1,1-dipropyl-urée ;
1-{3-[6-(3-diméthylamino-propoxy)-5-fluoro-1H-benzimidazol-2-yl]-1H-pyrazol-4-yl}-3-éthyl-urée ;
3-{3-[6-(3-diméthylamino-propoxy)-5-fluoro-1H-benzimidazol-2-yl]-1H-pyrazol-4-yl}-1-éthyl-1-isopropyl-urée ;
N-{3-[6-(3-diméthylamino-propoxy)-5-fluoro-1H-benzimidazol-2-yl]-1H-pyrazol-4-yl}-4-méthyl-pipérazine-1-carboxamide ;
N-[3-(5-fluoro-6-perhydro-1,4-oxazépin-4-yl-1H-benzimidazol-2-yl)-1H-pyrazol-4-yl]-pipéridine-1-carboxamide ;
3-{3-[5-fluoro-6-(4-méthyl-pipérazin-1-yl)-1H-benzimidazol-2-yl]-1H-pyrazol-4-yl}-1,1-diisopropyl-urée ;
3-{3-[5-fluoro-6-(4-méthyl-perhydro-1,4-diazépin-1-yl)-1H-benzimidazol-2-yl]-1H-pyrazol-4-yl}-1,1-diisopropyl-urée.

9. Composé selon la revendication 1 de formule :

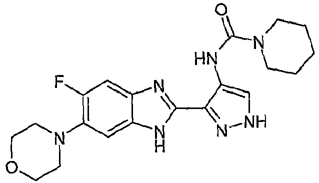

10. Composé selon l'une quelconque des revendications 1 à 9 sous forme :
non chirale, ou
racémique, ou
enrichie en un stéréo-isomère, ou
enrichie en un énantiomère,
sous ses différentes formes tautomères et éventuellement salifié.

11. Composition pharmaceutique comprenant un composé selon l'une quelconque des revendications 1 à 10, en combinaison avec un excipient pharmaceutiquement acceptable.

12. Composé selon l'une quelconque des revendications 1 à 10 pour son utilisation en tant que médicament.

13. Composé selon l'une quelconque des revendications 1 à 10 pour son utilisation pour le traitement du cancer.

14. Composé selon l'une quelconque des revendications 1 à 10 pour son utilisation pour le traitement d'une maladie choisie dans le groupe constitué du psoriasis, du glaucome, des leucémies et des tumeurs solides, des inflammations, et des maladies liées à une dérégulation de protéines kinases.

## Patentansprüche

1. Verbindung der folgenden allgemeinen Formel (I): 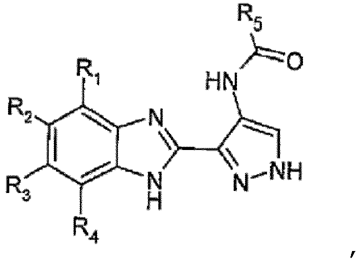 worin:
- R₁ und R₄ unabhängig voneinander aus der Gruppe ausgewählt sind, bestehend aus: H, Me, Et, CO₂R_{c}, CH₂OR_{c}, OR_{c}, F, Cl, C(=O)NHR_{d}; wobei R_{c} aus H, (C₁-C₆)Alkyl, substituiertem (C₁-C₆) Alkyl, (C₃-C₇)Cycloalkyl, substituiertem (C₃-C₇)Cycloalkyl, Aryl, substituiertem Aryl, Heteroaryl und substituiertem Heteroaryl; und wobei R_{d} ausgewählt ist aus H, (C₁-C₆)Alkyl, substituiertem (C₁-C₆)Alkyl, (C₃-C₇)Cycloalkyl, substituiertem (C₃-C₇)Cycloalkyl und gegebenenfalls substituiertem (C₃-C₇)Heterocycloalkyl, das 1 bis 3 aus N, O und S ausgewählte Heteroatome umfasst;
- R₂ F ist,
- R₃ eine Gruppe darstellt, ausgewählt aus den Gruppen Morpholinyl, Homomorpholinyl, -O(CH₂)ₙpiperidinyl mit n = 2, 3 oder 4, -O(CH₂)ₙN(Me)₂ mit n = 2, 3 oder 4, N-Methylpiperazinyl und N-Methylhomopiperazinyl, -NH(CH₂)₂NMe₂, und
- R₅ aus der Gruppe ausgewählt ist, bestehend aus NMeEt, NH(ⁱPr), NEt₂, N((ⁱPr)₂, NEt(ⁱPr), Pyrrolidinyl, Piperidinyl, Morpholinyl, N-Methylpiperazinyl, NHCy, NCy₂, NMe(ⁱPr) , NH(^{t}Bu), NH(ⁱBu), N(ⁿBu)₂, Piperazinyl, NH(Et), N(ⁿPr)₂, NEt(ⁱPr), wobei Cy für einen Cyclopropylrest steht;
wobei sich "substituiert" auf das Vorhandensein eines oder mehrerer Substituenten bezieht, die aus Halogen, C₁-C₆Alkyl, C₃-C₁₀Cycloalkyl, Hydroxy, C₁-C₆Alkyloxy, Aryloxy, Amino, C₁-C₆Alkylamino, C₁-C₆Dialkylamino, Acyl oder Phenyl ausgewählt sind.

2. Verbindung nach Anspruch 1, **dadurch gekennzeichnet, dass** R₅ aus der Gruppe ausgewählt ist, bestehend aus NMeEt, NH(ⁱPr), NEt₂, N(ⁱPr)₂, NEt(ⁱPr), Pyrrolidinyl, Piperidinyl, Morpholinyl, N-Methylpiperazinyl, NHCy und NCy₂, wobei Cy für einen Cyclpropylrest steht.

3. Verbindung nach einem der Ansprüche 1 oder 2, **dadurch gekennzeichnet, dass** R₁ und R₄ beide für H stehen.

4. Verbindung nach Anspruch 1 der Formel (Ia): 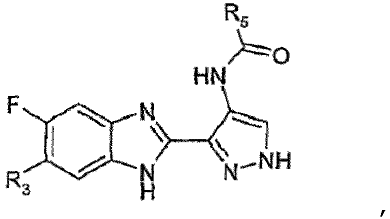 worin R₃ wie in Anspruch 1 definiert ist und R₅ NH(ⁱPr), NHEt, NEt₂, N(ⁱPr)₂, NEt(ⁱPr), Pyrrolidinyl, Piperidinyl oder Morpholinyl darstellt.

5. Verbindung nach Anspruch 4, **dadurch gekennzeichnet, dass** R₃ Morpholinyl, Homomorpholinyl, -O(CH₂)ₙpiperidinyl mit n = 2, 3 oder 4, -O(CH₂)₃N(Me)₂, N-Methylpiperazinyl, N-Methylhomopiperazinyl darstellt und R₅ NEt₂ oder Piperidinyl darstellt.

6. Verbindung nach einem der Ansprüche 4 und 5, worin R₃ aus -O(CH₂)₃NMe₂, N-Methylpiperazinyl, N-Methylhomopiperazinyl, Morpholin-4-yl und Homomorpholinyl ausgewählt ist.

7. Verbindung nach einem der Ansprüche 1 bis 6, **dadurch gekennzeichnet, dass** R₅ Piperidin-1-yl ist.

8. Verbindung nach Anspruch 1 mit der folgenden Bezeichnung:
N-[3-(5-Fluor-6-morpholin-4-yl-1H-benzimidazol-2-yl)-1H-pyrazol-4-yl]-piperidin-1-carboxamid;
1,1-Diethyl-3-[3-(5-fluor-6-morpholin-4-yl-1H-benzimidazol-2-yl)-1H-pyrazol-4-yl]-harnstoff;
N-{3-[5-Fluor-6-(2-piperidin-1-ylethoxy)-1H-benzimidazol-2-yl]-1H-pyrazol-4-yl}piperidin-1-carboxamid;
1,1-Diethyl-3-{3-[5-fluor-6-(2-piperidin-1-ylethoxy)-1H-benzimidazol-2-yl]-1H-pyrazol-4-yl}-harnstoff;
3-{3-[6-(3-Dimethylaminopropoxy)-5-fluor-1H-benzimidazol-2-yl]-1H-pyrazol-4-yl}-1,1-diethylharnstoff;
1,1-Diethyl-3-{3-[5-fluor-6-(3-piperidin-1-ylpropoxy)-1H-benzimidazol-2-yl]-1H-pyrazol-4-yl}-harnstoff;
N-{3-[6-(3-Dimethylaminopropoxy)-5-fluor-1H-benzimidazol-2-yl]-1H-pyrazol-4-yl}-piperidin-1-carboxamid;
3-[3-(5-Fluor-6-perhydro-1,4-oxazepan-4-yl-1H-benzimidazol-2-yl)-1H-pyrazol-4-yl]-1,1-diisopropylharnstoff;
1,1-Diethyl-3-[3-(5-fluor-6-perhydro-1,4-oxazepin-4-yl-1H-benzimidazol-2-yl)-1H-pyrazol-4-yl]-harnstoff;
N-{3-[5-Fluor-6-(4-methylpiperazin-1-yl)-1H-benzimidazol-2-yl]-1H-pyrazol-4-yl}-piperidin-1-carboxamid;
1,1-Diethyl-3-{3-[5-fluor-6-(4-methylpiperazin-1-yl)-1H-benzimidazol-2-yl]-1H-pyrazol-4-yl}-harnstoff;
3-[3-(5-Fluor-6-morpholin-4-yl-1H-benzimidazol-2-yl)-1H-pyrazol-4-yl]-1,1-diisopropylharnstoff;
3-{3-[6-(2-Dimethylaminoethylamino)-5-fluor-1H-benzimidazol-2-yl]-1H-pyrazol-4-yl}-1,1-diethylharnstoff;
N-{3-[5-Fluor-6-(4-methylperhydro-1,4-diazepin-1-yl)-1H-benzimidazol-2-yl]-1H-pyrazol-4-yl}-piperidin-1-carboxamid;
1,1-Diethyl-3-{3-[5-fluor-6-(4-methylperhydro-1,4-diazepin-1-yl)-1H-benzimidazol-2-yl]-1H-pyrazol-4-yl}-harnstoff;
Cyclopropyl-3-{3-[6-(3-dimethylaminopropoxy)-5-fluor-1H-benzimidazol-2-yl]-1H-pyrazol-4-yl}-harnstoff;
N-{3-[6-(3-Dimethylaminopropoxy)-5-fluor-1H-benzimidazol-2-yl]-1H-pyrazol-4-yl}pyrrolidin-1-carboxamid;
1-{3-[6-(3-Dimethylaminopropoxy)-5-fluor-1H-benzimidazol-2-yl]-1H-pyrazol-4-yl}-3-isopropylharnstoff;
N-{3-[6-(3-Dimethylaminopropoxy)-5-fluor-1H-benzimidazol-2-yl]-1H-pyrazol-4-yl}-morpholin-4-carboxamid;
3-{3-[6-(3-Dimethylaminopropoxy)-5-fluor-1H-benzimidazol-2-yl]-1H-pyrazol-4-yl}-1-isopropyl-1-methylharnstoff;
1-tert-Butyl-3-{3-[6-(3-dimethylaminopropoxy)-5-fluor-1H-benzimidazol-2-yl]-1H-pyrazol-4-yl}-harnstoff;
1-{3-[6-(3-Dimethylaminopropoxy)-5-fluor-1H-benzimidazol-2-yl]-1H-pyrazol-4-yl}-3-isobutylharnstoff;
3-{3-[6-(3-Dimethylaminopropoxy)-5-fluor-1H-benzimidazol-2-yl]-1H-pyrazol-4-yl}-1,1-diisopropylharnstoff;
3-{3-[6-(3-Dimethylaminopropoxy)-5-fluor-1H-benzimidazol-2-yl]-1H-pyrazol-4-yl}-1,1-dipropylharnstoff;
1-{3-[6-(3-Dimethylaminopropoxy)-5-fluor-1H-benzimidazol-2-yl]-1H-pyrazol-4-yl}-3-ethylharnstoff;
3-{3-[6-(3-Dimethylaminopropoxy)-5-fluor-1H-benzimidazol-2-yl]-1H-pyrazol-4-yl}-1-ethyl-1-isopropylharnstoff;
N-{3-[6-(3-Dimethylaminopropoxy)-5-fluor-1H-benzimidazol-2-yl]-1H-pyrazol-4-yl}-4-methylpiperazin-1-carboxamid;
N-[3-(5-Fluor-6-perhydro-1,4-oxazepin-4-yl-1H-benzimidazol-2-yl)-1H-pyrazol-4-yl]-piperidin-1-carboxamid;
3-{3-[5-Fluor-6-(4-methylpiperazin-1-yl)-1H-benzimidazol-2-yl]-1H-pyrazol-4-yl}-1,1-diisopropylharnstoff;
3-{3-[5-Fluor-6-(4-methylperhydro-1,4-diazepin-1-yl)-1H-benzimidazol-2-yl]-1H-pyrazol-4-yl}-1,1-diisopropylharnstoff.

9. Verbindung nach Anspruch 1 der Formel

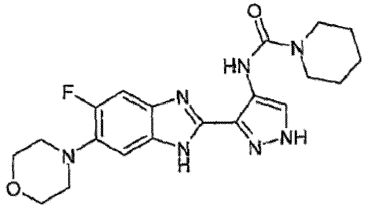

10. Verbindung nach einem der Ansprüche 1 bis 9 in folgender Form:
nicht chiraler oder
racemischer oder
an einem Stereoisomer angereicherter oder
an einem Enantiomer angereicherter,
in ihren verschiedenen tautomeren Formen und gegebenenfalls in Form eines Salzes.

11. Pharmazeutische Zusammensetzung, umfassend eine Verbindung nach einem der Ansprüche 1 bis 10 in Kombination mit einem pharmazeutisch annehmbaren Exzipienten.

12. Verbindung nach einem der Ansprüche 1 bis 10 für die Verwendung als Arzneimittel.

13. Verbindung nach einem der Ansprüche 1 bis 10 für die Verwendung zur Behandlung von Krebs.

14. Verbindung nach einem der Ansprüche 1 bis 10 für die Verwendung zur Behandlung einer Krankheit, ausgewählt aus der Gruppe, bestehend aus Psoriasis, Glaukom, Leukämien und soliden Tumoren, Entzündungen und Krankheiten, die mit einer Deregulation von Proteinkinasen in Zusammenhang stehen.

## Claims

1. Compound of general formula (I) below: 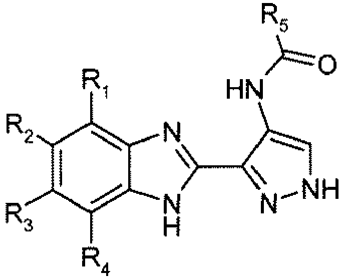 in which:
- R₁ and R₄ are independently selected from the group comprising: H, Me, Et, CO₂R_{c}, CH₂OR_{c}, OR_{c}, F, Cl and C(=O)NHR_{d}; in which R_{c} is chosen from H, (C₁-C₆) alkyl, substituted (C₁-C₆)alkyl, (C₃-C₇)-cycloalkyl, substituted (C₃-C₇)cycloalkyl, aryl, substituted aryl, heteroaryl and substituted heteroaryl; and in which R_{d} is chosen from H, (C₁-C₆) alkyl, substituted (C₁-C₆) alkyl, (C₃-C₇) cycloalkyl, substituted (C₃-C₇)cycloalkyl, and optionally substituted (C₃-C₇)heterocycloalkyl comprising from 1 to 3 heteroatoms chosen from N, O and S,
- R₂ is F,
- R₃ represents a group chosen from the groups morpholinyl, homomorpholinyl, -O(CH₂)ₙpiperidinyl with n=2, 3 or 4, -O(CH₂)ₙN(Me)₂ with n=2, 3 or 4, N-methylpiperazinyl and N-methylhomopiperazinyl, -NH(CH₂)₂NMe₂, and
- R₅ is selected from the group comprising NMeEt, NH(ⁱPr), NEt₂, N(ⁱPr)₂, NEt(ⁱPr), pyrrolidinyl, piperidinyl, morpholinyl, N-methylpiperazinyl, NHCy, NCy₂, NMe(ⁱPr), NH(^{t}Bu), NH(ⁱBu), N(ⁿBu)₂, piperazinyl, NH(Et), N(ⁿPr)₂ and NEt(ⁱPr), where Cy denotes a cyclopropyl radical,
where "substituted" refers to the presence of one or more substituent(s) chosen from halogen, C₁-C₆ alkyl, C₃-C₁₀ cycloalkyl, hydroxyl, C₁-C₆ alkyloxy, aryloxy, amino, C₁-C₆ alkylamino, C₁-C₆ dialkyl-amino, acyl or phenyl.

2. Compound according to Claim 1, **characterized in that** R₅ is selected from the group comprising: NMeEt, NH(ⁱPr), NEt₂, N(ⁱPr)₂, NEt(ⁱPr), pyrrolidinyl, piperidinyl, morpholinyl, N-methylpiperazinyl, NHCy and NCy₂, where Cy denotes a cyclopropyl radical.

3. Compound according to either of Claims 1 and 2, **characterized in that** R₁ and R₄ both denote H.

4. Compound according to Claim 1, corresponding to formula (Ia) : 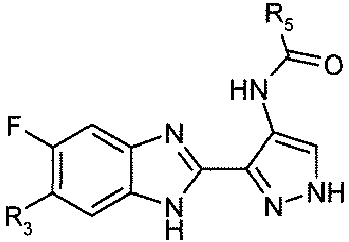 in which R₃ is as defined in Claim 1 and R₅ is NH(ⁱPr), NHEt, NEt₂, N(ⁱPr)₂, NEt(ⁱPr), pyrrolidinyl, piperidinyl or morpholinyl.

5. Compound according to Claim 4, **characterized in that** R₃ is morpholinyl, homomorpholinyl, -O(CH₂)ₙpiperidinyl with n=2, 3 or 4, -O(CH₂)₃NMe₂, N-methylpiperazinyl or N-methylhomopiperazinyl, and R₅ is NEt₂ or piperidinyl.

6. Compound according to any either of Claims 4 and 5, in which R₃ is chosen from -O(CH₂)₃NMe₂, N-methylpiperazinyl, N-methylhomopiperazinyl, morpholin-4-yl and homomorpholinyl.

7. Compound according to one of Claims 1 to 6, **characterized in that** R₅ is piperidin-1-yl.

8. Compound according to Claim 1, the name of which follows:
N-[3-(5-Fluoro-6-morpholin-4-yl-1H-benzimidazol-2-yl)-1H-pyrazol-4-yl]piperidine-1-carboxamide;
1,1-Diethyl-3-[3-(5-fluoro-6-morpholin-4-yl-1H-benzimidazol-2-yl)-1H-pyrazol-4-yl]urea;
N-{3-[5-fluoro-6-(2-piperidin-1-ylethoxy)-1H-benzimidazol-2-yl]-1H-pyrazol-4-yl}piperidine-1-carboxamide;
1,1-Diethyl-3-{3-[5-fluoro-6-(2-piperidin-1-ylethoxy)-1H-benzimidazol-2-yl]-1H-pyrazol-4-yl}urea;
3-{3-[6-(3-Dimethylaminopropoxy)-5-fluoro-1H-benzimidazol-2-yl]-1H-pyrazol-4-yl}-1,1-diethylurea;
1,1-Diethyl-3-{3-[5-fluoro-6-(3-piperidin-1-ylpropoxy)-1H-benzimidazol-2-yl]-1H-pyrazol-4-yl}urea;
N-{3-[6-(3-dimethylaminopropoxy)-5-fluoro-1H-benzimidazol-2-yl]-1H-pyrazol-4-yl}piperidine-1-carboxamide;
3-[3-(5-Fluoro-6-perhydro-1,4-oxazepan-4-yl-1H-benzimidazol-2-yl)-1H-pyrazol-4-yl]-1,1-diisopropylurea;
1,1-Diethyl-3-[3-(5-fluoro-6-perhydro-1,4-oxazepin-4-yl-1H-benzimidazol-2-yl)-1H-pyrazol-4-yl]urea;
N-{3-[5-fluoro-6-(4-methylpiperazin-1-yl)-1H-benzimidazol-2-yl]-1H-pyrazol-4-yl}piperidine-1-carboxamide;
1,1-Diethyl-3-{3-[5-fluoro-6-(4-methylpiperazin-1-yl)-1H-benzimidazol-2-yl]-1H-pyrazol-4-yl}urea;
3-[3-(5-Fluoro-6-morpholin-4-yl-1H-benzimidazol-2-yl)-1H-pyrazol-4-yl]-1,1-diisopropylurea;
3-{3-[6-(2-Dimethylaminoethylamino)-5-fluoro-1H-benzimidazol-2-yl]-1H-pyrazol-4-yl}-1,1-diethylurea;
N-{3-[5-fluoro-6-(4-methylperhydro-1,4-diazepin-1-yl)-1H-benzimidazol-2-yl]-1H-pyrazol-4-yl}piperidine-1-carboxamide;
1,1-Diethyl-3-{3-[5-fluoro-6-(4-methylperhydro-1,4-diazepin-1-yl)-1H-benzimidazol-2-yl]-1H-pyrazol-4-yl}urea;
Cyclopropyl-3-{3-[6-(3-dimethylaminopropoxy)-5-fluoro-1H-benzimidazol-2-yl]-1H-pyrazol-4-yl}urea;
N-{3-[6-(3-Dimethylaminopropoxy)-5-fluoro-1H-benzimidazol-2-yl]-1H-pyrazol-4-yl}pyrrolidine-1-carboxamide;
1-{3-[6-(3-Dimethylaminopropoxy)-5-fluoro-1H-benzimidazol-2-yl]-1H-pyrazol-4-yl}-3-isopropylurea;
N-{3-[6-(3-Dimethylaminopropoxy)-5-fluoro-1H-benzimidazol-2-yl]-1H-pyrazol-4-yl}morpholine-4-carboxamide;
3-{3-[6-(3-Dimethylaminopropoxy)-5-fluoro-1H-benzimidazol-2-yl]-1H-pyrazol-4-yl}-1-isopropyl-1-methylurea;
1-tert-Butyl-3-{3-[6-(3-dimethylaminopropoxy)-5-fluoro-1H-benzimidazol-2-yl]-1H-pyrazol-4-yl}urea;
1-{3-[6-(3-Dimethylaminopropoxy)-5-fluoro-1H-benzimidazol-2-yl]-1H-pyrazol-4-yl}-3-isobutyl-urea;
3-{3-[6-(3-Dimethylaminopropoxy)-5-fluoro-1H-benzimidazol-2-yl]-1H-pyrazol-4-yl}-1,1-diisopropylurea;
3-{3-[6-(3-Dimethylaminopropoxy)-5-fluoro-1H-benzimidazol-2-yl]-1H-pyrazol-4-yl}-1,1-dipropylurea;
1-{3-[6-(3-Dimethylaminopropoxy)-5-fluoro-1H-benzimidazol-2-yl]-1H-pyrazol-4-yl}-3-ethylurea;
3-{3-[6-(3-Dimethylaminopropoxy)-5-fluoro-1H-benzimidazol-2-yl]-1H-pyrazol-4-yl}-1-ethyl-1-isopropylurea;
N-{3-[6-(3-Dimethylaminopropoxy)-5-fluoro-1H-benzimidazol-2-yl]-1H-pyrazol-4-yl}-4-methyl-piperazine-1-carboxamide;
N-[3-(5-fluoro-6-perhydro-1,4-oxazepin-4-yl-1H-benzimidazol-2-yl)-1H-pyrazol-4-yl]piperidine-1-carboxamide;
3-{3-[5-Fluoro-6-(4-methylpiperazin-1-yl)-1H-benzimidazol-2-yl]-1H-pyrazol-4-yl}-1,1-diisopropylurea;
3-{3-[5-Fluoro-6-(4-methylperhydro-1,4-diazepin-1-yl)-1H-benzimidazol-2-yl]-1H-pyrazol-4-yl}-1,1-diisopropylurea.

9. Compound according to Claim 1, of formula:

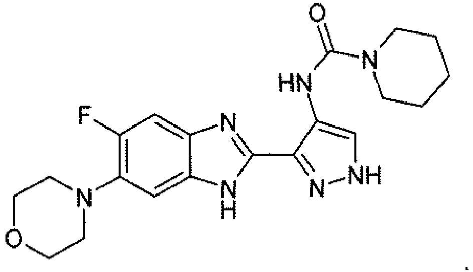

10. Compound according to any one of Claims 1 to 9, in a form which is:
nonchiral, or
racemic, or
enriched in one stereoisomer, or
enriched in one enantiomer;
in the various tautomeric forms thereof and
which is optionally salified.

11. Pharmaceutical composition comprising a compound according to any one of Claims 1 to 10, in combination with a pharmaceutically acceptable excipient.

12. Compound according to any one of Claims 1 to 10, for the use thereof as a medicament.

13. Compound according to any one of Claims 1 to 10, for the use thereof for the treatment of cancer.

14. Compound according to any one of Claims 1 to 10, for the use thereof for the treatment of a disease chosen from the group comprising psoriasis, glaucoma, leukaemias and solid tumours, inflammations, and diseases associated with a disruption of protein kinases.
